# EUROPÄISCHE PATENTSCHRIFT

(11) **EP 0 523 512 B1**
(45) Veröffentlichungstag und Bekanntmachung des Hinweises auf die Patenterteilung: **15.01.1997**
(21) Anmeldenummer: 92111454.2
(22) Anmeldetag: 06.07.1992
(51) Int. Cl.: C07D 215/56, A61K 31/47, C07D 471/04, C07D 401/04, C07D 498/04, C07D 487/04, C07D 487/08

(54) **8-Vinyl- und 8-Ethinyl-chinoloncarbonsäuren**
8-vinyl- and 8-ethinyl-quinolone carboxylic acids
Acides quinolone-carboxyliques-vinyl-8 et -éthinyl-8

(30) Priorität: 19.07.1991 DE 4123918
(43) Veröffentlichungstag der Anmeldung: 20.01.1993
(73) Patentinhaber: BAYER AG, 51368 Leverkusen (DE)
(72) Erfinder: Petersen, Uwe, Dr., W-5090 Leverkusen (DE); Himmler, Thomas, Dr., W-5068 Odenthal-Glöbusch (DE); Schenke, Thomas, Dr., W-5060 Bergisch Gladbach 2 (DE); Krebs, Andreas, Dr., W-5068 Odenthal (DE); Grohe, Klaus, Dr., W-5068 Odenthal (DE); Bremm, Klaus, Klaus-Dieter, Dr., W-5600 Wuppertal (DE); Metzger, Karl Georg, Dr., W-5600 Wuppertal 1 (DE); Endermann, Rainer, Dr., W-5600 Wuppertal 1 (DE); Zeiler, Hans-Joachim, Dr., W-5600 Wuppertal (DE)

(56) Entgegenhaltungen:
- EP-A- 0 237 955
- GB-A- 2 188 317
- US-A- 4 123 536
- US-A- 5 047 538
- J.Clin.Pharmacol. 28,156(1988)
- Antimicrob.Agents Chemother.33,131(1989)

## Beschreibung

Die Erfindung betrifft neue 8-Vinyl- und 8-Ethinyl-chinoloncarbonsäuren, Verfahren zu ihrer Herstellung sowie diese enthaltende antibakterielle Mittel und Futterzusatzstoffe.

Es ist bereits bekannt geworden, daß 8-Alkyl-chinoloncarbonsäuren antibakteriell wirksam sind: So wurden 8-Methyl-chinoloncarbonsäuren zum Beispiel in EP 237 955 und JP 2 019 377 und 8-Trifluormethyl-chinoloncarbonsäuren in US 4 780 468, US 4 803 205 und 4 933 335 beschrieben.

Auch aus der GB-A-2 188 317, US-PS 4,123,536, sowie aus J. Clin. Pharmacol. **28**, 156 (1988) und antimicrob. Agents Chemother. **33**, 131 (1989) sind bereits antimikrobiell wirksame Chinolonderivate bekanntgeworden, deren Wirkstärke jedoch nicht befriedigt.

Es wurde nun gefunden, daß die neuen Verbindungen der Formel (I) 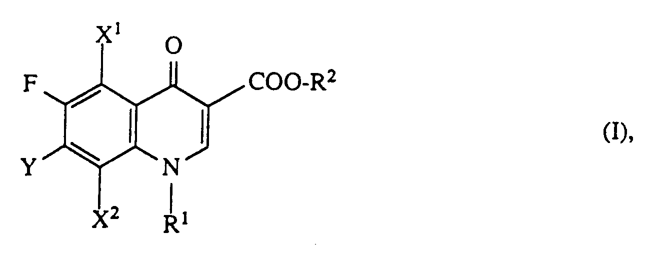 in welcher
- R¹: für gegebenenfalls durch Hydroxy, Halogen oder C₁-C₃-Alkoxy substituiertes geradkettiges oder verzweigtes C₁-C₄-Alkyl, für gegebenenfalls durch Halogen oder C₁-C₃-Alkyl substituiertes C₃-C₆-Cycloalkyl, C₂-C₄-Alkenyl, ferner für C₁-C₃-Alkoxy, Amino, Monoalkylamino mit 1 bis 3 C-Atomen, Dialkylamino mit 2 bis 6 C-Atomen oder für gegebenenfalls durch Halogen ein- bis dreifach substituiertes Phenyl steht,
- R²: für Wasserstoff, Alkyl mit 1 bis 4 Kohlenstoffatomen oder (5-Methyl-2-oxo-1,3-dioxol-4-yl)-methyl steht
- X¹: für Wasserstoff, Fluor, Chlor, Amino oder Methyl,
- X²: für 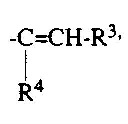 -C≡C-R⁵ oder -CH₂-CH=CH₂ steht,
worin
- R³: Wasserstoff, C₁-C₃-Alkyl, C₁-C₃-Alkoxy oder Alkoxymethyl mit 1 bis 3 C-Atomen im Alkoxyteil;
- R⁴: Wasserstoff oder Halogen und
- R⁵: Wasserstoff, gegebenenfalls durch Halogen ein- bis dreifach substituiertes C₁-C₆-Alkyl, C₂-C₃-Alkenyl, Alkoxy mit 1 bis 3 C-Atomen, Alkoxymethyl mit 1 bis 3 C-Atomen im Alkoxyteil, Halogen oder Trimethylsilyl bedeutet und
- Y: für 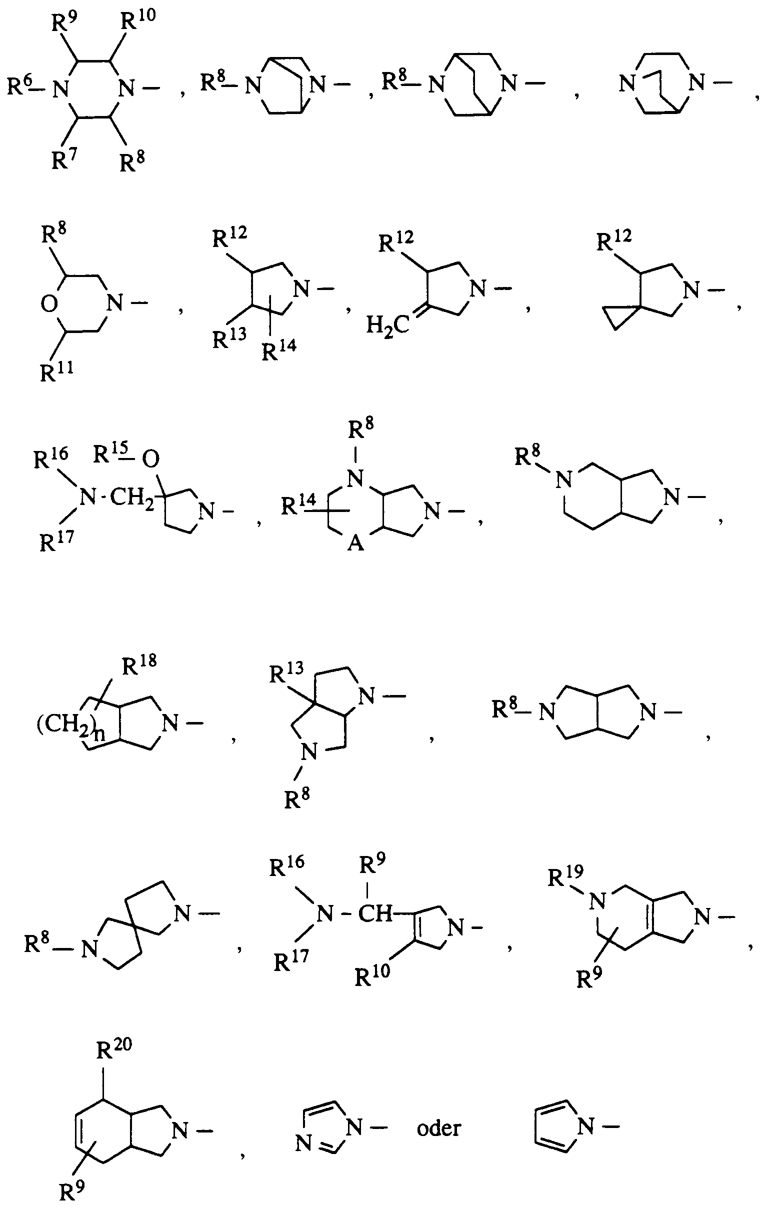 steht,
worin
- R⁶: für Wasserstoff, gegebenenfalls durch Hydroxy oder Methoxy substituiertes geradkettiges oder verzweigtes C₁-C₄-Alkyl, Cyclopropyl, Oxoalkyl mit 1 bis 4 C-Atomen, Acyl mit 1 bis 3 C-Atomen,
- R⁷: für Wasserstoff, Methyl, Phenyl, Thienyl oder Pyridyl,
- R⁸: für Wasserstoff oder Methyl,
- R⁹: für Wasserstoff oder Methyl,
- R¹⁰: für Wasserstoff oder Methyl,
- R¹¹: für Wasserstoff, Methyl oder 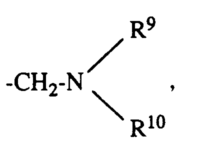
- R¹²: für Wasserstoff, Methyl, Amino, gegebenenfalls durch Hydroxy substituiertes Alkyl- oder Dialkylamino mit 1 oder 2 C-Atomen im Alkylteil, Aminomethyl, Aminoethyl, gegebenenfalls durch Hydroxy substituiertes Alkyl- oder Dialkylaminomethyl mit 1 oder 2 C-Atomen im Alkylteil oder 1-Imidazolyl,
- R¹³: für Wasserstoff, Hydroxy, Methoxy, Methylthio oder Halogen, Methyl, Hydroxymethyl,
- R¹⁴: für Wasserstoff oder Methyl,
- R¹⁵: für Wasserstoff, Methyl oder Ethyl,
- R¹⁶: für Wasserstoff, Methyl oder Ethyl,
- R¹⁷: für Wasserstoff, Methyl oder Ethyl,
- R¹⁸: für Hydroxy, 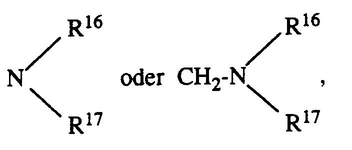
- R¹⁹: für Wasserstoff, gegebenenfalls durch Hydroxy substituiertes C₁-C₃-Alkyl, Alkoxycarbonyl mit 1 bis 4 Kohlenstoffatomen im Alkoxyteil oder C₁-C₃-Acyl,
- R²⁰: für Wasserstoff, Hydroxy, 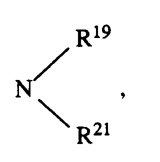 Hydroxymethyl oder 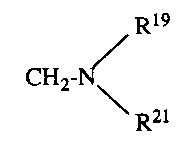 steht, worin
- R²¹: Wasserstoff oder Methyl bedeutet,
- A: für CH₂, O oder für eine direkte Bindung steht und
- n: für 1 oder 2 steht,
und deren pharmazeutisch verwendbaren Hydrate und Säureadditionssalze sowie die Alkali-, Erdalkali-, Silber- und Guanidiniumsalze der zugrundeliegenden Carbonsäuren eine hohe antibakterielle Wirkung aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt sind die Verbindungen der Formel (I),
in welcher
- R¹: für gegebenenfalls durch Hydroxy substituiertes C₁-C₂-Alkyl, C₃-C₅-Cycloalkyl, Vinyl, Amino, Monoalkylamino mit 1 bis 2 C-Atomen, Dialkylamino mit 2 bis 4 C-Atomen oder für gegebenenfalls durch Halogen ein- oder zweifach substituiertes Phenyl steht,
- R²: für Wasserstoff, Alkyl mit 1 bis 3 Kohlenstoffatomen oder (5-Methyl-2-oxo-1,3-dioxol-4-yl)-methyl steht
- X¹: für Wasserstoff, Fluor, Chlor, Amino oder Methyl,
- X²: für 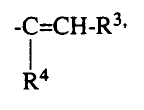 -C≡C-R⁵ oder -CH₂-CH=CH₂ steht,
worin
- R³: Wasserstoff, C₁-C₂-Alkyl, Methoxy oder Methoxymethyl,
- R⁴: Wasserstoff und
- R⁵: Wasserstoff, gegebenenfalls durch Fluor ein- bis dreifach substituiertes C₁-C₄-Alkyl, C₂-C₃-Alkenyl, Methoxy oder Trimethylsilyl bedeutet und
- Y: für 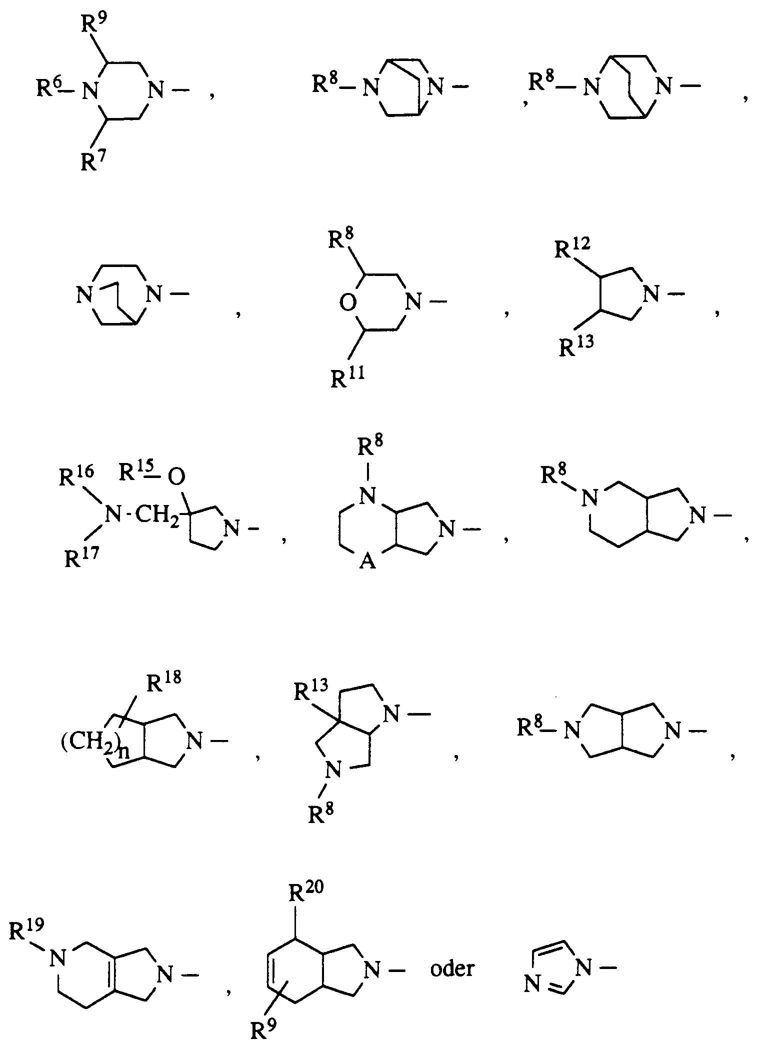 steht,
worin
- R⁶: für Wasserstoff, gegebenenfalls durch Hydroxy substituiertes geradkettiges oder verzweigtes C₁-C₃-Alkyl, Oxoalkyl mit 1 bis 4 C-Atomen,
- R⁷: für Wasserstoff, Methyl oder Phenyl,
- R⁸: für Wasserstoff oder Methyl,
- R⁹: für Wasserstoff oder Methyl,
- R¹¹: für Wasserstoff, Methyl oder -CH₂-NH₂,
- R¹²: für Wasserstoff, Methyl, Amino, Methylamino, Dimethylamino, Aminomethyl, Methylaminomethyl oder Ethylaminomethyl,
- R¹³: für Wasserstoff, Hydroxy, Methoxy, Fluor, Methyl oder Hydroxymethyl,
- R¹⁵: für Wasserstoff oder Methyl,
- R¹⁶: für Wasserstoff oder Methyl,
- R¹⁷: für Wasserstoff oder Methyl,
- R¹⁸: für 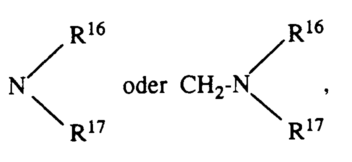
- R¹⁹: für Wasserstoff, Methyl oder Ethyl,
- R²⁰: für 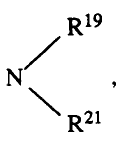 steht,
worin
- R²¹: Wasserstoff oder Methyl bedeutet,
- A: für CH₂, O oder für eine direkte Bindung steht und
- n: für 1 oder 2 steht.

Besonders bevorzugt sind Verbindungen der Formel (I),
in welcher
- R¹: für Methyl, Ethyl, Cyclopropyl oder gegebenenfalls durch Fluor ein-oder zweifach substituiertes Phenyl,
- R²: für Wasserstoff, Methyl oder Ethyl,
- X¹: für Wasserstoff, Fluor, Chlor, Amino oder Methyl,
- X²: für -CH=CH₂ oder -C≡C-R⁵ steht,
worin
- R⁵: Wasserstoff, C₁-C₄-Alkyl, C₂-C₃-Alkenyl oder Trimethylsilyl bedeutet und
- Y: für 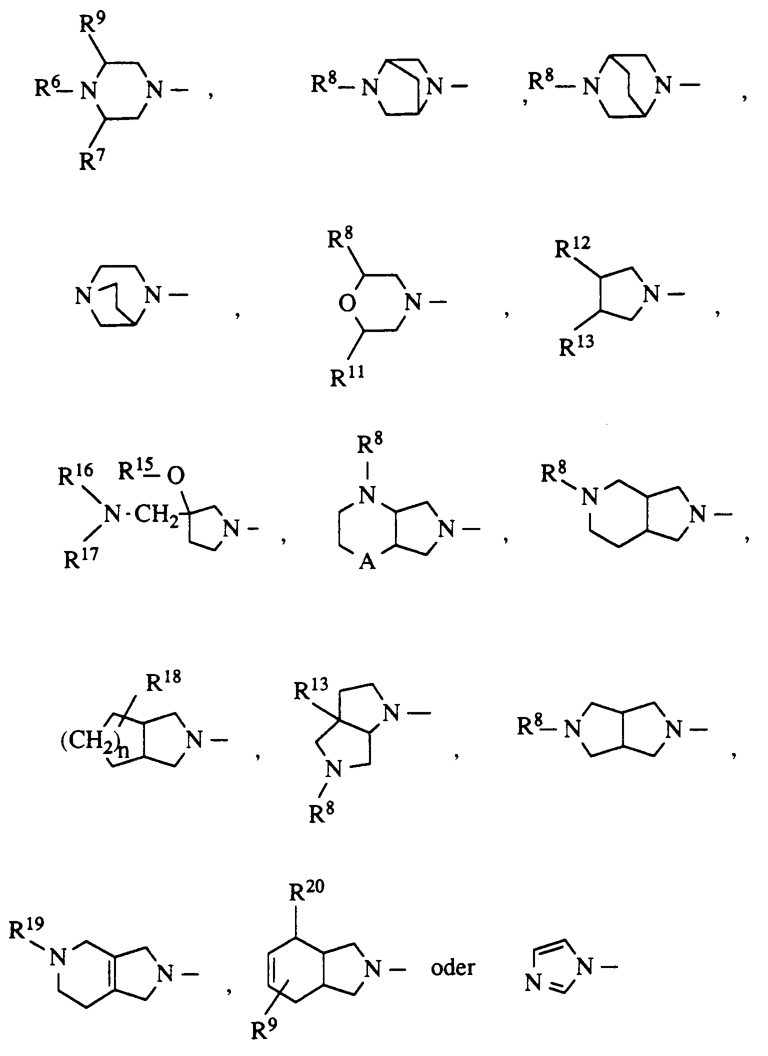 steht,
worin
- R⁶: für Wasserstoff, Methyl, gegebenenfalls durch Hydroxy substituiertes Ethyl,
- R⁷: für Wasserstoff oder Methyl,
- R⁸: für Wasserstoff oder Methyl,
- R⁹: für Wasserstoff oder Methyl,
- R¹¹: für Wasserstoff oder -CH₂-NH₂,
- R¹²: für Wasserstoff, Methyl, Amino, Methylamino, Aminomethyl oder Ethylaminomethyl,
- R¹³: für Wasserstoff, Hydroxy oder Methoxy,
- R¹⁵: für Wasserstoff oder Methyl,
- R¹⁶: für Wasserstoff oder Methyl,
- R¹⁷: für Wasserstoff oder Methyl,
- R¹⁸: für 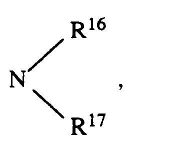
- R¹⁹: für Wasserstoff oder Methyl,
- R²⁰: für 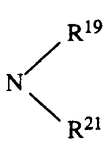 steht,
worin
- R²¹: Wasserstoff oder Methyl bedeutet,
- A: für CH₂, O oder für eine direkte Bindung steht und
- n: für 1 steht.

Weiterhin wurde gefunden, daß man die Verbindungen der Formel (I) erhält, wenn man eine Verbindung der Formel (II) 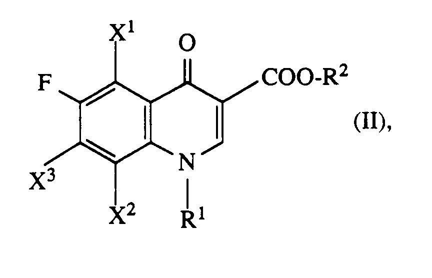 in welcher
- R¹, R², X¹ und X²: die oben angegebene Bedeutung haben und
- X³: für Halogen, insbesondere Fluor oder Chlor, steht,
mit Verbindungen der Formel (III)

Y-H (III),

in welcher
- Y: die oben angegebene Bedeutung hat,
gegebenenfalls in Gegenwart von Säurefängern umsetzt.

Verwendet man beispielsweise 1-Cyclopropyl-8-ethinyl-6,7-difluor-1,4-dihydro-4-oxo-3-chinolincarbonsäure und 1-Methylpiperazin als Ausgangsverbindungen, so kann der Reaktionsverlauf durch das folgende Formelschema wiedergegeben werden:

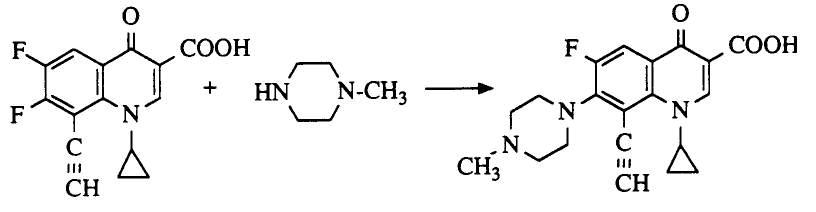

Die 8-(1-Chlorvinyl)-chinoloncarbonsäuren werden auch durch Umsetzung der 8-Ethinyl-chinoloncarbonsäuren mit Salzsäure bei Temperaturen von 10°C bis 100°C, vorzugsweise 20°C bis 60°C, erhalten.

Die als Ausgangsverbindungen verwendeten Verbindungen der Formel (II) sind neu. Sie können hergestellt werden, indem man Chinolincarbonsäure-Derivate der Formel (IV) 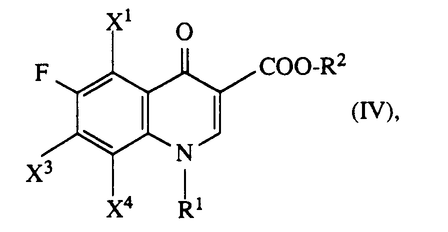 in welcher
- R¹, R², X¹ und X³: die oben angegebene Bedeutung haben und
- X⁴: für Halogen, insbesondere Jod, Brom oder Chlor, steht,
mit metallorganischen Vinyl- oder Alkinylverbindungen der Formel (V)

M-X² (V),

in welcher
- X²: die oben angegebene Bedeutung hat und
- M: für SnR'₃, ZnX', B(OR'')₂,
worin
- R': C₁-C₄-Alkyl,
- R'': Wasserstoff oder C₁-C₄-Alkyl und
- X': Brom oder Chlor bedeutet,
steht,
in Gegenwart von Übergangsmetall-Katalysatoren umsetzt und gegebenenfalls vorhandene Schutzgruppen abspaltet.

Die für die Kupplungsreaktion benötigten metallorganischen Vinyl- und Alkinylverbindungen sind entweder bekannt oder können nach literaturbekannten Methoden synthetisiert werden. So lassen sich zum Beispiel Vinyl-trialkylzinn-Verbindungen aus den entsprechenden Vinyliodiden, -bromiden oder -chloriden dadurch herstellen, daß man durch Umsetzung mit Magnesium die Vinylgrignard-Verbindungen gewinnt und diese mit einem Trialkylzinnchlorid zu den gewünschten Vinylzinn-Derivaten reagieren läßt.

Metallorganische Alkinylverbindungen lassen sich in bekannter Weise zum Beispiel dadurch darstellen, daß man das 1-Alkin bei Temperaturen zwischen -20 und - 78°C in einem aprotischen Lösungsmittel wie zum Beispiel Tetrahydrofuran mit n-Butyl-, sec-Butyl- oder tert-Butyllithium metalliert und anschließend mit einer Metallhalogen-Verbindung wie zum Beispiel Zinkchlorid, Magnesiumbromid, Kupferiodid oder Trialkylzinnchlorid umsetzt. Bevorzugt ist die Umsetzung bei -78°C. Außer dem bevorzugten Lösungsmittel Tetrahydrofuran sind auch andere Ether wie Diethylether, Dipropylether oder tert-Butyl-methyl-ether oder Mischungen solcher Ether mit aprotischen aliphatischen oder aromatischen Lösungsmitteln wie n-Hexan oder Toluol möglich. Sowohl bei den Vinyl- als auch bei den Alkinylderivaten sind die Zinkchlorid- und Trialkylzinn-Derivate bevorzugt. Unter "Alkyl" wird bei den Trialkylzinnverbindungen C₁- bis C₆-Alkyl verstanden; bevorzugt sind Methyl und n-Butyl.

Trialkylvinylzinn-Verbindungen können nach literaturbekannten Methoden auch durch Hydrostannylierung von Alkinen mit Trialkylzinnhydriden in Gegenwart von Übergangsmetallkatalysatoren erhalten werden (J. Org. Chem. 55 (1990) 1857-1867).

Die metallorganischen Vinyl- und Alkinylverbindungen werden mit 8-Halogen-chinoloncarbonsäure-Derivaten der allgemeinen Formel (IV) in Gegenwart eines geeigneten Katalysators nach im Prinzip bekannten Verfahren umgesetzt. "Halogen" steht hierbei für Jod, Brom oder Chlor; bevorzugt sind Brom und Chlor, besonders bevorzugt ist Brom.

Als Katalysatoren kommen beispielsweise Übergangsmetallverbindungen der Metalle Kobalt, Ruthenium, Rhodium, Iridium, Nickel, Palladium oder Platin in Frage. Bevorzugt sind Verbindungen der Metalle Platin, Palladium und Nickel, besonders bevorzugt ist Palladium. Solche Übergangsmetalle können in Form ihrer Salze wie zum Beispiel als NiCl₂, PdCl₂ oder Pd(OAc)₂, oder in Form von Komplexen mit geeigneten Liganden eingesetzt werden. Bevorzugt ist die Verwendung von Komplexen. Als Liganden werden bevorzugt Phosphine wie zum Beispiel Triphenylphosphin, Tri(o-tolyl)phosphin, Trimethylphosphin, Tributylphosphin und Tri(2-furyl)phosphin eingesetzt, bevorzugt ist Triphenylphosphin. Als bevorzugte Komplexkatalysatoren seien Bis(triphenylphosphin)nickel(II)-chlorid, Bis(triphenylphosphin)palladium(II)-chlorid, Tris(triphenylphosphin)palladium(0) und Tetrakis(triphenylphosphin)palladium(0) genannt.

Die Komplexkatalysatoren werden in Anteilen von 0,1 bis 20 mol-%, bezogen auf eingesetzten 8-Halogen-chinoloncarbonsäureester, eingesetzt; bevorzugt sind Anteile von 0,5 bis 10 mol-%, ganz besonders bevorzugt Anteile von 1 bis 5 mol-%.

Die Kupplungsreaktionen werden in geeigneten inerten Lösungsmitteln wie beispielsweise Benzol, Toluol, Xylol, Dimethylformamid, Dimethylacetamid, Dimethoxyethan oder Gemischen solcher Lösungsmittel durchgeführt; bevorzugt sind Dimethylformamid und Toluol. Die Lösungsmittel werden vor Gebrauch nach bekannten Verfahren getrocknet und luftfrei gemacht.

Die Kupplungsreaktionen werden bei Temperaturen zwischen 20 und 200°C durchgeführt; bevorzugt sind Temperaturen zwischen 50 und 180°C.

Die Dauer der Umsetzung richtet sich nach der Reaktivität der Edukte und beträgt im allgemeinen zwischen 2 und 40 Stunden; bevorzugt sind Reakfionszeiten zwischen 4 und 24 Stunden.

Die Umsetzungen werden unter einer Schutzgasatmosphäre durchgeführt. Als Schutzgas kommen inerte Gase wie beispielsweise Helium, Argon oder Stickstoff in Frage; bevorzugt ist Stickstoff. Die Kupplungsreaktion wird im allgemeinen bei Normaldruck durchgeführt. Es ist jedoch selbstverständlich auch möglich, die Reaktion bei vermindertem oder erhöhtem Druck durchzuführen.

Die als Ausgangsverbindungen verwendeten Amine der Formel (III) sind zum größten Teil bekannt. Chirale Amine können sowohl als Racemate als auch als enantiomerenreine oder diastereomerenreine Verbindungen eingesetzt werden. Als Beispiele seien genannt:
Piperazin,
1-Methylpiperazin,
1-Ethylpiperazin,
1-(2-Hydroxyethyl)-piperazin,
3-Methylpiperazin,
cis-2,6-Dimethyl-piperazin,
cis-2,3-Dimethyl-piperazin,
1,2-Dimethylpiperazin,
1-Cyclopropyl-piperazin,
2-Phenyl-piperazin,
2-(4-Pyridyl)-piperazin,
2-(2-Thienyl)-piperazin,
1,4-Diazabicyclo[3.2.1]octan,
8-Methyl-3,8-diazabicyclo[3.2.1]octan-Dihydrochlorid,
3-Methyl-3,8-diazabicyclo[3.2.1]octan-Dihydrochlorid,
2,5-Diazabicyclo[2.2.1]heptan-Dihydrochlorid,
2-Methyl-2,5-diazabicyclo[2.2.1]heptan-Dihydrochlorid,
2,5-Diazabicyclo[2.2.2]octan-Dihydrochlorid,
2-Methyl-2,5-diazabicyclo[2.2.2]octan-Dihydrochlorid,
1,4-Diazabicyclo[3.1.1]heptan,
Morpholin,
2,6-Dimethyl-morpholin,
2-Aminomethyl-morpholin,
2-tert.-Butoxycarbonylaminomethyl-morpholin,
2-Methylaminomethyl-morpholin,
2-Dimethylaminomethyl-morpholin,
Imidazol,
4-Methyl-imidazol,
Pyrrol,
3-Aminomethyl-2,5-dihydro-pyrrol,
3-Aminomethyl-4-methyl-2,5-dihydro-pyrrol,
3-(1-Aminoethyl)-2,5-dihydro-pyrrol,
3-Amino-azetidin,
3-tert.-Butoxycarbonylamino-azetidin,
3-tert.-Butoxycarbonylamino-2-methyl-azetidin,
3-tert.-Butoxycarbonylamino-3-methyl-azetidin,
3-tert.-Butoxycarbonylaminomethyl-azetidin,
Pyrrolidin,
3-Methylpyrrolidin,
3-Amino-pyrrolidin,
3-tert.-Butoxycarbonylamino-pyrrolidin,
3-(2,2-Dimethyl-propylidenamino)-pyrrolidin,
3-Methylamino-pyrrolidin,
3-Dimethylamino-pyrrolidin,
3-Aminomethyl-pyrrolidin,
3-tert.-Butoxycarbonylaminomethyl-pyrrolidin,
4-Chlor-3-tert.-butoxycarbonylaminomethyl-pyrrolidin,
3-tert.-Butoxycarbonylaminomethyl-3-methyl-pyrrolidin,
3-tert.-Butoxycarbonylamino-4-methyl-pyrrolidin,
3-tert.-Butoxycarbonylaminomethyl-3-methoxy-pyrrolidin,
3-Methylaminomethyl-pyrrolidin,
3-Ethylaminomethyl-pyrrolidin,
4-tert.-Butoxycarbonylamino-2-methyl-pyrrolidin,
2-Methyl-3-methylamino-pyrrolidin,
2-Methyl-4-methylamino-pyrrolidin,
3-(2-Hydroxyethylamino)-pyrrolidin,
3-Hydroxy-pyrrolidin,
3-Hydroxymethyl-pyrrolidin,
4-Amino-3-hydroxy-pyrrolidin,
3-Hydroxy-4-methylamino-pyrrolidin,
3-tert.-Butoxycarbonylamino-4-methoxy-pyrrolidin,
3-Methylaminomethyl-3-hydroxy-pyrrolidin,
3-Dimethylaminomethyl-3-hydroxy-pyrrolidin,
3-Diethylaminomethyl-3-hydroxy-pyrrolidin,
3-tert.-Butylaminomethyl-3-hydroxy-pyrrolidin,
3-Methylamino-4-hydroxymethyl-pyrrolidin,
4-Methoxy-3-methylamino-pyrrolidin,
3-Methoxy-3-methylaminomethyl-pyrrolidin,
3-Amino-4-methoxy-2-methyl-pyrrolidin,
3-tert.-Butoxycarbonylamino-3-methyl-pyrrolidin,
3-Methyl-4-tert.-butoxycarbonylaminomethyl-pyrrolidin,
3-Methoxy-4-tert.-butoxycarbonylaminomethyl-pyrrolidin,
3-(1-Imidazolyl)-pyrrolidin,
6-Hydroxy-3-azabicyclo[3.3.0]octan,
6-Amino-3-azabicyclo[3.3.0]octan,
1-Amino-3-azabicyclo[3.3.0]octan,
1-Aminomethyl-3-azabicyclo(3.3.0]octan,
1-Ethylaminomethyl-3-azabicyclo[3.3.0]octan,
6-Amino-3-azabicyclo[4.3.0]nonan,
3-Amino-4-methylen-pyrrolidin,
7-Amino-5-azaspiro[2.4]heptan,
3,7-Diazabicyclo[3.3.0]octan,
3-Methyl-3,7-diazabicyclo[3.3.0]octan,
2,8-Diazabicyclo[4.3.0]nonan,
2-Methyl-2,8-diazabicyclo[4.3.0]nonan,
3-Methyl-3,8-diazabicyclo[4.3.0]nonan,
2-Oxa-5,8-diazabicyclo[4.3.0]nonan,
5-Methyl-2-oxa-5,8-diazabicyclo[4.3.0]nonan,
2,7-Diazabicyclo[3.3.0]octan,
2-Methyl-2,7-diazabicyclo[3.3.0]octan,
3-Methyl-2,7-diazabicyclo[3.3.0]octan,
4-Methyl-2,7-diazabicyclo[3.3.0]octan,
5-Methyl-2,7-diazabicyclo[3.3.0]octan-7-carbonsäure-tert.-butylester,
7-Methyl-2,7-diazabicyclo[3.3.0]octan.
8-Methyl-2,7-diazabicyclo[3.3.0]octan,
7,8-Dimethyl-2,7-diazabicyclo[3.3.0]octan,
2,3-Dimethyl-2,7-diazabicyclo[3.3.0]octan,
2,8-Dimethyl-2,7-diazabicyclo[3.3.0]octan,
1,4-Diazatricyclo[6.2.0.0^{2,6}]decan,
1,4-Diazatricyclo[6.3.0.0^{2,6}]undecan,
2,7-Diazaspiro[4.4]nonan,
2-Methyl-2,7-diazaspiro[4.4]nonan,
4-Amino-1,3,3a,4,7,7a-hexahydroisoindol,
4-Methylamino-1,3,3a,4,7,7a-hexahydroisoindol,
5-Methyl-4-methylamino-1,3,3a,4,7,7a-hexahydroisoindol,
6-Methyl-4-methylamino-1,3,3a,4,7,7a-hexahydroisoindol,
7-Methyl-4-methylamino-1,3,3a,4,7,7a-hexahydroisoindol,
7a-Methyl-4-methylamino-1,3,3a,4,7,7a-hexahydroisoindol,
6,7-Dimethyl-4-methylamino-1,3,3a,4,7,7a-hexahydroisoindol,
4-Dimethylamino-1,3,3a,4,7,7a-hexahydroisoindol,
4-Ethylamino-1,3,3a,4,7,7a-hexahydroisoindol,
4-Aminomethyl-1,3,3a,4,7,7a-hexahydroisoindol,
4-Methylaminomethyl-1,3,3a,4,7,7a-hexahydroisoindol,
4-Hydroxy-1,3,3a,4,7,7a-hexahydroisoindol,
2,3,4,5,6,7-Hexahydro-1H-pyrrolo[3,4-c]pyridin,
5-Methyl-2,3,4,5,6,7-hexahydro-1H-pyrrolo[3,4-c]pyridin,
5-Ethyl-2,3,4,5.6,7-hexahydro-1H-pyrrolo[3,4-c]pyridin,
5-(tert.-Butoxycarbonyl)-2,3,4,5,6,7-hexahydro-1H-pyrrolo[3,4-c]pyridin.

Die substituierten 1,3,3a,4,7,7a-Hexahydro-isoindole sind überwiegend neu. Sie können zum Beispiel durch Diels-Alder-Reaktion von Dienen der Formel (1) 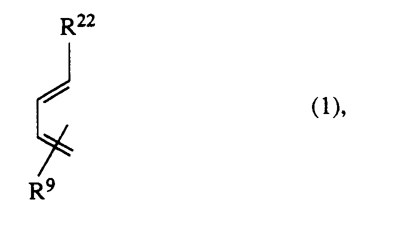 wobei R⁹ die oben angegebene Bedeutung hat und R²² entweder mit R²⁰ identisch ist oder eine funktionelle Gruppe darstellt, die in R²⁰ umgewandelt werden kann, mit Dienophilen der Formel (2), 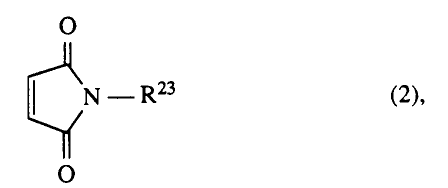 in welcher R²³ Wasserstoff oder eine Schutzgruppe wie Trimethylsilyl, Benzyl, C₁-C₄-Alkylphenylmethyl, Methoxybenzyl oder Benzylhydryl bedeuten, und der nachfolgenden Reduktion der Carbonylgruppen sowie gegebenenfalls der Abspaltung der Schutzgruppe erhalten werden.

Für die Diels-Alder-Reaktion kommen als Verdünnungsmittel alle inerten organischen Lösungsmittel in Frage. Hierzu gehören vorzugsweise Ether, wie Diisopropylether, Di-n-butylether, Dimethoxyethan, Tetrahydrofuran und Anisol, Kohlenwasserstoffe, wie z.B. Hexan, Methylcyclohexan, Toluol, Xylol und Mesitylen und halogenierte Kohlenwasserstoffe, wie z.B. Chloroform, 1,2-Dichlorethan und Chlorbenzol. Die Diels-Alder-Reaktion kann aber auch ohne Lösungsmittel durchgeführt werden.

Die Reaktionstemperaturen können in einem größeren Bereich variiert werden. Im allgemeinen arbeitet man zwischen etwa -20°C und +200°C, vorzugsweise zwischen -20°C und +150°C. Die Diels-Alder-Reaktion wird normalerweise bei Normaldruck durchgeführt. Zur Beschleunigung der Reaktion können aber auch Drucke bis zu 1,5 GPa eingesetzt werden.

Die Reduktion der Carbonylgruppen kann mit komplexen Hydriden erreicht werden. Als Hydride können z.B. Lithiumaluminiumhydrid, Lithiumborhydride, Lithiumtriethylborhydrid. Natrium-bis-[2-methoxyethoxy]-aluminiumhydrid oder Natriumborhydrid in Gegenwart von Lewis-Säure-Katalysatoren wie Chlortrimethylsilan, Bortrifluorid-Etherat oder Aluminiumchlorid eingesetzt werden.

Als Verdünnungsmittel können Ether, wie z.B. Diethylether, Tetrahydrofuran, Dioxan oder Dimethoxyethan und Kohlenwasserstoffe, wie z.B. Hexan, Methylcyclohexan und Toluol oder auch deren Gemische verwendet werden.

Die Reaktionstemperaturen können im Bereich zwischen -40 und +180°C, vorzugsweise zwischen 0° und 140°C, variiert werden. Die Reduktion wird im allgemeinen unter Normaldruck durchgeführt, sie kann aber auch unter vermindertem Druck oder unter Überdruck durchgeführt werden.

Die Anwendung von Drucken zwischen 100 und 1000 kPa empfiehlt sich, um mit leicht siedenden Lösungsmitteln höhere Reaktionstemperaturen zu erreichen.

Die komplexen Hydride werden mindestens in einer der Stöchiometrie der Reduktion entsprechenden Menge eingesetzt. Im allgemeinen wird aber ein Überschuß, vorzugsweise zwischen 30 und 300 %, eingesetzt.

Die Abspaltung einer eventuellen Schutzgruppe erfolgt nach den allgemein bekannten Methoden der Schutzgruppenchemie (vergl. z.B. T.W. Greene, "Protective Groups in Organic Synthesis", John Wiley & Sons, New York 1981).

Die Ausgangsstoffe der Formel (1) und (2) sind bekannt oder können nach allgemein bekannten Methoden der organischen Chemie hergestellt werden [vergl. z.B. J. Am. Chem. Soc. 100, 5179 (1978), J. Org. Chem. 43, 2164 (1978), DE 39 27 115, J. Org. Chem. 40, 24 (1975)].

Verwendet man beispielsweise 1-(tert.-Butyloxycarbonylamino)-1,3-butadien und Maleinimid als Ausgangsmaterialien und Lithiumaluminiumhydrid als Reduktionsmittel, so kann der Reaktionsablauf durch das folgende Formelschema wiedergegeben werden:

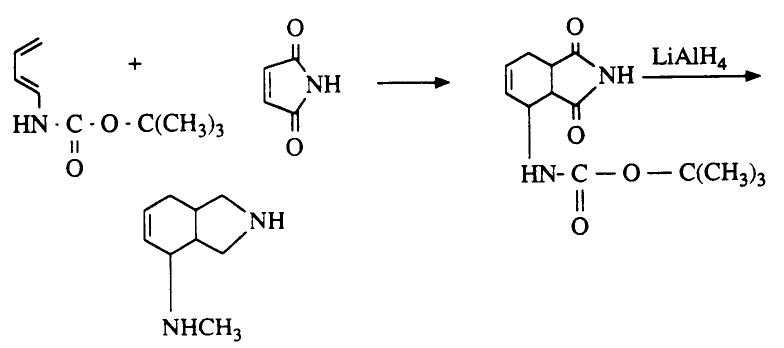

In einer besonderen Ausführungsform des Herstellungsverfahrens können alle Stufen bei Verwendung eines geeigneten Lösungsmittels, wie z.B. Tetrahydrofuran, ohne Isolierung der Zwischenprodukte durchgeführt werden. Verwendet man z.B. 1-(tert.-Butyloxycarbonylamino)-1,3-pentadien und N-Trimethylsilyl-maleinimid als Ausgangsmaterialien, so kann der Reaktionsablauf durch das folgende Formelschema wiedergegeben werden:

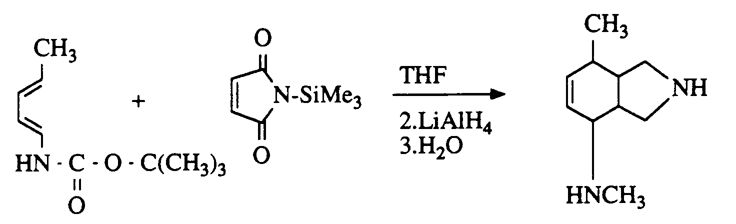

Durch NMR-Spektroskopie kann in diesem Fall nachgewiesen werden, daß alle Substituenten am 6-Ring cis-Anordnung zueinander aufweisen.

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol der Verbindung (III) ein.

Freie Aminogruppen können während der Umsetzung durch eine geeignete Aminoschutzgruppe, zum Beispiel durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. McOmie, Protective Groups in Organic Chemistry (1973), Seite 43).

Die erfindungsgemäßen Ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können auch die in der folgenden Tabelle aufgeführten Wirkstoffe hergestellt werden. Im Falle von chiralen Verbindungen können diese sowohl als diastereomere Mischungen oder jeweils als diastereomer oder enantiomer reine Verbindungen hergestellt und verwendet werden.

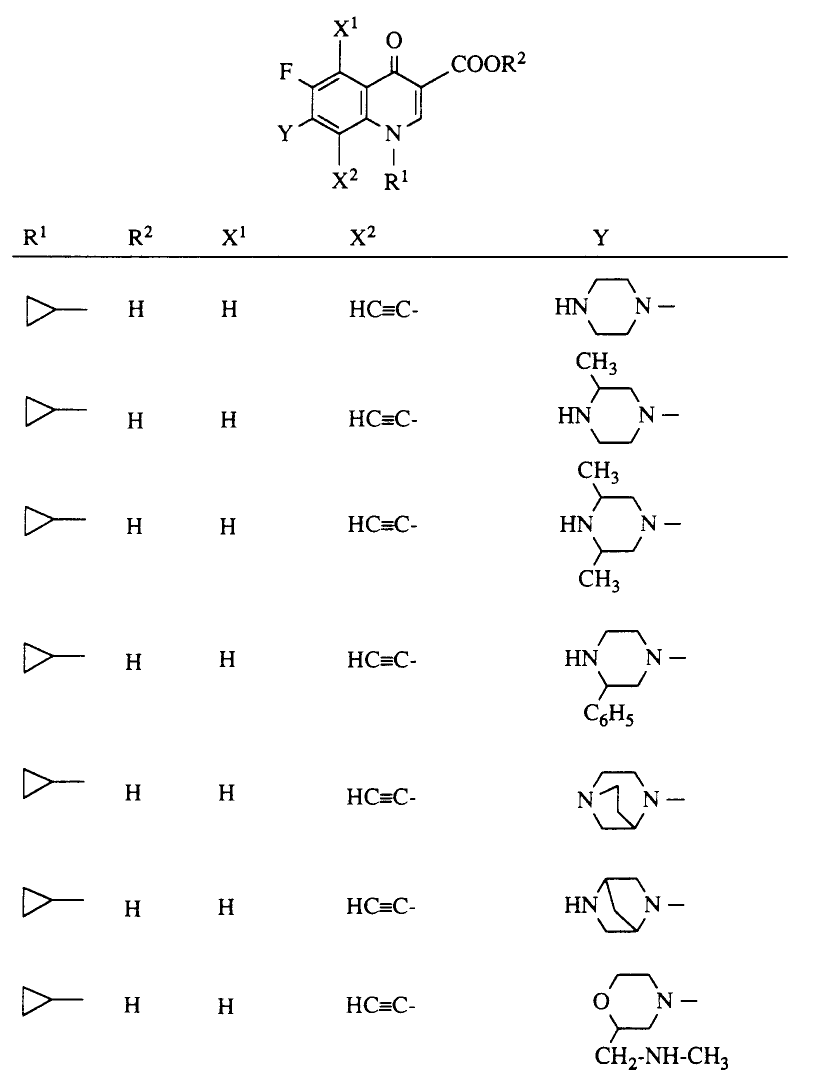

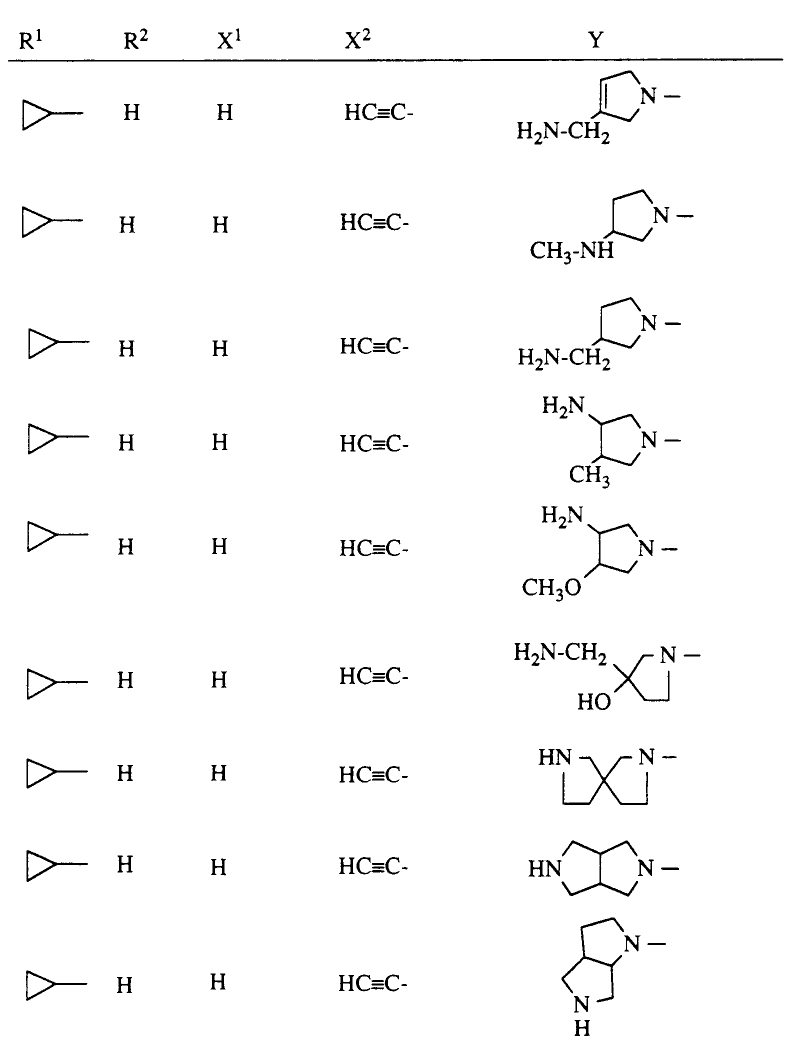

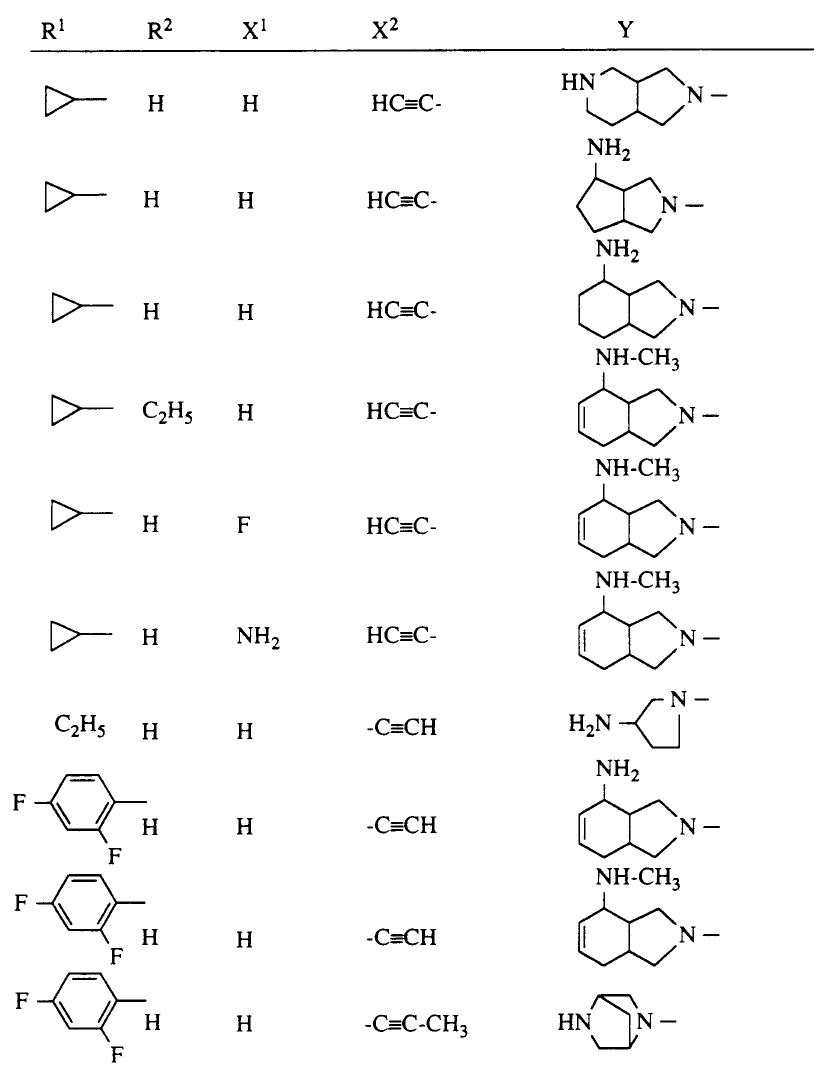

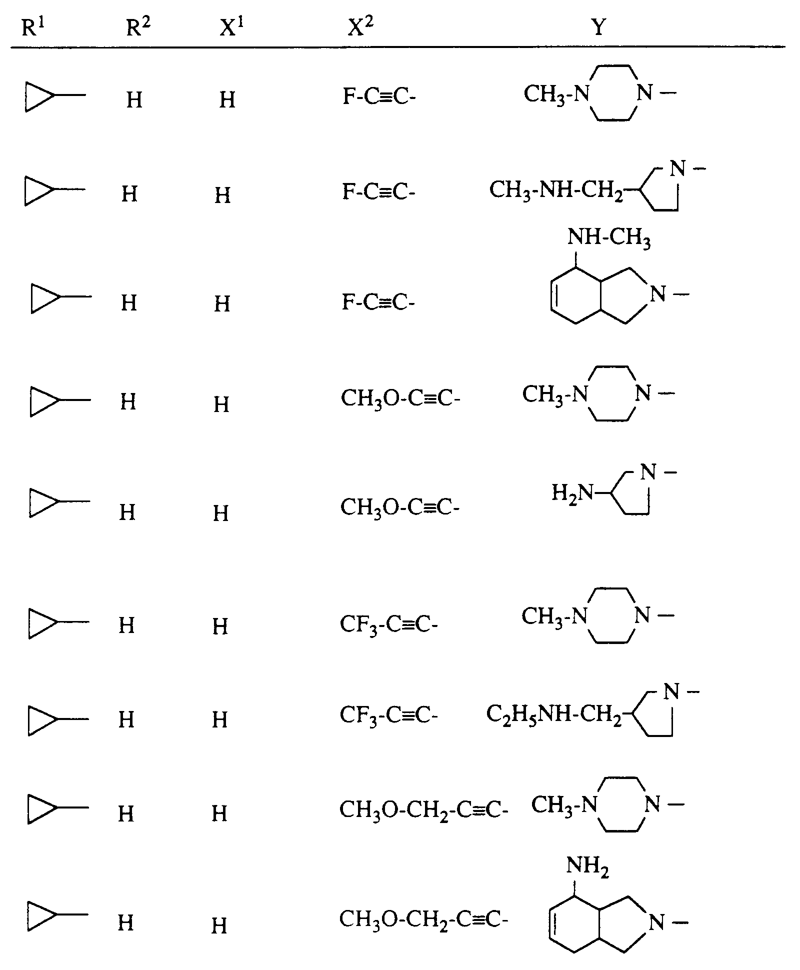

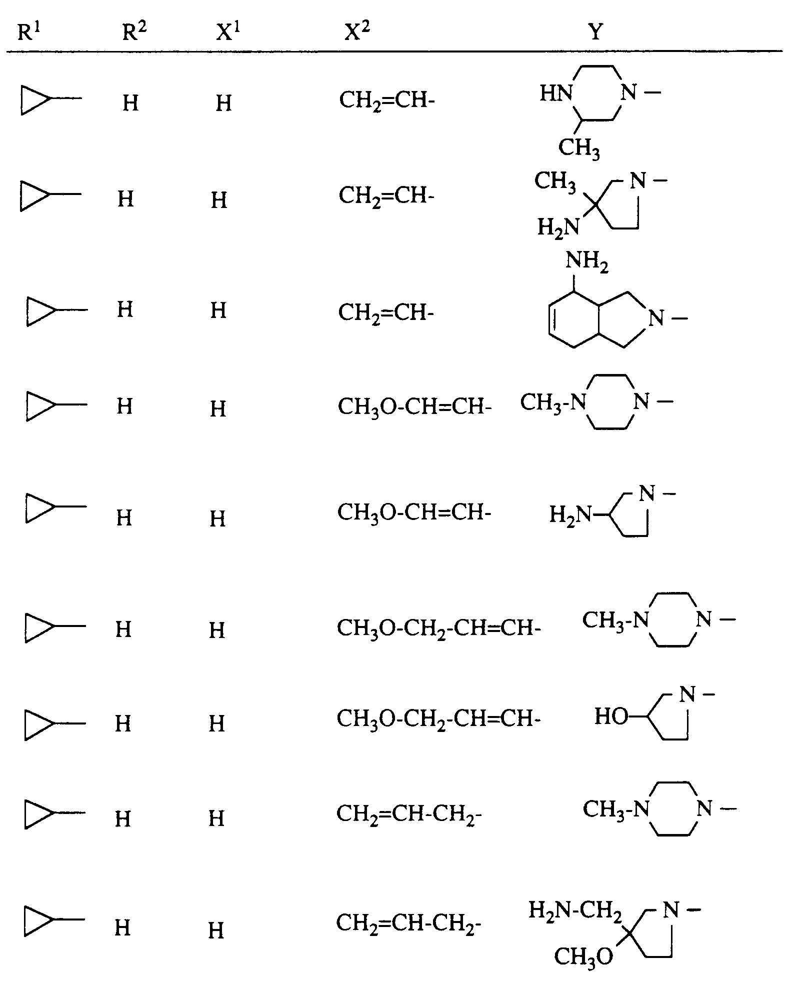

Die erfindungsgemäßen Verbindungen wirken stark antibiotisch und zeigen bei geringer Toxizität ein breites antibakterielles Spektrum gegen grampositive und gramnegative Keime, insbesondere gegen Enterobakterien; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen weit unterhalb von Konzentrationen bisher bekannter Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Pseudomonas aeruginosa, Enterococcus faecalis und Escherichia coli beobachtet werden.

Auch gegenüber Bakterien, die gegenüber vergleichbaren Substanzen als weniger empfindlich eingestuft werden, insbesondere resistenten Staphylococcus aureus, Escherichia coli, Pseudomonas aeruginosa und Enterococcus faecalis zeigen die erfindungsgemäßen Verbindungen überraschende Wirkungssteigerungen.

Auf Grund der hohen Wirksamkeit der erfindungsgemäßen Verbindungen gegen Bakterien und bakterienähnliche Mikroorganismen sind sie besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich zu Ciprofloxacin aufgeführt.

**Tabelle**

| MHK-Werte | | | | | | | | | | | |
|---|---|---|---|---|---|---|---|---|---|---|---|
| Teststamm: | | Beispiel | | | | | | | | | Ciprofloxacin |
| | | 1a | 5 | 6 | 8 | 9 | 14 | 15 | 18 | 22 | |
| E. coli | Neumann | 0,02 | 0,02 | 0,02 | 0,13 | 0,25 | 0,13 | 0,02 | 0,03 | 0,03 | 0,02 |
| Micrococcus luteus | 9341 | | 0,5 | 0,25 | 1 | 2 | 0,25 | 8 | 1 | 0,5 | 2 |
| Staphylococcus aureus | ICB 25701 | 4 | 1 | 1 | 1 | 4 | 0,25 | 32 | 4 | 4 | 16 |
| | 1756 | 0,13 | 0,03 | 0,06 | 0,06 | 0,13 | 0,06 | 0,02 | 0,13 | 0,06 | 0,25 |
| | 133 | 0,13 | 0,13 | 0,06 | 0,06 | 0,13 | 0,06 | 0,02 | 0,13 | 0,13 | 0,25 |
| Enterococcus faecalis | 27101 | 0,25 | 0,13 | 0,13 | 0,25 | 0,5 | 0,13 | 1 | 0,25 | 0,13 | 0,5 |
| | 9790 | 0,25 | 0,13 | 0,13 | 0,25 | 1 | 0,13 | 1 | 0,25 | 0,25 | 0,5 |
| Acinetobacter calcoaceticus | 14068 | | 0,03 | 0,13 | 0,25 | 0,5 | 0,13 | 0,03 | 0,03 | 0,25 | 0,25 |

### Herstellung der Zwischenprodukte

### Beispiel Z1

### 1-Cyclopropyl-6,7-difluor-1,4-dihydro-4-oxo-8-vinyl-3-chinolincarbonsäure-ethylester

3,72 g 8-Brom-1-cyclopropyl-6,7-difluor-1,4-dihydro-4-oxo-3-chinolincarbonsäure-ethylester, 4,4 g Tributylvinylstannan und 0,46 g Tetrakis(triphenylphosphin)palladium(0) werden in 40 ml absolutem Toluol 2 bis 3 Stunden unter einer Stickstoffatmosphäre zum Rückfluß erhitzt. Es wird heiß filtriert, das bei Raumtemperatur ausgefallene Produkt abgesaugt, mit Toluol nachgewaschen und getrocknet. Man erhält 2,55 g 1-Cyclopropyl-6,7-difluor-1,4-dihydro-4-oxo-8-vinyl-3-chinolincarbonsäureethylester(79 % der Theorie).
Schmelzpunkt: 178-179°C.

### Beispiel Z2

### 1-Cyclopropyl-6,7-difluor-1,4-dihydro-4-oxo-8-vinyl-3-chinolincarbonsäure

0,9 g 1-Cyclopropyl-6,7-difluor-1,4-dihydro-4-oxo-8-vinyl-3-chinolincarbonsäure-ethylester werden in einem Gemisch aus 8 ml Eisessig, 0,6 ml Wasser und 0,2 ml konzentrierter Schwefelsäure 4 Stunden zum Rückfluß erhitzt. Das Reaktionsgemisch wird dann bei Rückflußtemperatur mit 10 ml Wasser versetzt. Der Feststoff wird bei Raumtemperatur abgesaugt, mit Wasser gewaschen und getrocknet. Es werden 0,58 g 1-Cyclopropyl-6,7-difluor-1,4-dihydro-4-oxo-8-vinyl-3-chinolincarbonsäure (71 % der Theorie) erhalten.
Schmelzpunkt: 182-184°C.

### Beispiel Z3

### 1-Cyclopropyl-6,7-difluor-1,4-dihydro-8-(trimethylsilyl-ethinyl)-4-oxo-3-chinolincarbonsäure-ethylester

22,2 g 8-Brom-1-cyclopropyl-6,7-difluor-1,4-dihydro-4-oxo-3-chinolincarbonsäure-ethylester, 30,2 g Tributylstannyl-trimethylsilyl-acetylen und 3,48 g Tetrakis(triphenylphosphin)palladium(0) werden für 3 Stunden in 300 ml absolutem Toluol unter einer Stickstoffatmosphäre zum Rückfluß erhitzt. Nach Abkühlen des Reaktionsgemisches auf ca. -18°C wird abgesaugt, der Feststoff mit Toluol nachgewaschen und getrocknet. Man erhält 18,8 g 1-Cyclopropyl-6,7-difluor-1,4-dihydro-8-(trimethylsilylethinyl)-4-oxo-3-chinolincarbonsäure-ethylester (80 % der Theorie).
Schmelzpunkt: 171-172°C.

### Beispiel Z4

### 1-Cyclopropyl-8-ethinyl-6,7-difluor-1,4-dihydro-4-oxo-3-chinolincarbonsäure-ethylester

18,8 g 1-Cyclopropyl-6,7-difluor-1,4-dihydro-8-(trimethylsilylethinyl)-4-oxo-3-chinolincarbonsäure-ethylester und 9,7 g Kaliumfluorid werden in einem Gemisch von 300 ml Dimethylformamid, 200 ml Chloroform und 15 ml Wasser 3 Stunden bei Raumtemperatur gerührt. Danach wird filtriert, das Filtrat mit 120 ml Wasser versetzt und mit verdünnter wäßriger Salzsäure sauer gestellt. Nach Ausschütteln mit Chloroform wird die organische Phase über Natriumsulfat getrocknet und eingeengt. Der erhaltene Rückstand wird aus Methanol umkristallisiert. Es werden so 9 g 1-Cyclopropyl-8-ethinyl-6,7-difluor-1,4-dihydro-4-oxo-3-chinolincarbonsäure-ethylester (59 % der Theorie) erhalten.
Schmelzpunkt: 186-187°C.

### Beispiel Z5

### 1-Cyclopropyl-8-ethinyl-6,7-difluor-1,4-dihydro-4-oxo-3-chinolincarbonsäure

10,3 g 1-Cyclopropyl-8-ethinyl-6,7-difluor-1,4-dihydro-4-oxo-3-chinolincarbonsäure-ethylester werden in einem Gemisch aus 100 ml Eisessig, 8 ml Wasser und 3 ml konzentrierter Schwefelsäure 4 Stunden unter Rückfluß erhitzt. Nach Abkühlen auf Raumtemperatur wird der Feststoff abgesaugt, mit Wasser gewaschen und getrocknet. Man erhält so 5,7 g 1-Cyclopropyl-8-ethinyl-6,7-difluor-1-4-dihydro-4-oxo-3-chinolincarbonsäure (62 % der Theorie).
Schmelzpunkt: 233°C.

### Beispiel Z6

### 1-Cyclopropyl-6,7-difluor-8-(1-hexinyl)-1,4-dihydro-4-oxo-3-chinolincarbonsäure-ethylester

1,9 g 8-Brom-1-cyclopropyl-6,7-difluor-1,4-dihydro-4-oxo-3-chinolincarbonsäure-ethylester, 3,5 g 1-Tributylstannyl-hex-1-in und 0,29 g Tetrakis(triphenylphosphin)palladium(0) werden in 20 ml absolutem Toluol 8 Stunden unter einer Stickstoffatmosphäre zum Rückfluß erhitzt. Das Reaktionsgemisch wird eingeengt, der Rückstand mit 30 ml Hexan verrührt und der so erhaltene Feststoff aus Cyclohexan umkristallisiert. Man erhält 0,7 g 1-Cyclopropvl-6,7-difluor-8-(1-hexinyl)-1,4-dihydro-4-oxo-3-chinolincarbonsäure-ethylester (36 % der Theorie).
¹H-NMR (200 MHz, CDCl₃): δ 0,95 (t; 3 H), 1,1-1,7 (m; 11 H), 2,50 (t; 2 H), 4,1-4,3 (m; 1 H), 4,38 (q; 2 H), 8,14 (dd; 1 H), 8,56 (s; 1 H) ppm.

### Beispiel Z7

### 1-Cyclopropyl-6,7-difluor-8-(1-hexinyl)-1,4-dihydro-4-oxo-3-chinolincarbonsäure

0,7 g 1-Cyclopropyl-6,7-difluor-8-(1-hexinyl)-1,4-dihydro-4-oxo-3-chinolincarbonsäure-ethylester werden in einem Gemisch aus 6 ml Eisessig, 0,5 ml Wasser und 0,1 ml konzentrierter Schwefelsäure 3 Stunden zum Rückfluß erhitzt. Nach Versetzen des Reaktionsgemisches mit 100 ml Wasser wird der Feststoff abgesaugt und getrocknet. Es werden 0,5 g 1-Cyclopropyl-6,7-difluor-8-(1-hexinyl)-1,4-dihydro-4-oxo-3-chinolincarbonsäure erhalten (85 % der Theorie).
¹H-NMR (200 MHz, CDCl₃): δ 0,96 (t; 3 H), 1,1-1,7 (m; 8 H), 4,3-4,5 (m; 1 H), 8,20 (dd; 1 H), 8,85 (s; 1 H) ppm.
Schmelzpunkt: 118-121°C.

### Beispiel Z8

### 1-Cyclopropyl-6,7-difluor-1,4-dihydro-8(3,3-dimethylbutin-1-yl)-4-oxo-3-chinolincarbonsäure-ethylester

Entsprechend Beispiel Z6 erhält man mit 1-Tributylstannyl-3,3-dimethyl-but-1-in 0,87 g 1-Cyclopropyl-6,7-difluor-1,4-dihydro-8-(3,3-dimethylbutin-1-yl)-4-oxo-3-chinolincarbonsäure-ethylester (46 % der Theorie).
Schmelzpunkt: 170-172°C.

### Beispiel Z9

### 1-Cyclopropyl-6,7-difluor-1,4-dihydro-8-(3,3-dimethylbutin-1-yl)-4-oxo-3-chinolincarbonsäure

Die Verseifung von 0,75 g des Esters aus Beispiel Z8 entsprechend Beispiel Z7 ergibt 0,56 g 1-Cyclopropyl-6,7-difluor-1,4-dihydro-8-(3,3-dimethylbutin-1-yl)-4-oxo-3-chinolincarbonsäure (81 % der Theorie).
Schmelzpunkt: 199-201°C.

### Beispiel Z10

### 1-(2,4-Difluorphenyl)-6,7-difluor-1,4-dihydro-8-(trimethylsilylethinyl)-4-oxo-3-chinolincarbonsäure-ethylester

6,7 g 8-Brom-1-(2,4-difluorphenyl)-6,7-difluor-1,4-dihydro-4-oxo-3-chinolincarbonsäure-ethylester (Beispiel Z20), 10,8 g Triburylstannyl-trimethylsilyl-acetylen und 0,87 g Tetrakis(triphenylphosphin)-palladium(0) werden in 50 ml absolutem Toluol 24 Stunden unter einer Stickstoffatmosphäre zum Rückfluß erhitzt. Bei -18°C kristallisiert das Produkt aus dem Reaktionsgemisch aus. Man erhält 4,8 g 1-(2,4-Difluorphenyl)-6,7-difluor-1,4-dihydro-8-(trimethylsilylethinyl)-4-oxo-3-chinolincarbonsäure-ethylester (69 % der Theorie).
Schmelzpunkt: 173-174°C.

### Beispiel Z11

### 1-(2,4-Difluorphenyl)-8-ethinyl-6,7-difluor-1,4-dihydro-4-oxo-3-chinolincarbonsäure-ethylester

Eine Lösung von 4,6 g 1-(2,4-Difluorphenyl)-6,7-difluor-1,4-dihydro-8-(trimethylsilylethinyl)-4-oxo-3-chinolincarbonsäure-ethylester in 20 ml Chloroform wird bei Raumtemperatur zu einer Lösung von 2 g Kaliumfluorid in einem Lösungsmittelgemisch aus 3 ml Wasser, 25 ml Chloroform und 50 ml Dimethylformamid getropft. Es wird 1 Stunde bei ca. 20°C gerührt, dann die Reaktionsmischung mit weiterem Chloroform versetzt, mehrmals mit Wasser ausgeschüttelt, die organische Phase getrocknet und eingeengt. Der erhaltene Rückstand wird aus Methanol umkristallisiert. Es werden 3,4 g 1-(2,4-Difluorphenyl)-8-ethinyl-6,7-difluor-1,4-dihydro-4-oxo-3-chinolincarbonsäure-ethylester erhalten (87 % der Theorie).
Schmelzpunkt: 189°C.

### Beispiel Z12

### 1-(2,4-Difluorphenyl)-8-ethinyl-6,7-difluor-1,4-dihydro-4-oxo-3-chinolincarbonsäure

1,17 g 1-(2,4-Difluorphenyl)-8-ethinyl-6,7-difluor-1,4-dihydro-4-oxo-3-chinolincarbonsäure-ethylester werden in einem Gemisch aus 9 ml Eisessig, 0,75 ml Wasser und 0,2 ml konzentrierter Schwefelsäure 1 Stunde unter Rückfluß erhitzt. Der bei Raumtemperatur auskristallisierte Feststoff wird abgesaugt und getrocknet. Man erhält 0,98 g 1-(2,4-Difluorphenyl)-8-ethinyl-6,7-difluor-1,4-dihydro-4-oxo-3-chinolincarbonsäure (90 % der Theorie).
Schmelzpunkt: 220°C (Zersetzung).

### Beispiel Z13

### 1-Cyclopropyl-6,7-difluor-1,4-dihydro-4-oxo-8-(propin-1-yl)-3-chinolincarbonsäure-ethylester

7,5 g 8-Brom-1-cyclopropyl-6,7-difluor-1,4-dihydro-4-oxo-3-chinolincarbonsäure-ethylester, 9,1 g 1-Tributylstannyl-prop-1-in und 1,16 g Tetrakis(triphenylphosphin)palladium(0) werden in 80 ml absolutem Toluol für 8 Stunden unter einer Stickstoffatmosphäre zum Rückfluß erhitzt. Der bei -18°C auskristallisierte Feststoff wird abgesaugt und getrocknet. Man erhält 2,05 g 1-Cyclopropyl-6,7-difluor-1,4-dihydro-4-oxo-8-(propin-1-yl)-3-chinolincarbonsäure-ethylester (31 % der Theorie).
¹H-NMR (200 MHz, CDCl₃): δ 1,1-1,35 (m; 4 H), 1,40 (t; 3 H), 2,16 (d; 3 H), 4,1-4,3(m; 1 H), 4,35 (q; 2 H), 8,15 (dd; 1 H), 8,56 (s; 1 H) ppm.
Schmelzpunkt: 180-182°C

### Beispiel Z14

### 1-Cyclopropyl-6,7-difluor-1,4-dihydro4-oxo-8-(propin-1-yl)-3-chinolincarbonsäure

1,4 g 1-Cyclopropyl-6,7-difluor-1,4-dihydro-4-oxo-8-(propin-1-yl)-3-chinolincarbonsäure-ethylester werden in einem Gemisch aus 20 ml Eisessig, 1,5 ml Wasser und 0,5 ml konzentrierter Schwefelsäure 1 Stunde unter Rückfluß gekocht. Nach Versetzen mit ca. 10 ml Wasser wird der ausgefallene Feststoff isoliert und getrocknet. Es werden auf diese Weise 1,05 g 1-Cyclopropyl-6,7-difluor-1,4-dihydro-4-oxo-8-(propin-1-yl)-3-chinolincarbonsäure erhalten (82 % der Theorie).
¹H-NMR (200 MHz, CDCl₃): δ 1,4 (m; 4 H), 2,26 (d; 3 H), 4,4-4,6 (m; 1 H), 8,16 (dd; 1 H), 8,81 (s; 1 H) ppm.
Schmelzpunkt: 212-213°C.

### Beispiel Z15

### 1-Ethyl-6,7-difluor-1,4-dihydro-8-(trimethylsilylethinyl)-4-oxo-3-chinolincarbonsäure-ethylester

5,4 g 8-Brom-1-ethyl-6,7-difluor-1,4-dihydro-4-oxo-3-chinolincarbonsäure-ethylester (Beispiel Z22), 10,8 g Tributylstannyl-trimethylsilyl-acetylen und 0,87 g Tetrakis(triphenylphosphin)palladium(0) werden in 50 ml absolutem Toluol unter einer Stickstoffatmosphäre 24 Stunden zum Rückfluß erhitzt. Das Reaktionsgemisch wird eingeengt, der Rückstand in 100 ml Hexan verrührt, der resultierende Feststoff abgesaugt und getrocknet. Man erhält 4,53 g 1-Ethyl-6,7-difluor-1,4-dihydro-8-(trimethylsilylethinyl)-4-oxo-3-chinolincarbonsäure-ethylester (80 % der Theorie).
Schmelzpunkt: 151-152°C.

### Beispiel Z16

### 1-Cyclopropyl-6,7-difluor-1,4-dihydro-8-(trimethylsilylethinyl)-4-oxo-3-chinolincarbonsäure-ethylester

1,64 g 8-Chlor-1-cyclopropy1-6,7-difluor-1,4-dihydro-4-oxo-3-chinolincarbonsäure-ethylester, 3 g Tributylstannyl-trimethylsilyl-acetylen und 0,29 g Tetrakis(triphenylphosphin)palladium(0) werden in 20 ml absolutem Toluol 42 Stunden unter einer Stickstoffatmosphäre zum Rückfluß erhitzt. Das Reaktionsgemisch wird auf ca. -18°C abgekühlt und filtriert. Nach Trocknen des Filterrückstandes erhält man 0,74 g 1-Cyclopropyl-6,7-difluor-1,4-dihydro-8-(trimethylsilylethinyl)-4-oxo-3-chinolincarbonsäure-ethylester (38 % der Theorie).

### Beispiel Z17

### 1-Cyclopropyl-6,7-difluor-1,4-dihydro-8-(3-methyl-but-3-en-1-inyl)-4-oxo-3-chinolincarbonsäure-ethylester

1,86 g 8-Brom-1-cyclopropyl-6,7-difluor-1,4-dihydro-4-oxo-3-chinolincarbonsäure-ethylester, 2,8 g 1-Tributylstannyl-3-methyl-but-3-en-1-in und 0,29 g Tetrakis(triphenylphosphin)palladium(0) werden in 20 ml absolutem Toluol 6 Stunden unter einer Stickstoffatmosphäre zum Rückfluß erhitzt. Das Reaktionsgemisch wird heiß filtriert, eingeengt und der Rückstand mit Hexan verrührt. Nach Absaugen und Trocknen werden 1,43 g 1-Cyclopropyl-6,7-difluor-1,4-dihydro-8-(3-methyl-but-3-en-1-inyl)-4-oxo-3-chinolincarbonsäure-ethylester (80 % der Theorie) erhalten.
Schmelzpunkt: 169-171°C.

### Beispiel Z18

### 1-Cyclopropyl-6,7-difluor-1,4-dihydro-8-(3-methyl-but-3-en-1-inyl)-4-oxo-3-chinolincarbonsäure

0,715 g 1-Cyclopropyl-6,7-difluor-1,4-dihydro-8-(3-methyl-but-3-en-1-inyl)-4-oxo-3-chinolincarbonsäure-ethylester werden in einem Gemisch aus 10 ml Eisessig, 0,5 ml Wasser und 0,2 ml konzentrierter Schwefelsäure 1,5 Stunden zum Rückfluß erhitzt. Das Reaktionsgemisch wird in 100 ml Wasser gegeben. Der ausgefallene Feststoff wird abgesaugt, mit Wasser nachgewaschen und getrocknet. Man erhält 0,53 g 1-Cyclopropyl-6,7-difluor-1,4-dihydro-8-(3-methyl-but-3-en-1-inyl)-4-oxo-3-chinolincarbonsäure (80 % der Theorie).
Schmelzpunkt: 204-206°C.

### Beispiel Z19

### 2-(3-Brom-2,4,5-trifluor-benzoyl)-3-(2,4-difluorphenylamino)-acrylsäureethylester

40 g (0,1 mol) 2-(3-Brom-2,4,5-trifluor-benzoyl)-3-ethoxy-acrylsäureethylester werden in 180 ml Ethanol unter Eiskühlung mit 14,5 g (0,11 mol) 2,4-Difluor-anilin versetzt. Man läßt die Mischung über Nacht bei 10°C stehen, saugt den ausgefallenen Niederschlag ab, wäscht mit kaltem Ethanol und trocknet im Vakuum.
Ausbeute: 38 g (81 % der Theorie).
Schmelzpunkt: 102-103°C (unter Zersetzung) (aus Isopropanol).

### Beispiel Z20

### 8-Brom-1-(2,4-difluorphenyl)-6,7-difluor-1,4-dihydro-4-oxo-3-chinolincarbonsäure-ethylester

38 g (82 mmol) 2-(3-Brom-2,4,5-trifluor-benzoyl)-3(2,4-difluorphenylamino)acrylsäureethylester werden in 200 ml Dimethylformamid mit 7,6 g Natriumfluorid versetzt und 2 Stunden unter Rückfluß erhitzt. Die Mischung wird auf Eiswasser ausgegossen, der Niederschlag abgesaugt, gut mit Wasser gewaschen und bei 80°C im Umlufttrockenschrank getrocknet.
Ausbeute: 34,7 g (95 % der Theorie).
Schmelzpunkt: 208-210°C (unter Zersetzung) (aus Glykolmonomethylether).
Durch saure Verseifung dieses Esters erhält man 8-Brom-1-(2,4-difluorphenyl)-6,7-difluor-1,4-dihydro-4-oxo-3-chinolincarbonsäure vom Schmelzpunkt 210-221°C (unter Zersetzung).

### Beispiel Z21

### 2-(3-Brom-2,4,5-trifluor-benzoyl)-3-ethylamino-acrylsäureethylester

20 g (0,05 mol) 2-(3-Brom-2,4,5-trifluor-benzoyl)-3-ethoxy-acrylsäure-ethylester werden in 40 ml Ethanol unter Eiskühlung mit 5,5 g einer 50 %igen wäßrigen Ethylamin-Lösung versetzt. Man läßt die Mischung über Nacht bei 10°C stehen, versetzt die Suspension mit 200 ml Wasser, saugt den ausgefallenen Niederschlag ab, wäscht mit Wasser und trocknet bei 60°C im Vakuum.
Ausbeute: 17,3g (91 % der Theorie).
Schmelzpunkt: 101-102°C (unter Zersetzung) (aus Isopropanol).

### Beispiel Z22

### 8-Brom-1-ethyl-6,7-difluor-1,4-dihydro-4-oxo-3-chinolincarbonsäure-ethylester

16 g (42 mmol) 2-(3-Brom-2,4,5-trifluor-benzoyl)-3-ethylaminoacrylsäure-ethylester werden analog Beispiel Z20 umgesetzt.
Ausbeute: 14,6 g (96 % der Theorie).
Schmelzpunkt: 172-173°C (unter Zersetzung) (aus Glykolmonomethylether).
Durch saure Verseifung erhält man aus diesem Ester die 8-Brom-1-ethyl-6,7-difluor-1,4-dihydro-4-oxo-3-chinolincarbonsäure vom Schmelzpunkt 215-217°C (unter Zersetzung).

### Beispiel Z23

### 4-Methylamino-1,3,3a,4,7,7a-hexahydroisoindol

### Methode I:

14,4 g (60 mmol) 70 %iges 1-(tert.-Butyloxycarbonylamino)-1,3-butadien [J.Org.Chem. 43, 2164 (1978)] werden als Lösung in 30 ml absolutem Tetrahydrofuran zu vorgelegten 10,1 g (60 mmol) N-Trimethylsilylmaleinimid [J.Org.Chem. 40, 24 (1975)] in 30 ml absolutem Tetrahydrofuran getropft. Nach dem Abklingen der exothermen Reaktion wird noch 1 Stunde unter Rückflußkühlung gekocht.

Die erkaltete Reaktionsmischung wird dann unter Stickstoff zu vorgelegten 7,6 g (0,2 mol) Lithiumaluminiumhydrid in 200 ml absolutem Tetrahydrofuran getropft. Dann wird 14 Stunden unter Rückflußkühlung gekocht. Zu der erkalteten Reaktionsmischung werden dann nacheinander 7,6 g Wasser in 23 ml Tetrahydrofuran, 7,6 g 10 %ige Natronlauge und 22,8 g Wasser getropft. Die Salze werden abfiltriert und das Filtrat im Vakuum eingeengt. Der Rückstand (10,3 g) wird bei 87°C/0,8 mbar destilliert.

Das Destillat wird in 80 ml absolutem Pentan aufgenommen, filtriert und das Produkt durch Abkühlen auf -70°C kristallisiert.
Ausbeute: 3,3 g, Schmelzpunkt: 72-82°C.

Durch Behandeln mit äquimolarer Menge 2n-Salzsäure erhält man 4-Methylamino-1,3,3a,4,7,7a-hexahydro-isoindol-Dihydrochlorid vom Schmelzpunkt 265-268°C (aus Methanol).

### Methode II:

a) 4-(tert.-Butyloxycarbonylamino)-1,3-dioxo-1,3,3a,4,7,7a-hexahydroisoindol,
   48,0 g (0,5 mol) Maleinimid werden in 200 ml absolutem Tetrahydrofuran gelöst vorgelegt und 120 g (0,5 mol) ca. 70 %iges 1-(tert.-Butyloxycarbonylamino)-1,3-butadien als Lösung in 500 ml absolutem Tetrahydrofuran zugetropft, wobei die Temperatur bei 20 bis 30°C gehalten wird. Über Nacht wird bei Raumtemperatur nachgerührt. Dann wird eingeengt und aus Essigsäureethylester umkristallisiert. Es werden 57 g Produkt mit einem Schmelzpunkt von 177 bis 182°C erhalten. Aus der Mutterlauge werden weitere 13 g mit einem Schmelzpunkt von 158 bis 160°C erhalten.
b) 4-Methylamino-1,3,3a,4,7,7a-hexahydroisoindol
   27,1 g (0,71 mol) Lithiumaluminiumhydrid werden unter Stickstoff in 300 ml absolutem Tetrahydrofuran vorgelegt und eine Lösung von 57 g (0,21 mol) 4-(tert.-Butyloxycarbonylamino)-1,3-dioxo-1,3,3a,4,7,7a-hexahydroisoindol in 570 ml absolutem Tetrahydrofuran zugetropft Dann wird über Nacht unter Rückflußkühlung gekocht Nach dem Erkalten werden nacheinander 27,1 g Wasser in 82 ml Tetrahydrofuran, 27,1 g 10 %ige Natronlauge und 81,3 g Wasser zu dem Ansatz getropft Die Salze werden abgesaugt, mit Tetrahydrofuran gewaschen und das Filtrat im Vakuum eingeengt. Der Rückstand wird im Hochvakuum destilliert.
   Ausbeute: 19,1 g.

### Beispiel Z24

### 4-Amino-1,3,3a,4,7,7a-hexahyro-isoindol

13,3 g (50 mmol) 4-tert.-Butyloxycarbonylamino-1,3-dioxo-1,3,3a,4,7,7a- hexahydro-isoindol (aus Beispiel Z23, Methode II) werden in 166 ml Trifluoressigsäure über Nacht bei Raumtemperatur gerührt. Dann wird die Trifluoressigsäure bei 10 mbar abdestilliert und der Rückstand bei 50°C im Hochvakuum von Säureresten befreit. Anschließend wird in absolutem Tetrahydrofuran aufgenommen und im Vakuum eingeengt. Der Rückstand wird in 100 ml absolutem Tetrahydrofuran aufgenommen und unter Stickstoff zu einer Lösung von 11,3 g (0,3 mol) Lithiumaluminiumhydrid in 300 ml absolutem Tetrahydrofuran getropft. Dann wird 16 Stunden unter Rückflußkühlung gekocht. Nach dem Erkalten werden nacheinander 11,3 g Wasser in 34 ml Tetrahydrofuran, 11,3 ml 10 %ige Natronlauge und 34 ml Wasser zugetropft. Der Niederschlag wird abgesaugt und mit Tetrahydrofuran gewaschen. Das Filtrat wird eingeengt und der Rückstand destilliert.
Ausbeute: 2,2 g, Gehalt: 92 % (gaschromatographisch bestimmt)
Siedepunkt: 70°C/0,2 mbar.

### Beispiel Z25

### 7-Methyl-4-methylamino-1,3,3a,4,7,7a-hexahydro-isoindol

In Analogie zu Beispiel Z23, Methode I werden 21,9 g (0,12 mol) 1-(tert.-Butyloxycarbonylamino)-1,3-pentadien mit 20,3 g (0,12 mol) N-Trimethylsilyl-maleinimid umgesetzt und anschließend mit 15,2 g (0,4 mol) Lithiumaluminiumhydrid reduziert. Das Rohprodukt wird aus Tetrahydrofuran umkristallisiert.
Ausbeute: 6,2 g, Schmelzpunkt: 106-108°C.

### Beispiel Z26

### 1-Cyclopropyl-6,7-difluor-1,4-dihydro-8-(3-methoxy-propin-1-yl)-4-oxo-3-chinolincarbonsäure

A) 1,86 g (5 mmol) 8-Brom-1-cyclopropyl-6,7-difluor-1,4-dihydro-4-oxo-3-chinolincarbonsäureethylester werden in 20 ml absolutem Toluol mit 2,5 g (7 mmol) 1-Tributyl-stannyl-3-methoxy-propin und 0,29 g (entsprechend 5 mol-%) Tetrakis-(triphenylphosphin)-palladium (0) versetzt und 4 Stunden in einer Stickstoffatmosphäre unter Rückfluß erhitzt. Das Reaktionsgemisch wird eingeengt, der Rückstand mit Hexan verrührt, der Feststoff abgesaugt und über wenig Kieselgel chromatographisch gereinigt.
   Ausbeute: 0,74 g (41 % der Theorie) 1-Cyclopropyl-6,7-difluor-1,4-dihydro-8-(3-methoxy-propin-1-yl)-4-oxo-3-chinolincarbonsäureethylester.
   Schmelzpunkt: 144-146°C
B) 0,36 g (1 mmol) des Produkts aus Stufe A wird in einer Mischung aus 3 ml Eisessig, 0,2 ml Wasser und 0,05 ml konzentrierter Schwefelsäure 1 Stunde unter Rückfluß erhitzt. Die Mischung wird auf Wasser ausgegossen, der Niederschlag abfiltriert und aus Ethanol umkristallisiert.
   Ausbeute: 153 mg (46 % der Theorie) 1-Cyclopropyl-6,7-difiuor-1,4-dihydro-8-(3-methoxy-propin-1-y1)-4-oxo-3- chinolincarbonsäure.
   Schmelzpunkt: 170-172°C
   ¹H-NMR (270 MHz, CDCl₃): δ 1,24 m (2H), 1,4 m (2H), 3,45 s (OCH₃), 4,35m (1H), 4,41 s (O-CH₂-), 8,27 "t" (1H), 8,87 ppm s (1H).

### Herstellung der Wirkstoffe

### Beispiel 1

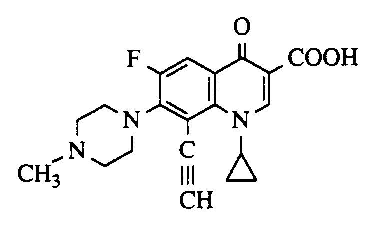

A) 2,32 g (8 mmol) 1-Cyclopropyl-8-ethinyl-6,7-difluor-1,4-dihydro-4-oxo-3-chinolincarbonsäure werden in einer Mischung aus 60 ml Acetonitril und 30 ml Dimethylformamid mit 0,92 g (8 mmol) 1,4-Diazabicyclo[2.2.2]octan und 1,2 g (12 mmol) N-Methylpiperazin 1 Stunde unter Rückfluß erhitzt. Die Suspension wird eingeengt, der Rückstand mit Acetonitril verrührt, das ungelöste Kristallisat abgesaugt und getrocknet.
   Ausbeute: 1,83 g (62 % der Theorie) 1-Cyclopropyl-8-ethinyl-6-fluor-1,4-dihydro-7-(4-methyl-1-piperazinyl)-4-oxo-3-chinolincarbonsäure.
   Schmelzpunkt: 228-230°C (unter Zersetzung)
   ¹H-NMR (d⁶-DMF): δ 4,95 ppm s (-C≡C-H).
B) Analog erhält man mit 2-Methylpiperazin 1-Cyclopropyl-8-ethinyl-6-fluor-1,4-dihydro-7-(3-methyl-1-piperazinyl)-4-oxo-3-chinolincarbonsäure.
   ¹H-NMR (d⁶-DMSO): δ 5,03 ppm s (-C≡C-H).
   Massenspektrum: m/e 369 (M⁺), 325 (M⁺-CO₂), 300, 293, 269, 243, 44 (CO₂).

Analog Beispiel 1 erhält man mit den Produkten aus den Beispielen Z14, Z7 und Z9:

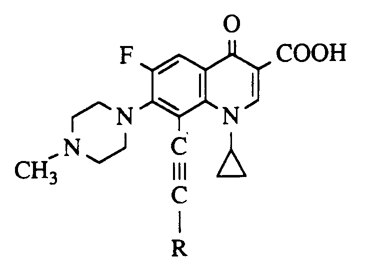

### Beispiel 2 (R=CH₃): 1-Cyclopropyl-6-fluor-1,4-dihydro-7-(4-methyl-1-piperazinyl)-4-oxo-8-(propin-1-yl)-3-chinolincarbonsäure.

Schmelzpunkt: 246-249°C (unter Zersetzung).

### Beispiel 3 (R=CH₂CH₂CH₂CH₃): 1-Cyclopropyl-6-fluor-8-(hexin-1-yl)-1,4-dihydro-7-(4-methyl-1-piperazinyl)-4-oxo-3-chinolincarbonsäure.

Schmelzpunkt: 206-208°C (unter Zersetzung).

### Beispiel 4 (R=C(CH₃)₃: 1-Cyclopropyl-8-(3,3-dimethylbutin-1-yl)-6-fluor-1,4-dihydro-7-(4-methyl-1-piperazinyl)-4-oxo-3-chinolincarbonsäure.

Schmelzpunkt: 234-237°C (unter Zersetzung).

### Beispiel 5

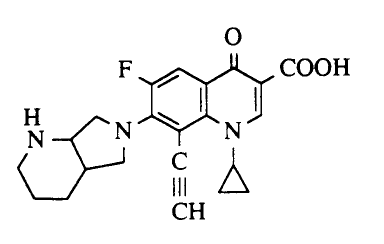

Analog Beispiel 1 wird mit cis-2,8-Diazabicyclo[4.3.0]nonan zu 1-Cyclopropyl-7-(cis-2,8-diazabicyclo[4.3.0]non-8-yl)-8-ethinyl-6-fluor-1,4-dihydro-4-oxo-3-chinolincarbonsäure vom Schmelzpunkt 225-227°C (unter Zersetzung) umgesetzt.
¹H-NMR (d⁶-DMF): δ 4,9 s (-C≡C-H).

### Beispiel 6

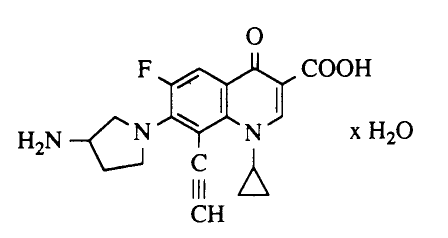

3,6 g (12 mmol) 1-Cyclopropyl-8-ethinyl-6,7-difluor-1,4-dihydro-4-oxo-3-chinolincarbonsäure werden in einer Mischung aus 120 ml Acetonitril und 60 ml Dimethylformamid mit 1,56 g (14 mmol) 1,4-Diazabicyclo[2.2.2]octan und 2,75 g (18 mmol) 3-(2,2-Dimethylpropylidenamino)pyrrolidin 1 Stunde unter Rückfluß erhitzt. Die Lösung wird eingeengt, der Rückstand mit etwa 100 ml Wasser verrührt (pH 7), der Niederschlag abgesaugt, mit Wasser gewaschen und anschließend zur vollständigen Abspaltung der Schutzgruppe in 50 ml Wasser suspendiert und 1 Stunde im Ultraschallbad behandelt. Danach wird abgesaugt, mit Wasser gewaschen und bei 80°C im Vakuum getrocknet.
Ausbeute: 3,8 g (82 % der Theorie) 7-(3-Amino-1-pyrrolidinyl)-1-cyclopropyl-8-ethinyl-6-fluor-1,4-dihydro-4-oxo-3-chinolincarbonsäure-Hydrat.
Schmelzpunkt: 193-196°C (unter Zersetzung).

### Beispiel 7

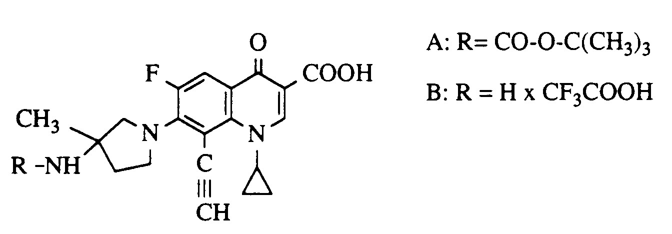

A) Analog Beispiel 1 wird mit 3-tert.-Butoxycarbonylamino-3-methyl-pyrrolidin zu 7-(3-tert.-Butoxycarbonylamino-3-methyl-1-pyrrolidinyl)-1-cyclo-propyl-8-ethinyl-6-fluor-1,4-dihydro-4-oxo-3-chinolincarbonsäure vom Schmelzpunkt 244-246°C (unter Zersetzung) umgesetzt.
   ¹H-NMR (d⁶-DMSO): δ 4,92 ppm s(-C≡C-H).
B) 500 mg des Produktes aus Stufe A werden in 5 ml Trifluoressigsäure unter Eiskühlung gelöst, die Lösung im Vakuum eingeengt, der Rückstand durch Verrühren mit dreimal etwa 1 ml Ethanol zur Kristallisation gebracht, das Salz abgesaugt, mit Ethanol gewaschen und getrocknet.
   Ausbeute: 270 mg (52 % der Theorie) 7-(3-Amino-3-methyl-1-pyrrolidinyl)-1-cyclopropyl-8-ethinyl-6-fluor-1,4-dihydro-4-oxo-3-chinolincarbonsäure-Trifluoracetat.
   Schmelzpunkt: 242-244°C (unter Zersetzung).

### Beispiel 8

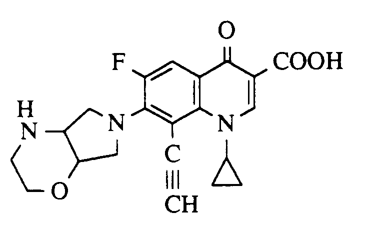

Analog Beispiel 1 wird mit 2-Oxa-5,8-diazabicyclo[4.3.0]nonan zu 1-Cyclopropyl-8-ethinyl-6-fluor-1,4-dihydro-7-(2-oxa-5,8-diazabicyclo[4.3.0]non-8-yl)-4-oxo-3-chinolincarbonsäure vom Schmelzpunkt 290°C (unter Zersetzung; sintert bereits ab etwa 170°C) umgesetzt.
¹H-NMR (d⁶-DMSO): δ 5,0 ppm s (-C≡C-H).

### Beispiel 9

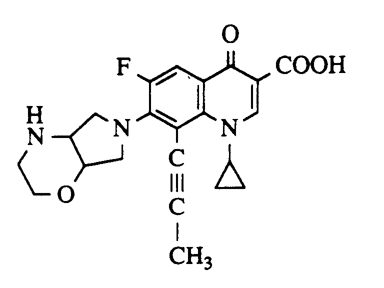

Analog Beispiel 1 wird 2-Oxa-5,8-diazabicyclo[4.3.0]nonan mit dem Produkt aus Beispiel Z14 zu 1-Cyclopropyl-6-fluor-1,4-dihydro-7-(2-oxa-5,8-diazabicyclo[4.3.0]non-8-yl)-4-oxo-8-(propin-1-yl)-3-chinolincarbonsäure vom Schmelzpunkt 241-242°C (unter Zersetzung) umgesetzt.

### Beispiel 10

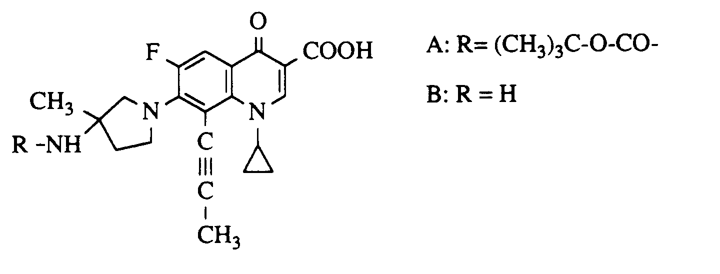

A) 303 mg (1 mmol) des Produkts aus Beispiel Z14 werden in einer Mischung aus 6 ml Acetonitril und 3 ml Dimethylformamid mit 240 mg 3-tert.-Butoxycarbonylamino-3-methyl-pyrrolidin und 134 mg (1,2 mmol) 1,4-Diazabicyc-lo[2.2.2]octan versetzt und 2 Stunden unter Rückfluß erhitzt. Man engt im Vakuum ein, verrührt mit 30 ml Wasser und trocknet bei 80° C im Vakuum.
   Ausbeute: 420 mg (87 % der Theorie) 7-(3-tert.-Butoxycarbonylamino-3-methyl-1-pyrrolidinyl)-1-cyclopropyl-6-fluor-1,4-dihydro-4-oxo-8-(propin-1-yl)-3-chinolincarbonsäure.
   Schmelzpunkt: 195-196°C (unter Zersetzung).
   ¹H-NMR (d⁶-DMSO): δ 1,42 s (CH₃ am Pyrrolidin), 2,12 ppm s (CH₃-C≡C-).
B) 180 mg des Produkts aus Stufe A werden bei 0°C in 1,6 ml Trifluoressigsäure gelöst und die Lösung nach 1,25 Stunden eingeengt. Der Rückstand wird chromatographisch gereinigt (Kieseigel, Dichlormethan/Methanol/17 %iges wäßriges Ammoniak = 30:8:1). Man isoliert 10 mg 7-(3-Amino-3-methyl-1-pyrrolidinyl)-1-cyclopropyl-6-fluor-1,4-dihydro-oxo-8-(propin-1-yl)-3-chinolincarbonsäure vom Schmelzpunkt 209-210°C (unter Zersetzung).
   Massenspektrum: m/e 383 (M⁺), 309, 298, 267 (100 %), 133, 70.

### Beispiel 11

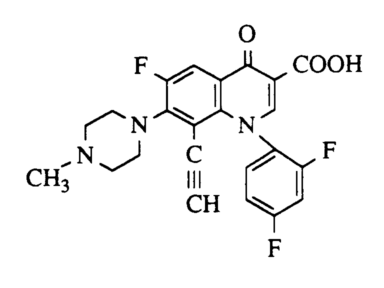

Analog Beispiel 1 setzt man das Produkt aus Beispiel Z12 mit N-Methylpiperazin zu 8-Ethinyl-6-fluor-1-(2,4-difluorphenyl)-1,4-dihydro-7-(4-methyl-1-piperazinyl)-4-oxo-3-chinolincarbonsäure vom Schmelzpunkt 193-195°C (unter Zersetzung) um.
¹H-NMR (CDCl₃): 3,35 s (-C≡CH).

### Beispiel 12

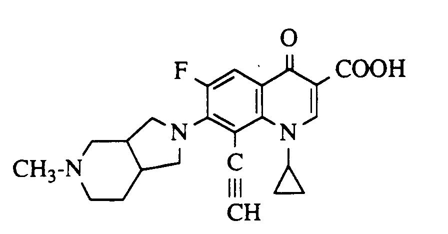

Analog Beispiel 1 wird mit 3-Methyl-3,8-diazabicyclo[4.3.0]nonan umgesetzt und das als Rohprodukt anfallende 1-Cyclopropyl-8-ethinyl-6-fluor-1,4-dihydro-7-(3-methyl-3,8-diazabicyclo[4.3.0]non-8-yl)-4-oxo-3-chinolincarbonsäure chromatographisch (Kieselgel; Dichlormethan/Methan/20 %iges wäßriges Ammoniak 2:4:1) gereinigt.
¹H-NMR (CDCl₃): 4,15 s (-C≡C-H).

### Beispiel 13

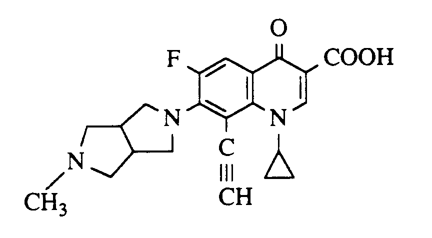

Analog Beispiel 1 wird mit 3-Methyl-3,7-diazabicyclo[3.3.0]octan zu 1-Cyclopropyl-8-ethinyl-6-fluor-1,4-dihydro-7-(7-methyl-3,7-diazabicyclo[3.3.0]oct-3-yl)-4-oxo-3-chinolincarbonsäure vom Schmelzpunkt 212-216°C (unter Zersetzung) umgesetzt.
¹H-NMR (d⁶-DMF): δ 4,95 s (-C≡C-H).

### Beispiel 14

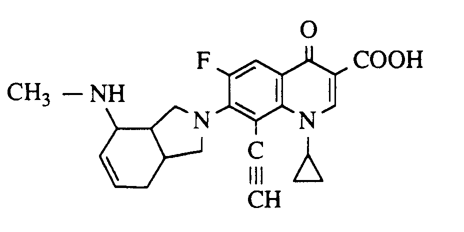

Analog Beispiel 1 wird mit 4-Methylamino-1,3,3a,4,7,7a-hexahydroisoindol zu 1-Cyclopropyl-8-ethinyl-6-fluor-1,4-dihydro-7-(4-methylamino-1,3,3a,4,7,7a-hexahydroisoindol-2-yl)-4-oxo-3-chinolincarbonsäure vom Schmelzpunkt 128-133°C (unter Zersetzung) umgesetzt.
¹H-NMR (d⁶-DMSO): δ 4,93 ppm s(-C≡CH).

### Beispiel 15

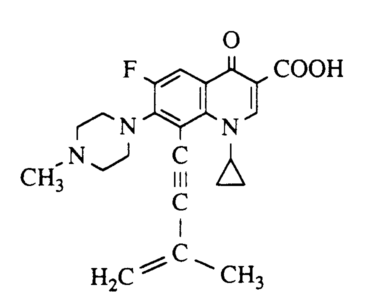

164 mg (0,5 mmol) des Produkts aus Beispiel Z18 werden analog Beispiel 1 mit 1-Methylpiperazin zu 120 mg 1-Cyclopropyl-6-fluor-1,4-dihydro-8-(3-methyl-but-3-en-1-inyl)-7-(4-methyl-1-piperazinyl)-4-oxo-3-chinolincarbonsäure vom Schmelzpunkt 195-197°C (unter Zersetzung) (unkristallisiert aus Glykolmonomethylether) umgesetzt.
¹H-NMR (CDCl₃): δ 5,36 m (>C=CH₂), 2,4 s (N-CH₃), 2,0 t (C-CH₃).

### Beispiel 16

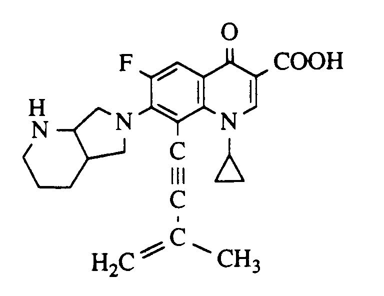

Analog Beispiel 15 wird mit 2,8-Diazabicyclo[4.3.0]nonan zu 1-Cyclopropyl-7-(2,8-diazabicyclo[4.3.0]non-8-yl)-6-fluor-1,4-dihydro-8-(3-methyl-but-3-en-1-inyl)-4-oxo-3-chinolincarbonsäure vom Schmelzpunkt 201-202°C (unter Zersetzung) umgesetzt.

### Beispiel 17

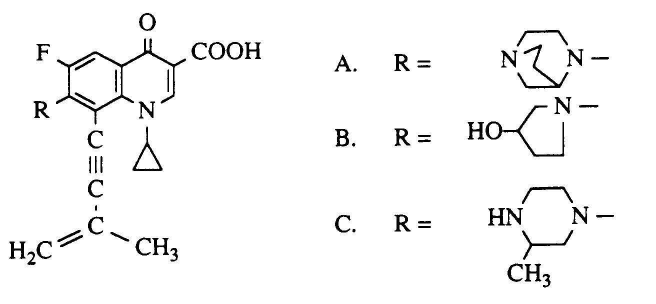

Analog Beispiel 15 wird mit
- A.: 1,4-Diazabicyclo[3.2.1]octan
- B.: 3-Hydroxypyrrolidin
- C.: 2-Methylpiperazin
zu folgenden Verbindungen umgesetzt:
- A.: 1-Cyclopropyl-7-(1,4-diazabicyclo[3.2.1]oct-4-yl)-6-fluor-1,4-dihydro8-(3-methyl-but-3-en-1-inyl)-4-oxo-3-chinolincarbonsäure,
- B.: 1-Cyclopropyl-6-fluor-1,4-dihydro-7-(3-hydroxy-1-pyrrolidinyl)-8-(3-methyl-but-3-en-1-inyl)4-oxo-3-chinolincarbonsäure, Schmelzpunkt: 190-198°C (unter Zersetzung),
- C.: 1-Cyclopropyl-6-fluor-1,4-dihydro-8-(3-methyl-but-3-en-1-inyl)-7-(3-methyl-1-piperazinyl)-4-oxo-3-chinolincarbonsäure.

### Beispiel 18

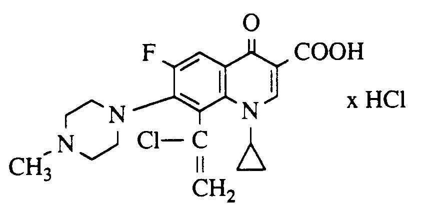

100 mg des Produkts aus Beispiel 1 werden in 40 ml 1n Salzsäure gelöst und während 2 Stunden bei 30°C gerührt. Man erhält eine Suspension, die eingeengt wird. Der Rückstand wird mit wenig Isopropanol verrührt, der Niederschlag abgesaugt, mit Isopropanol gewaschen und bei 90°C im Vakuum getrocknet.
Ausbeute: 0,1 g (83 % der Theorie) 8-(1-Chlorvinyl)-1-cyclopropyl-6-fluor-1,4-dihydro-7-(4-methyl-1-piperazinyl)-4-oxo-3-chinolincarbonsäure-Hydrochlorid.
Schmelzpunkt: 251-252°C (unter Zersetzung).
¹H-NMR (d⁶-DMSO): δ 6,0 ppm dd

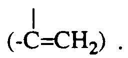

### Beispiel 19

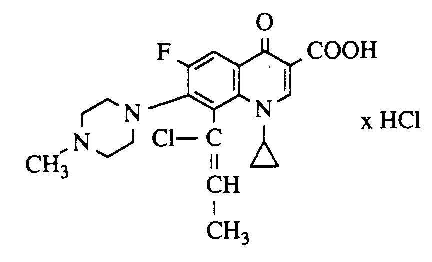

A) 100 mg des Produkts aus Beispiel 2 werden in 58 ml 4n-Salzsäure 5 Stunden auf 60°C erhitzt. Die Mischung wird eingeengt der Rückstand mit Diethylether verrührt und bei 70°C im Vakuum getrocknet.
   Ausbeute: 90 mg cis-trans-8-(1-Chlor-1-propenyl)-1-cyclopropyl-6-fluor-1,4-dihydro-7-(4-methyl-1-piperazinyl)-4-oxo-3-chinolincarbonsäure-Hydrochlorid.
   Schmelzpunkt: 236-237°C (unter Zersetzung).
   Massenspektrum: m/e 419 (M⁺), 71,58 (100%), 43,36.
   ¹H-NMR (d⁶-DMSO): δ 6,12 q und 6,35 q 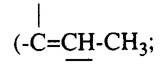 zwei Signale für cis-trans-Formen).
B) Analog entsteht mit dem Produkt aus Beispiel 3 cis-trans-8-(1-Chlor-1-hexenyl)-1-cyclopropyl-6-fluor-1,4-dihydro-7-(4-methyl-1-piperazinyl)-4-oxo-3-chinolincarbonsäure-Hydrochlorid.
   Massenspektrum: m/e 461 (M⁺), 425 (M-HCl), 71, 58 (100 %), 43, 36.

### Beispiel 20

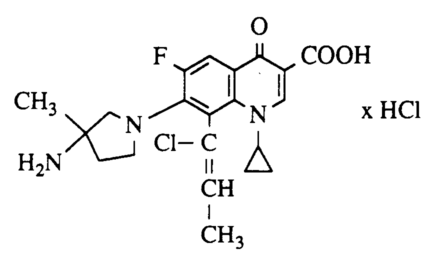

370 mg des Produkts aus Beispiel 10A werden in 9 ml halbkonzentrierter Salzsäure gelöst und die Losung im Hochvakuum eingeengt.
Ausbeute: 340 mg cis-trans-7-(3-Amino-3-methyl-1-pyrrolidinyl)-8-(1-chlor-1-propenyl)-1-cyclopropyl-6-fluor-1,4-dihydro-4-oxo-3-chinolincarbonsäure-Hydrochlorid.
¹H-NMR (d⁶-DMSO): δ 6,19 q und 6,36 q (>CH=CH-CH₃; 2 Signale für cis-trans-Formen).

### Beispiel 21

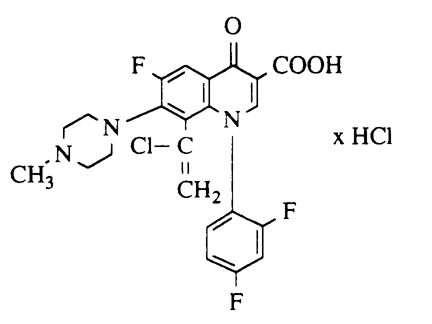

10 mg des Produkts aus Beispiel 11 werden in 4,5 ml 2,5n-Salzsäure während 1 Stunde auf 60°C erhitzt. Die Mischung wird eingeengt und als Rückstand 8-(1-Chlorvinyl)-6-fluor-1-(2,4-difluorphenyl)-1,4-dihydro-7-(4-methyl-1-piperazinyl)-4-oxo-3-chinolincarbonsäure-Hydrochlorid erhalten.
Massenspektrum: m/e 477 (M+), 442 (M⁺-Cl), 36 (100%, HCl).

### Beispiel 22

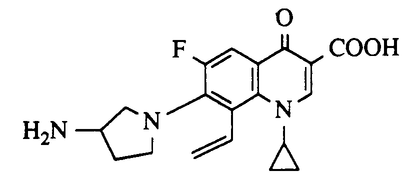

291 mg (1 mmol) des Produkts aus Beispiel Z2 werden in einer Mischung aus 20 ml Acetonitril und 10 ml Dimethylformamid mit 240 mg (2,2 mmol) 1,4-Diazabicyclo-[2.2.2]octan und 360 mg (2,3 mmol) 3-(2,2-Dimethyl-propylidenamino)-pyrrolidin versetzt und die Mischung 32 Stunden unter Rückfluß erhitz. Man engt ein, verrührt den dunklen öligen Rückstand mit 10 ml Wasser und saugt den ausgefallenen Feststoff (103 mg) ab, der chromatrographisch (Kieselgel; Dichlormethan/Methanol/17 %iges waßriges Ammoniak 30:8:1) gereinigt wird.
Ausbeute: 58 mg (16 % der Theorie) 7-(3-Amino-1-pyrrolidinyl)-1-cyclopropyl-6-fluor-1,4-dihydro-4-oxo-8-vinyl-3-chinolincarbonsäure.
Schmelzpunkt: 179-182°C (unter Zersetzung).
CI-Massenspektrum: m/e 358 ([M+H]⁺), 340 (M+H₋H₂O]⁺).

### Beispiel 23

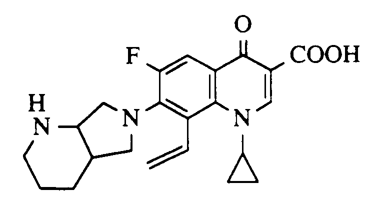

145 mg (0,5 mmol) des Produkts aus Beispiel Z2 werden in einer Mischung aus 10 ml Acetonitril und 5 ml Dimethylformamid mit 60 mg (0,54 mmol) 1,4-Diazabicyclo[2.2.2]octan und 140 mg (1,1 mmol) cis-2,8-Diazabicyclo-[4.3.0]nonan versetzt und 4 Stunden unter Rückfluß erhitzt. Die Losung wird eingeengt, mit etwa 5 ml Wasser verrührt und mit verdünnter Salzsäure auf pH 7 eingestellt. Der Niederschlag wird abgesaugt, mit Wasser gewaschen und bei 90°C im Vakuum getrocknet. Ausbeute: 120 mg (61 % der Theorie) 1-Cyclopropyl-7-(cis-2,8-diazabicyclo[4.3.0]non-8-yl)-6-fluor-1,4-dihydro-4-oxo-8-vinyl-3-chinolincarbonsäure.
Schmelzpunkt: 205-207°C (unter Zersetzung).
¹H-NMR (CF₃COOD): δ 5,05 d (1H), 5,7 d (1H), 7,55 dd (1H) (Signalgruppen für -CH=CH₂).

### Beispiel 24

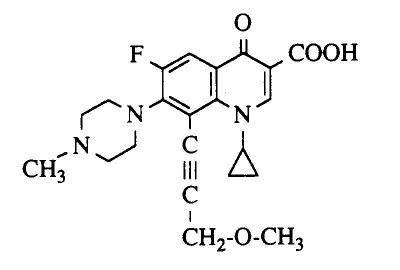

Man setzt analog Beispiel 1 mit dem Produkt aus Beispiel Z26 um und erhält 1-Cyclopropyl-6-fluor-1,4-dihydro-7-(4-methyl-1-piperazinyl)-8-(3-methoxy-propin-1-yl)-4-oxo-3-chinolincarbonsäure vom Schmelzpunkt 187-189°C.
¹H-NMR (CDCl₃): δ 8,95 s (1H), 8 d (1H), 4,37 s (O-CH₂), 4,35 m (1H), 3,58 m (4H), 3,43 s (O-CH₃), 2,58 m (4H), 2,38 s (N-CH₃), 1,33 m (2 H), 1,02 ppm m (2H).

### Beispiel 25

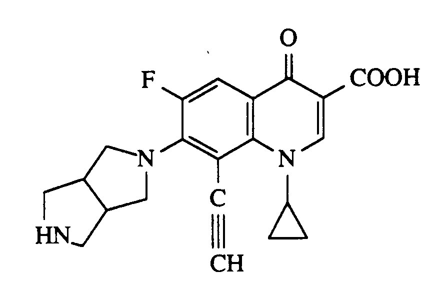

Man setzt analog Beispiel 1 mit 3,7-Diazabicyclo[3.3.0]octan um, chromatographiert das Reaktionsprodukt an Kieselgel mit Dichlormethan/Methanol/17 %Ammoniak (30:8:1) als Laufmittel und erhält 1-Cyclopropyl-7-(3,7-diazabicyclo[3.3.0]oct-3-yl)-8-ethinyl-6-fluor-1,4-dihydro-4-oxo-3-chinolincarbonsäure als "erstarrten Schaum".
¹H-NMR (d⁶-DMSO): δ 4,9 s (-C≡CH).

## Patentansprüche (Patentansprüche für folgende(n) Vertragsstaat(en): AT, BE, CH, DE, DK, FR, GB, IT, LI, LU, MC, NL, PT, SE)

1. Chinoloncarbonsäurederivate der Formel (I) 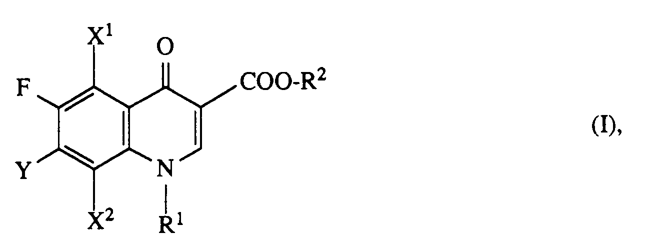 in welcher
R¹ für gegebenenfalls durch Hydroxy, Halogen oder C₁-C₃-Alkoxy substituiertes geradkettiges oder verzweigtes C₁-C₄-Alkyl, für gegebenenfalls durch Halogen oder C₁-C₃-Alkyl substituiertes C₃-C₆-Cycloalkyl, C₂-C₄-Alkenyl, ferner für C₁-C₃-Alkoxy, Amino, Monoalkylamino mit 1 bis 3 C-Atomen, Dialkylamino mit 2 bis 6 C-Atomen oder für gegebenenfalls durch Halogen ein- bis dreifach substituiertes Phenyl steht,
R² für Wasserstoff, Alkyl mit 1 bis 4 Kohlenstoffatomen oder (5-Methyl-2-oxo-1,3-dioxol-4-yl)-methyl steht
X¹ für Wasserstoff, Fluor, Chlor, Amino oder Methyl,
X² für 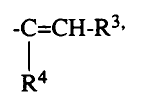 -C≡C-R⁵ oder -CH₂-CH=CH₂ steht,
worin
R³ Wasserstoff, C₁-C₃-Alkyl, C₁-C₃-Alkoxy oder Alkoxymethyl mit 1 bis 3 C-Atomen im Alkoxyteil,
R⁴ Wasserstoff oder Halogen und
R⁵ Wasserstoff, gegebenenfalls durch Halogen ein- bis dreifach substituiertes C₁-C₆-Alkyl, C₂-C₃-Alkenyl, Alkoxy mit 1 bis 3 C-Atomen, Alkoxymethyl mit 1 bis 3 C-Atomen im Alkoxyteil, Halogen oder Trimethylsilyl bedeutet und
Y für 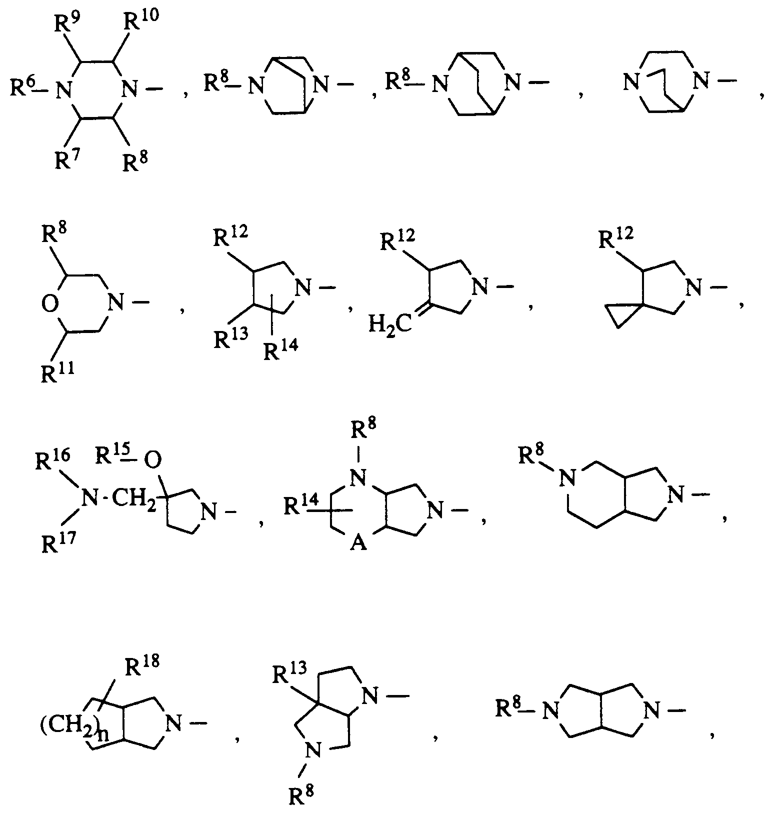 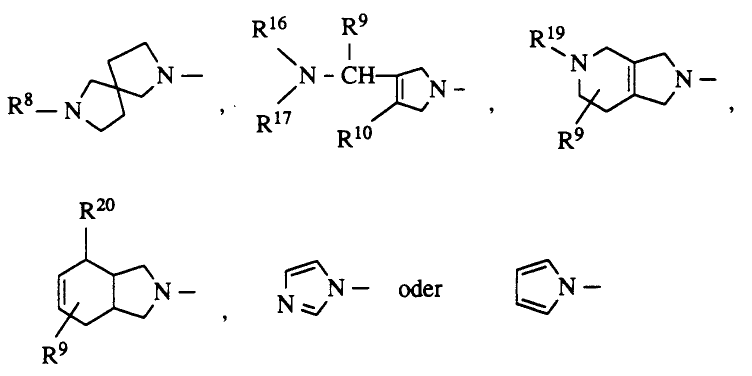 steht,
worin
R⁶ für Wasserstoff, gegebenenfalls durch Hydroxy oder Methoxy substituiertes geradkettiges oder verzweigtes C₁-C₄-Alkyl, Cyclopropyl, Oxoalkyl mit 1 bis 4 C-Atomen, Acyl mit 1 bis 3 C-Atomen,
R⁷ für Wasserstoff, Methyl, Phenyl, Thienyl oder Pyridyl,
R⁸ für Wasserstoff oder Methyl,
R⁹ für Wasserstoff oder Methyl,
R¹⁰ für Wasserstoff oder Methyl,
R¹¹ für Wasserstoff, Methyl oder 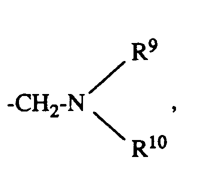
R¹² für Wasserstoff, Methyl, Amino, gegebenenfalls durch Hydroxy substituiertes Alkyl- oder Dialkylamino mit 1 oder 2 C-Atomen im Alkylteil, Aminomethyl, Aminoethyl, gegebenenfalls durch Hydroxy substituiertes Alkyl- oder Dialkylaminomethyl mit 1 oder 2 C-Atomen im Alkylteil oder 1-Imidazolyl,
R¹³ für Wasserstoff, Hydroxy, Methoxy, Methylthio oder Halogen, Methyl, Hydroxymethyl,
R¹⁴ für Wasserstoff oder Methyl,
R¹⁵ für Wasserstoff, Methyl oder Ethyl,
R¹⁶ für Wasserstoff, Methyl oder Ethyl,
R¹⁷ für Wasserstoff, Methyl oder Ethyl,
R¹⁸ für Hydroxy, 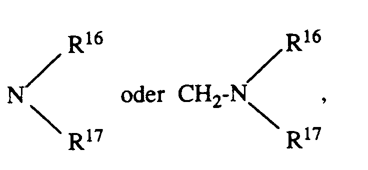
R¹⁹ für Wasserstoff, gegebenenfalls durch Hydroxy substituiertes C₁-C₃-Alkyl, Alkoxycarbonyl mit 1 bis 4 Kohlenstoffatomen im Alkoxyteil oder C₁-C₃-Acyl,
R²⁰ für Wasserstoff, Hydroxy, 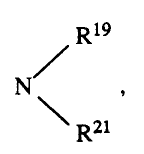 Hydroxymethyl oder 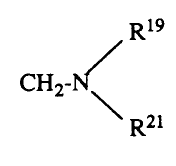 steht, worin
R²¹ Wasserstoff oder Methyl bedeutet,
A für CH₂, O oder für eine direkte Bindung steht und
n für 1 oder 2 steht,
und deren pharmazeutisch verwendbaren Hydrate und Säureadditionssalze sowie die Alkali-, Erdalkali-, Silber- und Guanidiniumsalze.

2. Chinoloncarbonsäurederivate gemäß Anspruch 1,
worin
R¹ für gegebenenfalls durch Hydroxy substituiertes C₁-C₂-Alkyl, C₃-C₅-Cycloalkyl, Vinyl, Amino, Monoalkylamino mit 1 bis 2 C-Atomen, Dialkylamino mit 2 bis 4 C-Atomen oder für gegebenenfalls durch Halogen ein- oder zweifach substituiertes Phenyl steht,
R² für Wasserstoff, Alkyl mit 1 bis 3 Kohlenstoffatomen oder (5-Methyl-2-oxo-1,3-dioxol-4-yl)-methyl steht
X¹ für Wasserstoff, Fluor, Chlor, Amino oder Methyl,
X² für 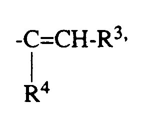 -C≡C-R⁵ oder -CH₂-CH=CH₂ steht,
worin
R³ Wasserstoff, C₁-C₂-Alkyl, Methoxy oder Methoxymethyl,
R⁴ Wasserstoff und
R⁵ Wasserstoff, gegebenenfalls durch Fluor ein- bis dreifach substituiertes C₁-C₄-Alkyl, C₂-C₃-Alkenyl, Methoxy oder Trimethylsilyl bedeutet und
Y für 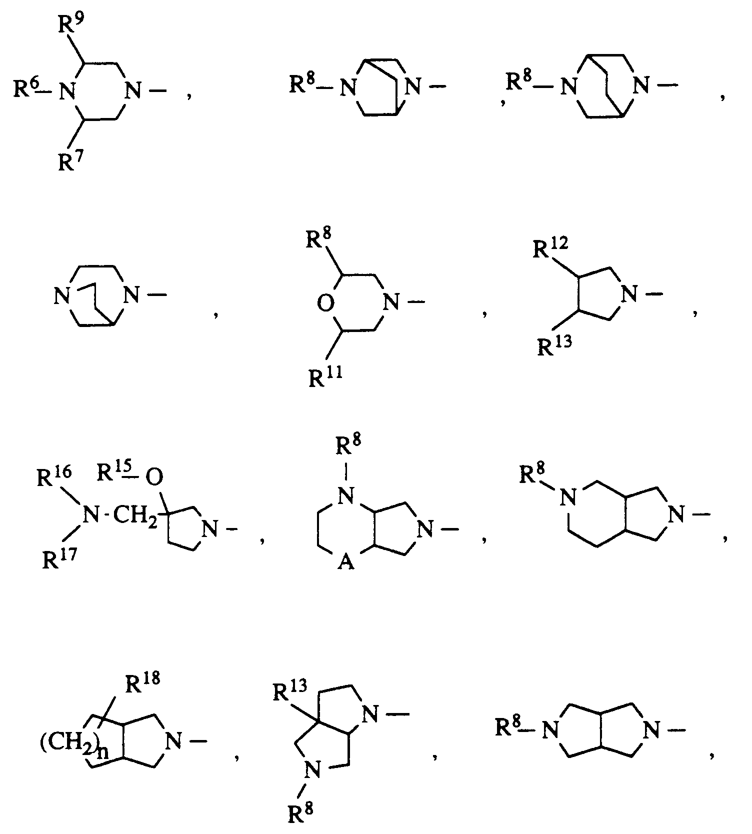 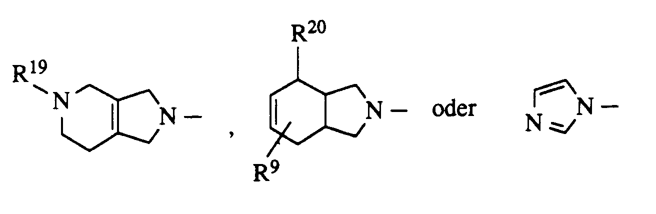 steht,
worin
R⁶ für Wasserstoff, gegebenenfalls durch Hydroxy substituiertes geradkettiges oder verzweigtes C₁-C₃-Alkyl, Oxoalkyl mit 1 bis 4 C-Atomen,
R⁷ für Wasserstoff, Methyl oder Phenyl,
R⁸ für Wasserstoff oder Methyl,
R⁹ für Wasserstoff oder Methyl,
R¹¹ für Wasserstoff, Methyl oder -CH₂-NH₂,
R¹² für Wasserstoff, Methyl, Amino, Methylamino, Dimethylamino, Aminomethyl, Methylaminomethyl oder Ethylaminomethyl,
R¹³ für Wasserstoff, Hydroxy, Methoxy, Fluor, Methyl oder Hydroxymethyl,
R¹⁵ für Wasserstoff oder Methyl,
R¹⁶ für Wasserstoff oder Methyl,
R¹⁷ für Wasserstoff oder Methyl,
R¹⁸ für 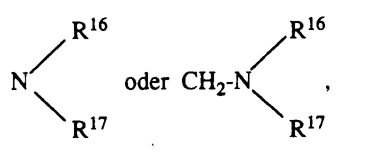
R¹⁹ für Wasserstoff, Methyl oder Ethyl,
R²⁰ für 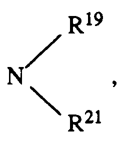 steht,
worin
R²¹ Wasserstoff oder Methyl bedeutet,
A für CH₂, O oder für eine direkte Bindung steht und
n für 1 oder 2 steht.

3. Chinoloncarbonsäurederivate gemäß Anspruch 1,
worin
R¹ für Methyl, Ethyl, Cyclopropyl oder gegebenenfalls durch Fluor ein- oder zweifach substituiertes Phenyl,
R² für Wasserstoff, Methyl oder Ethyl,
X¹ für Wasserstoff, Fluor, Chlor, Amino oder Methyl,
X² für -CH=CH₂ oder -C≡C-R⁵ steht,
worin
R⁵ Wasserstoff, C₁-C₄-Alkyl, C₂-C₃-Alkenyl oder Trimethylsilyl bedeutet und
Y für 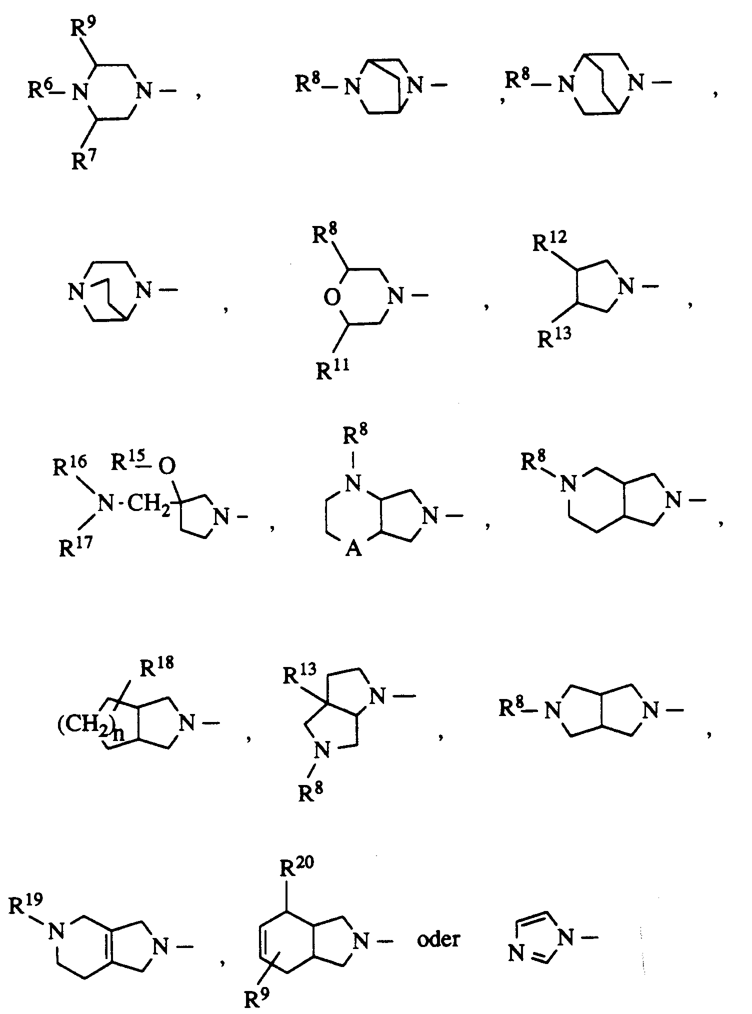 steht,
worin
R⁶ für Wasserstoff, Methyl, gegebenenfalls durch Hydroxy substituiertes Ethyl,
R⁷ für Wasserstoff oder Methyl,
R⁸ für Wasserstoff oder Methyl,
R⁹ für Wasserstoff oder Methyl,
R¹¹ für Wasserstoff oder -CH₂-NH₂,
R¹² für Wasserstoff, Methyl, Amino, Methylamino, Aminomethyl oder Ethylaminomethyl,
R¹³ für Wasserstoff, Hydroxy oder Methoxy,
R¹⁵ für Wasserstoff oder Methyl,
R¹⁶ für Wasserstoff oder Methyl,
R¹⁷ für Wasserstoff oder Methyl,
R¹⁸ für 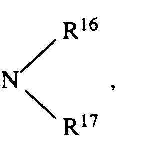
R¹⁹ für Wasserstoff oder Methyl,
R²⁰ für 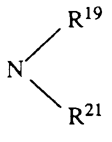 steht,
worin
R²¹ Wasserstoff oder Methyl bedeutet,
A für CH₂, O oder für eine direkte Bindung steht und
n für 1 steht.

4. Chinoloncarbonsäurederivate der Formel (II) 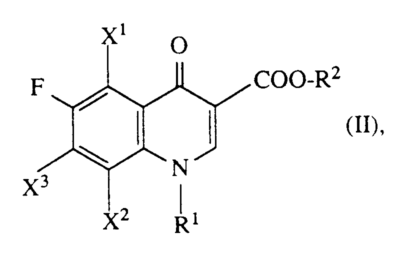 in welcher
R¹, R², X¹ und X² die Bedeutung gemäß Anspruch 1 haben und
X³ für Halogen, insbesondere Fluor oder Chlor, steht.

5. Chinolincarbonsäurederivate aus der Gruppe bestehend aus
1-Cyclopropyl-8-ethinyl-6-fluor-1,4-dihydro-7-(4-methyl-1-piperazinyl)-4-oxo-3-chinolincarbonsäure,
1-Cyclopropyl-7-(cis-2,8-diazabicyclo[4.3.0]-non-8-yl)-8-ethinyl-6-fluor-1,4-dihydro-4-oxo-3-chinolincarbonsäure,
7-(3-Amino-1-pyrrolidinyl)-1-cyclopropyl-8-ethinyl-6-fluor-1,4-dihydro-4-oxo-3-chinolincarbonsäure,
7-(3-Amino-3-methyl-1-pyrrolidinyl)-1-cyclopropyl-8-ethinyl-6-fluor-1,4-dihydro-4-oxo-3-chinolincarbonsäure-Trifluoracetat,
1-Cyclopropyl-6-fluor-1,4-dihydro-8-(3-methyl-but-3-en-1-inyl)-7-(4-methyl-1-piperazinyl)-4-oxo-3-chinolincarbonsäure,
1-Cyclopropyl-8-ethinyl-6-fluor-1,4-dihydro-7-(2-oxa5,8-diazabicyclo[4.3.0]non-8-yl)-4-oxo-3-chinolincarbonsäure,
1-Cyclopropyl-8-ethinyl-6-fluor-1,4-dihydro-7(7-methyl-3,7-diazabicyclo[3.3.0]oct-3-yl)-4-oxo-3-chinolincarbonsäure,
1-Cyclopropyl-8-ethinyl-6-fluor-1,4-dihydro-7-(4-methylamino-1,3,3a,4,7,7a-hexahydroisoindol-2-yl)-4-oxo-3-chinolincarbonsäure und
1-Cyclopropyl-7-(cis-2,8-diazabicyclo[4.3.0]non-8-yl6-fluor-1,4-dihydro-4-oxo-8-vinyl-3-chinolincarbonsäure und deren pharmakologisch verträgliche Hydrate und Säureadditionssalze sowie die Alkali-, Erdalkali-, Silber-und Guanidiniumsalze.

6. Chinolincarbonsäurederivate aus der Gruppe bestehend aus
8-Brom-1-(2,4-difluorphenyl)-6,7-difluor-1,4-dihydro-4-oxo-3-chinolincarbonsäure und
8-Brom-1-ethyl-6,7-difluor-1,4-dihydro-4-oxo-3-chinobonsäure und deren Hydrate und Säureadditionssalze sowie die Alkali-, Erdalkali-, Silber-und Guanidiniumsalze.

7. Verfahren zur Herstellung von Chinoloncarbonsäurederivaten gemäß Anspruch 1, dadurch gekennzeichnet, daß man eine Verbindung der Formel (II) 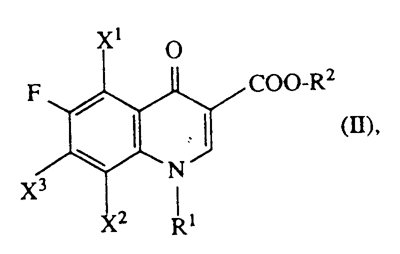 in welcher
R¹,R², X¹ und X² die Bedeutung gemäß Anspruch 1 haben und
X³ für Halogen, insbesondere Fluor oder Chlor, steht,
mit Verbindungen der Formel (III)
Y-H (III),
in welcher
Y die Bedeutung gemäß Anspruch 1 hat,
gegebenenfalls in Gegenwart von Säurefängern umsetzt.

8. Chinoloncarbonsäurederivate gemäß Ansprüchen 1 bis 5 zur Behandlung von Krankheiten.

9. Arzneimittel enthaltend Chinoloncarbonsäurederivate gemäß Ansprüchen 1 bis 5.

10. Verwendung von Chinoloncarbonsäurederivaten gemäß Ansprüchen 1 bis 5 bei der Herstellung von Arzneimitteln.

## Patentansprüche (Patentansprüche für folgende(n) Vertragsstaat(en): ES)

1. Verfahren zur Herstellung von Chinoloncarbonsäurederivaten der Formel (I) 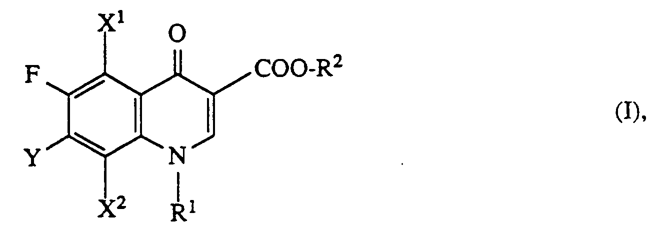 in welcher
R¹ für gegebenenfalls durch Hydroxy, Halogen oder C₁-C₃-Alkoxy substituiertes geradkettiges oder verzweigtes C₁-C₄-Alkyl, für gegebenenfalls durch Halogen oder C₁-C₃-Alkyl substituiertes C₃-C₆-Cycloalkyl, C₂-C₄-Alkenyl, ferner für C₁-C₃-Alkoxy, Amino, Moncalkylamino mit 1 bis 3 C-Atomen, Dialkylamino mit 2 bis 6 C-Atomen oder für gegebenenfalls durch Halogen ein- bis dreifach substituiertes Phenyl steht,
R² für Wasserstoff, Alkyl mit 1 bis 4 Kohlenstoffatomen oder (5-Methyl-2-oxo-1,3-dioxol-4-yl)-methyl steht
X¹ für Wasserstoff, Fluor, Chlor, Amino oder Methyl,
X² für 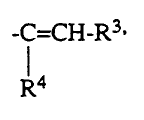 -C≡C-R⁵ oder -CH₂-CH=CH₂ steht,
worin
R³ Wasserstoff, C₁-C₃-Alkyl, C₁-C₃-Alkoxy oder Alkoxmethyl mit 1 bis 3 C-Atomen im Alkoxyteil,
R⁴ Wasserstoff oder Halogen und
R⁵ Wasserstoff, gegebenenfalls durch Halogen ein- bis dreifach substituiertes C₁-C₆-Alkyl, C₂-C₃-Alkenyl, Alkoxy mit 1 bis 3 C-Atomen, Alkoxymethyl mit 1 bis 3 C-Atomen im Alkoxyteil, Halogen oder Trimethylsilyl bedeutet und
Y für 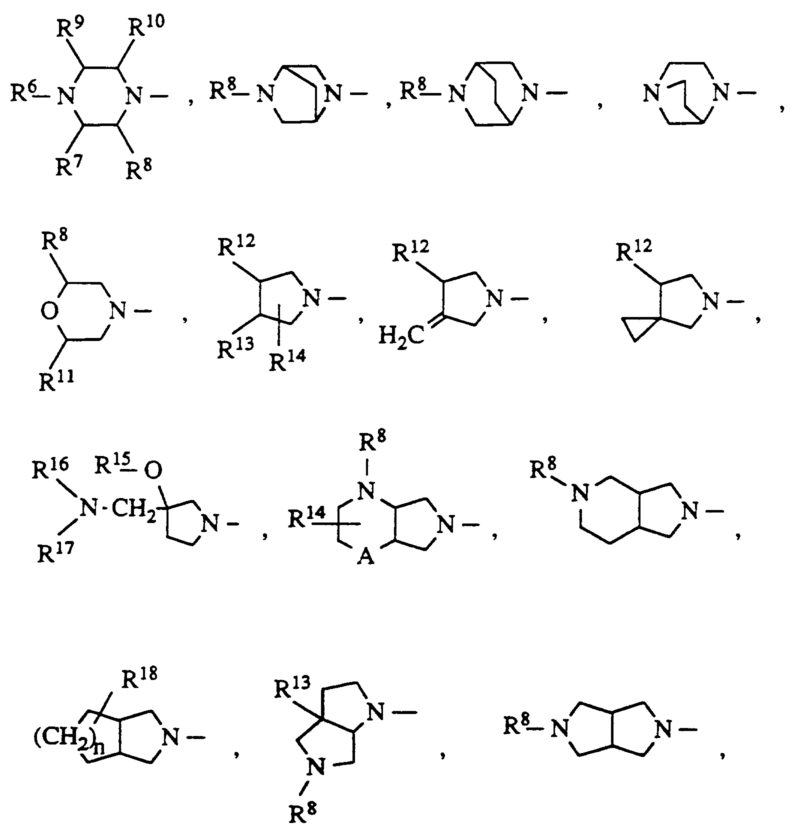 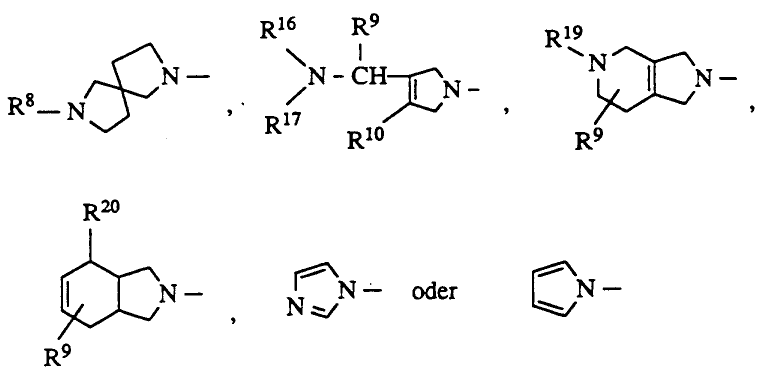 steht,
worin
R⁶ für Wasserstoff, gegebenenfalls durch Hydroxy oder Methoxy substituiertes geradkettiges oder verzweigtes C₁-C₄-Alkyl, Cyclopropyl, Oxoalkyl mit 1 bis 4 C-Atomen, Acyl mit 1 bis 3 C-Atomen,
R⁷ für Wasserstoff, Methyl, Phenyl, Thienyl oder Pyridyl,
R⁸ für Wasserstoff oder Methyl,
R⁹ für Wasserstoff oder Methyl,
R¹⁰ für Wasserstoff oder Methyl,
R¹¹ für Wasserstoff, Methyl oder 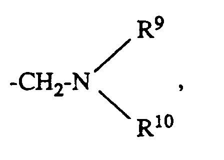
R¹² für Wasserstoff, Methyl, Amino, gegebenenfalls durch Hydroxy substituiertes Alkyl- oder Dialkylamino mit 1 oder 2 C-Atomen im Alkylteil, Aminomethyl, Aminoethyl, gegebenenfalls durch Hydroxy substituiertes Alkyl- oder Dialkylaminomethyl mit 1 oder 2 C-Atomen im Alkylteil oder 1-Imidazolyl,
R¹³ für Wasserstoff, Hydroxy, Methoxy, Methylthio oder Halogen, Methyl, Hydroxymethyl,
R¹⁴ für Wasserstoff oder Methyl,
R¹⁵ für Wasserstoff, Methyl oder Ethyl,
R¹⁶ für Wasserstoff, Methyl oder Ethyl,
R¹⁷ für Wasserstoff, Methyl oder Ethyl,
R¹⁸ für Hydroxy, 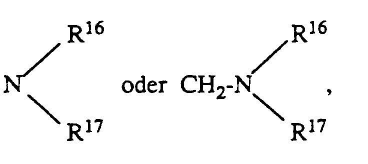
R¹⁹ für Wasserstoff, gegebenenfalls durch Hydroxy substituiertes C₁-C₃-Alkyl, Alkoxycarbonyl mit 1 bis 4 Kohlenstoffatomen im Alkoxyteil oder C₁-C₃-Acyl,
R²⁰ für Wasserstoff, Hydroxy, 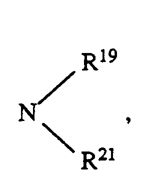 Hydroxymethyl oder 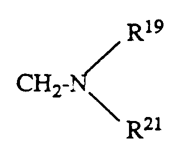 steht, worin
R²¹ Wasserstoff oder Methyl bedeutet,
A für CH₂, O oder für eine direkte Bindung steht und
n für 1 oder 2 steht,
und deren pharmazeutisch verwendbaren Hydrate und Säureadditionssalze sowie die Alkali-, Erdalkali-, Silber- und Guanidiniumsalze, dadurch gekennzeichnet, daß man eine Verbindung der Formel (II) 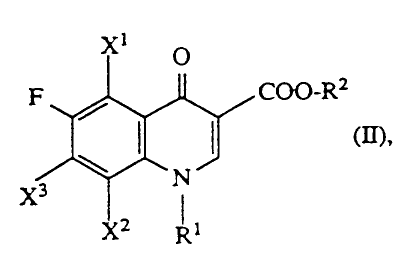 in welcher
R¹, R², X¹ und X² die obige Bedeutung haben und
X³ für Halogen, insbesondere Fluor oder Chlor, steht,
mit Verbindungen der Formel (III)
Y-H (III),
in welcher
Y die obige Bedeutung hat,
gegebenenfalls in Gegenwart von Säurefängern umsetzt, und die so erhaltenen Verbindungen gegebenenfalls auf an sich bekannte Weise in deren pharmazeutisch verwendbare Hydrate und Säureadditionssalze sowie die Alkali-, Erdalkali-, Silber- und Guanidiniumsalze überführt.

2. Verfahren gemäß Anspruch 1, dadurch gekennzeichnet, daß Chinoloncarbonsäurederivate der Formel (I) hergestellt werden
worin
R¹ für gegebenenfalls durch Hydroxy substituiertes C₁-C₂-Alkyl C₃-C₅-Cycloalkyl, Vinyl, Amino, Monoalkylamino mit 1 bis 2 C-Atomen, Dialkylamino mit 2 bis 4 C-Atomen oder für gegebenenfalls durch Halogen ein- oder zweifach substituiertes Phenyl steht,
R² für Wasserstoff, Alkyl mit 1 bis 3 Kohlenstoffatomen oder (5-Methyl-2-oxo-1,3-dioxol-4-yl)-methyl steht
X¹ für Wasserstoff, Fluor, Chlor, Amino oder Methyl,
X² für 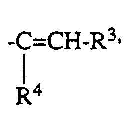 -C≡C-R⁵ oder -CH₂-CH=CH₂ steht,
worin
R³ Wasserstoff, C₁-C₂-Alkyl, Methoxy oder Methoxymethyl,
R⁴ Wasserstoff und
R⁵ Wasserstoff, gegebenenfalls durch Fluor ein- bis dreifach substituiertes C₁-C₄-Alkyl, C₂-C₃-Alkenyl, Methoxy oder Trimethylsilyl bedeutet und
Y für 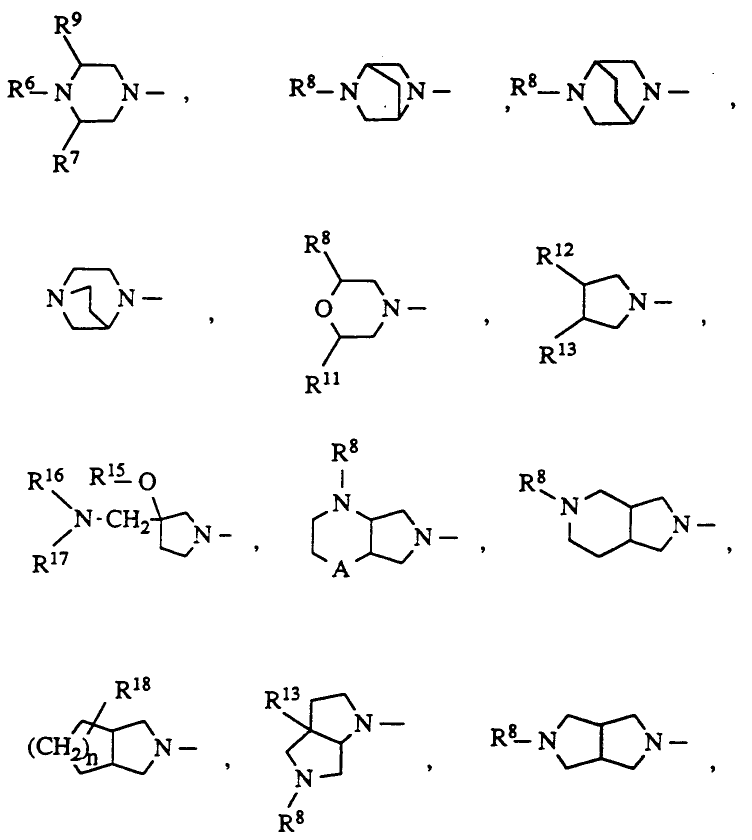 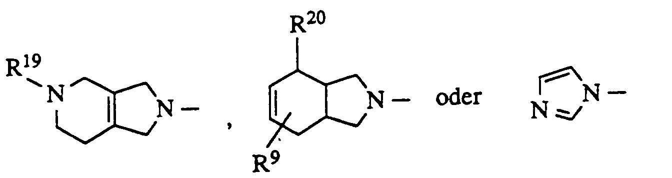 steht,
worin
R⁶ für Wasserstoff, gegebenenfalls durch Hydroxy substituiertes geradkettiges oder verzweigtes C₁-C₃-Alkyl, Oxoalkyl mit 1 bis 4 C-Atomen,
R⁷ für Wasserstoff, Methyl oder Phenyl,
R⁸ für Wasserstoff oder Methyl,
R⁹ für Wasserstoff oder Methyl,
R¹¹ für Wasserstoff, Methyl oder -CH₂-NH₂,
R¹² für Wasserstoff, Methyl, Amino, Methylamino, Dimethylamino, Aminomethyl, Methylaminomethyl oder Ethylaminomethyl,
R¹³ für Wasserstoff, Hydroxy, Methoxy, Fluor, Methyl oder Hydroxymethyl,
R¹⁵ für Wasserstoff oder Methyl,
R¹⁶ für Wasserstoff oder Methyl,
R¹⁷ für Wasserstoff oder Methyl,
R¹⁸ für 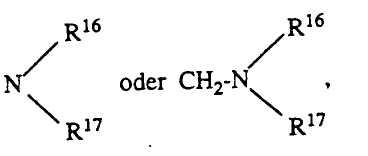
R¹⁹ für Wasserstoff, Methyl oder Ethyl,
R²⁰ für 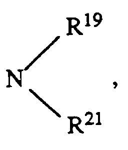 steht,
worin
R²¹ Wasserstoff oder Methyl bedeutet,
A für CH₂, O oder für eine direkte Bindung steht und
n für 1 oder 2 steht.

3. Verfahren gemaß Anspruch 1, dadurch gekennzeichnet, daß Chinoloncarbonsäurederivate der Formel (I) hergestellt werden
worin
R¹ für Methyl, Ethyl, Cyclopropyl oder gegebenenfalls durch Fluor ein- oder zweifach substituiertes Phenyl,
R² für Wasserstoff, Methyl oder Ethyl,
X¹ für Wasserstoff, Fluor, Chlor, Amino oder Methyl,
X² für -CH=CH₂ oder -C≡C-R⁵ steht,
worin
R⁵ Wasserstoff, C₁-C₄-Alkyl, C₂-C₃-Alkenyl oder Trimethylsilyl bedeutet und
Y für 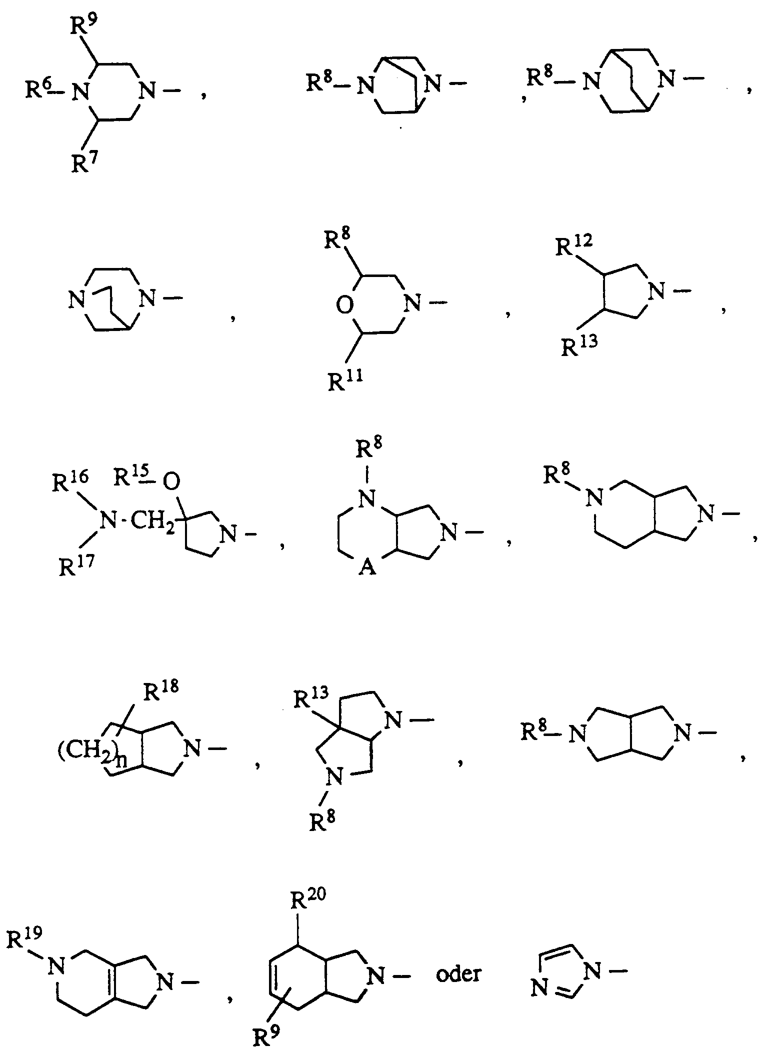 steht,
worin
R⁶ für Wasserstoff, Methyl, gegebenenfalls durch Hydroxy substituiertes Ethyl,
R⁷ für Wasserstoff oder Methyl,
R⁸ für Wasserstoff oder Methyl,
R⁹ für Wasserstoff oder Methyl,
R¹¹ für Wasserstoff oder -CH₂-NH₂,
R¹² für Wasserstoff, Methyl, Amino, Methylamino, Aminomethyl oder Ethylamininomethyl,
R¹³ für Wasserstoff, Hydroxy oder Methoxy,
R¹⁵ für Wasserstoff oder Methyl,
R¹⁶ für Wasserstoff oder Methyl,
R¹⁷ für Wasserstoff oder Methyl,
R¹⁸ für 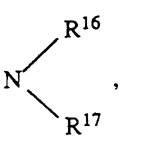
R¹⁹ für Wasserstoff oder Methyl,
R²⁰ für 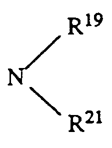 steht,
worin
R²¹ Wasserstoff oder Methyl bedeutet,
A für CH₂, O oder für eine direkte Bindung steht und
n für 1 steht.

4. Verfahren gemäß Anspruch 1, dadurch gekennzeichnet, daß Chinolincarbonsäurederivate aus der Gruppe bestehend aus
1-Cyclopropyl-8-ethinyl-6-fluor-1,4-dihydro-7-(4-methyl-1-piperazinyl)-4-oxo-3-chinolincarbonsäure,
1-Cyclopropyl-7-(cis-2,8-diazabicyclo[4.3.0]-non-8-yl)-8-ethinyl-6-fluor-1,4-dihydro-4-oxo-3-chinolincarbonsäure,
7-(3-Amino-1-pyrrolidinyl)-1-cyclopropyl-8-ethinyl-6-fluor-1,4-dihydro-4-oxo-3-chinolincarbonsäure,
7-(3-Amino-3-methyl-1-pyrrolidinyl)-1-cyclopropyl-8-ethinyl-6-fluor-1,4-dihydro-4-oxo-3-chinolincarbonsäure-Trifluoracetat,
1-Cyclopropyl-6-fluor-1,4-dihydro-8-(3-methyl-but-3-en-1-inyl)-7-(4-methyl-1-piperazinyl)-4-oxo-3-chinolincarbonsäure,
1-Cyclopropyl-8-ethinyl-6-fluor-1,4-dihydro-7-(2-oxa5,8-diazabicyclo[4.3.0]non-8-yl)-4-oxo-3-chinolincarbonsäure,
1-Cyclopropyl-8-ethinyl-6-fluor-1,4-dihydro-7-(7-methyl-3,7-diazabicyclo[3.3.0]oct-3-yl)-4-oxo-3-chinolincarbonsäure,
1-Cyclopropyl-8-ethinyl-6-fluor-1,4-dihydro-7-(4-methylamino-1,3,3a,4,7,7a-hexahydroisoindol-2-yl)-4-oxo-3-chinolincarbonsäure und
1-Cyclopropyl-7-(cis-2,8-diazabicyclo[4.3.0]non-8-yl6-fluor-1,4-dihydro-4-oxo-8-vinyl-3-chinolincarbonsäure und deren pharmakologisch verträgliche Hydrate und Säureadditionssalze sowie die Alkali-, Erdalkali-. Silber- und Guanidiniumsalze hergestellt werden.

5. Verwendung von gemäß Anspruch 1 erhältlichen Chinoloncarbonsäurederivaten bei der Herstellung von Arzneimitteln.

## Patentansprüche (Patentansprüche für folgende(n) Vertragsstaat(en): GR)

1. Chinoloncarbonsäurederivate der Formel (I) 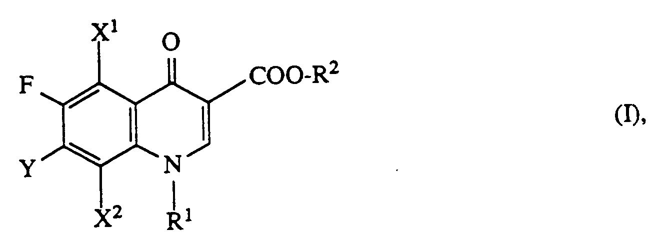 in welcher
R¹ für gegebenenfalls durch Hydroxy, Halogen oder C₁-C₃-Alkoxy substituiertes geradkettiges oder verzweigtes C₁-C₄-Alkyl, für gegebenenfalls durch Halogen oder C₁-C₃-Alkyl substituiertes C₃-C₆-Cycloalkyl, C₂-C₄-Alkenyl, ferner für C₁-C₃-Alkoxy, Amino, Monoalkylamino mit 1 bis 3 C-Atomen, Dialkylamino mit 2 bis 6 C-Atomen oder für gegebenenfalls durch Halogen ein- bis dreifach substituiertes Phenyl steht,
R² für Wasserstoff, Alkyl mit 1 bis 4 Kohlenstoffatomen oder (5-Methyl-2-oxo-1,3-dioxol-4-yl)-methyl steht
X¹ für Wasserstoff, Fluor, Chlor, Amino oder Methyl,
X² für 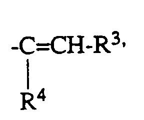 -C≡C-R⁵ oder -CH₂-CH=CH₂ steht,
worin
R³ Wasserstoff, C₁-C₃-Alkyl, C₁-C₃-Alkoxy oder Alkoxymethyl mit 1 bis 3 C-Atomen im Alkoxyteil,
R⁴ Wasserstoff oder Halogen und
R⁵ Wasserstoff, gegebenenfalls durch Halogen ein- bis dreifach substituiertes C₁-C₆-Alkyl, C₂-C₃-Alkenyl, Alkoxy mit 1 bis 3 C-Atomen, Alkoxymethyl mit 1 bis 3 C-Atomen im Alkoxyteil, Halogen oder Trimethylsilyl bedeutet und
Y für 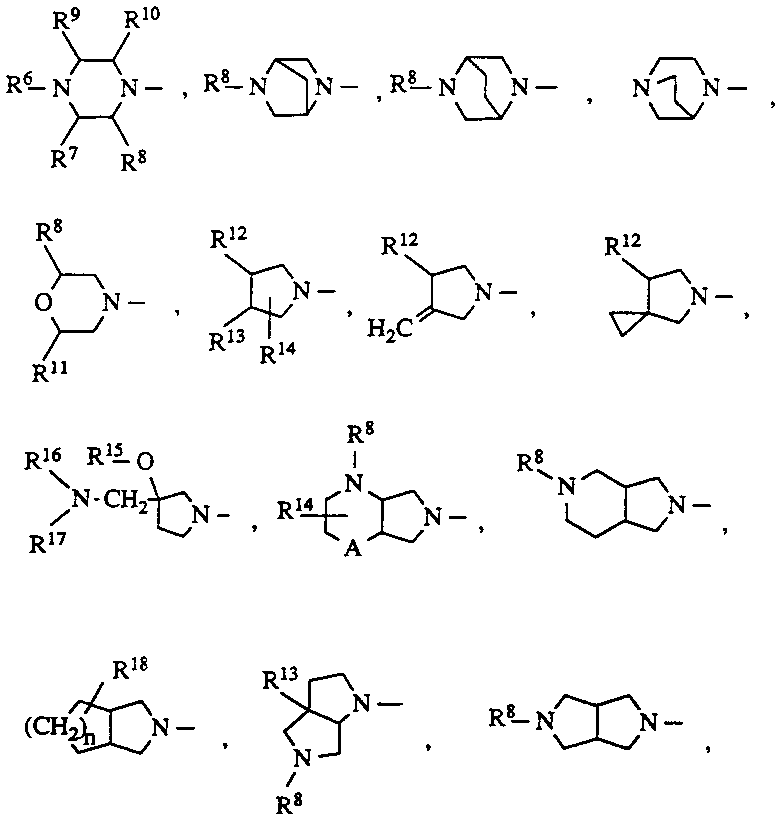 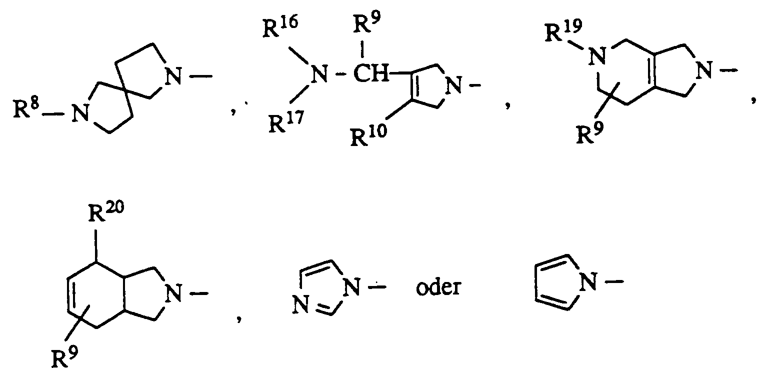 steht,
worin
R⁶ für Wasserstoff, gegebenenfalls durch Hydroxy oder Methoxy substituiertes geradkettiges oder verzweigtes C₁-C₄-Alkyl, Cyclopropyl, Oxoalkyl mit 1 bis 4 C-Atomen, Acyl mit 1 bis 3 C-Atomen,
R⁷ für Wasserstoff, Methyl, Phenyl, Thienyl oder Pyridyl,
R⁸ für Wasserstoff oder Methyl,
R⁹ für Wasserstoff oder Methyl,
R¹⁰ für Wasserstoff oder Methyl,
R¹¹ für Wasserstoff, Methyl oder 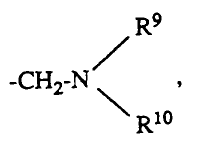
R¹² für Wasserstoff, Methyl, Amino, gegebenenfalls durch Hydroxy substituiertes Alkyl- oder Dialkylamino mit 1 oder 2 C-Atomen im Alkylteil, Aminomethyl, Aminoethyl, gegebenenfalls durch Hydroxy substituiertes Alkyl- oder Dialkylaminomethyl mit 1 oder 2 C-Atomen im Alkylteil oder 1-Imidazolyl,
R¹³ für Wasserstoff, Hydroxy` Methoxy, Methylthio oder Halogen, Methyl, Hydroxymethyl`
R¹⁴ für Wasserstoff oder Methyl,
R¹⁵ für Wasserstoff, Methyl oder Ethyl,
R¹⁶ für Wasserstoff, Methyl oder Ethyl,
R¹⁷ für Wasserstoff, Methyl oder Ethyl,
R¹⁸ für Hydroxy, 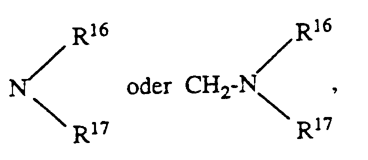
R¹⁹ für Wasserstoff, gegebenenfalls durch Hydroxy substituiertes C₁-C₃-Alkyl, Alkoxycarbonyl mit 1 bis 4 Kohlenstoffatomen im Alkoxyteil oder C₁-C₃-Acyl,
R²⁰ für Wasserstoff, Hydroxy, 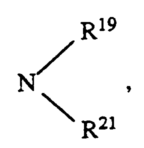 Hydroxymethyl oder 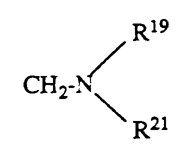 steht, worin
R²¹ Wasserstoff oder Methyl bedeutet,
A für CH₂, O oder für eine direkte Bindung seht und
n für 1 oder 2 steht,
und deren Hydrate und Säureadditionssalze sowie die Alkali-, Erdalkali-, Sibler- und Guanidiniumsalze.

2. Chinoloncarbonsäurederivate gemäß Anspruch 1,
worin
R¹ für gegebenenfalls durch Hydroxy substituiertes C₁-C₂-Alkyl, C₃-C₅-Cycloalkyl, Vinyl, Amino, Monoalkylamino mit 1 bis 2 C-Atomen, Dialkylamino mit 2 bis 4 C-Atomen oder für gegebenenfalls durch Halogen ein- oder zweilach substituiertes Phenyl steht,
R² für Wasserstoff, Alkyl mit 1 bis 3 Kohlenstoffatomer oder (5-Methyl-2-oxo-1,3-dioxol-4-yl)-methyl steht
X¹ für Wasserstoff, Fluor, Chlor, Amino oder Methyl,
X² für 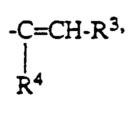 -C≡C-R⁵ oder -CH₂-CH=CH₂ steht,
worin
R³ Wasserstoff, C₁-C₂-Alkyl, Methoxy oder Methoxymethyl,
R⁴ Wasserstoff und
R⁵ Wasserstoff, gegebenenfalls durch Fluor ein- bis dreifach substituiertes C₁-C₄-Alkyl, C₂-C₃-Alkenyl, Methoxy oder Trimethylsilyl bedeutet und
Y für 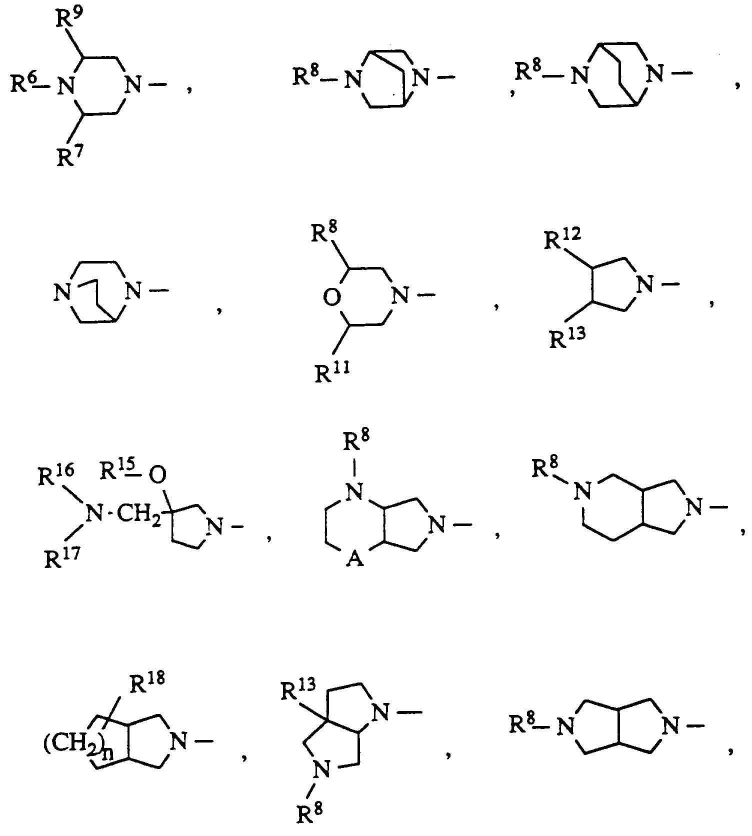 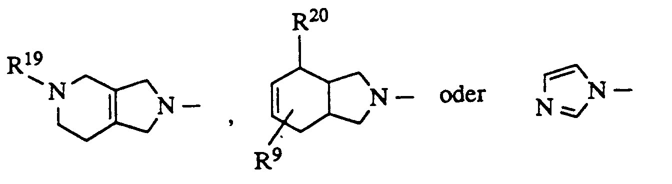 steht,
worin
R⁶ für Wasserstoff, gegebenenfalls durch Hydroxy substituiertes geradkettiges oder verzweigtes C₁-C₃-Alkyl, Oxoalkyl mit 1 bis 4 C-Atomen,
R⁷ für Wasserstoff, Methyl oder Phenyl,
R⁸ für Wasserstoff oder Methyl,
R⁹ für Wasserstoff oder Methyl,
R¹¹ für Wasserstoff, Methyl oder -CH₂-NH₂,
R¹² für Wasserstoff, Methyl, Amino, Methylamino, Dimethylamino, Aminomethyl, Methylaminomethyl oder Ethylaminomethyl,
R¹³ für Wasserstoff, Hydroxy, Methoxy, Fluor, Methyl oder Hydroxymethyl,
R¹⁵ für Wasserstoff oder Methyl,
R¹⁶ für Wasserstoff oder Methyl,
R¹⁷ für Wasserstoff oder Methyl,
R¹⁸ für 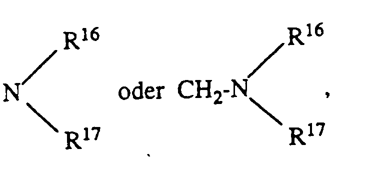
R¹⁹ für Wasserstoff, Methyl oder Ethyl,
R²⁰ für 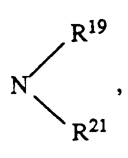 steht,
worin
R²¹ Wasserstoff oder Methyl bedeutet,
A für CH₂, O oder für eine direkte Bindung steht und
n für 1 oder 2 steht.

3. Chinoloncarbonsäurederivate gemäß Anspruch 1,
worin
R¹ für Methyl, Ethyl, Cyclopropyl oder gegebenenfalls durch Fluor ein- oder zweifach substituiertes Phenyl,
R² für Wasserstoff, Methyl oder Ethyl,
X¹ für Wasserstoff, Fluor, Chlor, Amino oder Methyl,
X² für -CH=CH₂ oder -C≡C-R⁵ steht,
worin
R⁵ Wasserstoff, C₁-C₄-Alkyl, C₂-C₃-Alkenyl oder Trimethylsilyl bedeutet und
Y für 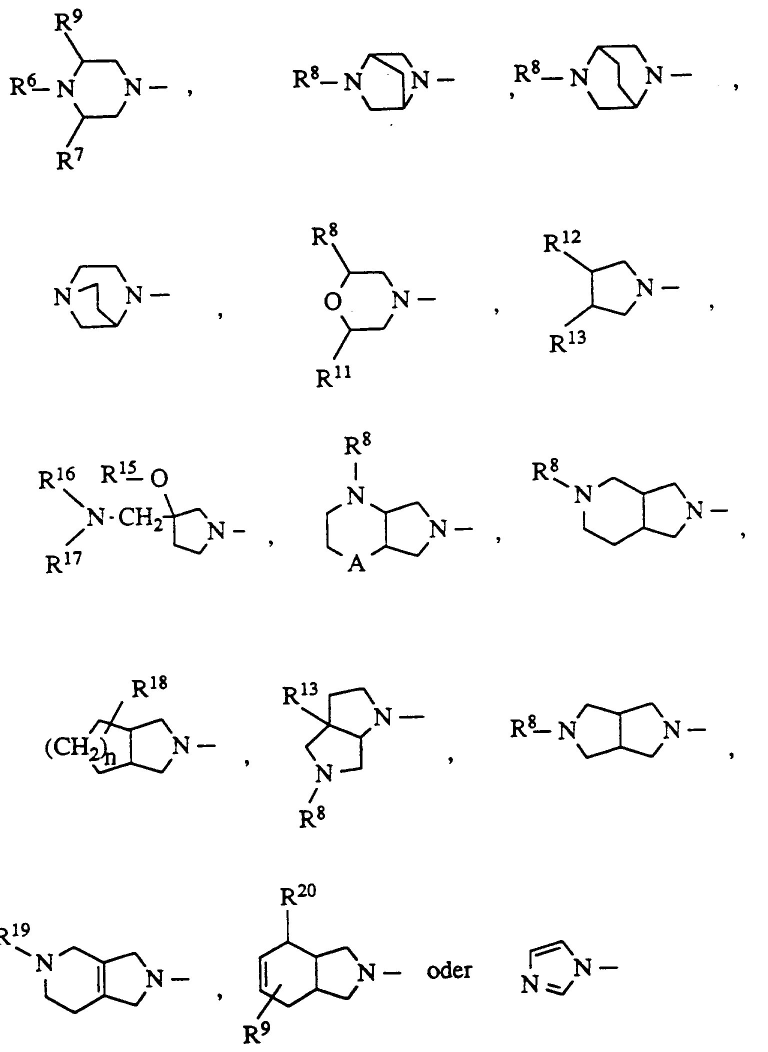 steht,
worin
R⁶ für Wasserstoff, Methyl, gegebenenfalls durch Hydroxy substituiertes Ethyl,
R⁷ für Wasserstoff oder Methyl,
R⁸ für Wasserstoff oder Methyl,
R⁹ für Wasserstoff oder Methyl,
R¹¹ für Wasserstoff oder -CH₂-NH₂,
R¹² für Wasserstoff, Methyl, Amino, Methylamino, Aminomethyl oder Ethylaminomethyl,
R¹³ für Wasserstoff, Hydroxy oder Methoxy,
R¹⁵ für Wasserstoff oder Methyl,
R¹⁶ für Wasserstoff oder Methyl,
R¹⁷ für Wasserstoff oder Methyl,
R¹⁸ für 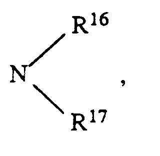
R¹⁹ für Wasserstoff oder Methyl,
R²⁰ für 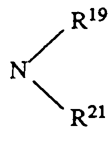 steht,
worin
R²¹ Wasserstoff oder Methyl bedeutet,
A für CH₂, O oder für eine direkte Bindung steht und
n für 1 steht,

4. Chinoloncarbonsäurederivate der Formel (II) 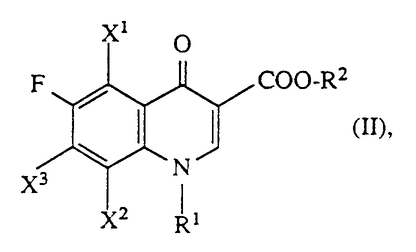 in welcher
R¹, R², X¹ und X² die Bedeutung gemäß Anspruch 1 haben und
X³ für Halogen, insbesondere Fluor oder Chlor, steht.

5. Chinolincarbonsäurederivate aus der Gruppe bestehend aus
1-Cyclopropyl-8-ethinyl-6-fluor-1,4-dihydro-7-(4-methyl-1-piperazinyl)-4-oxo-3-chinolincarbonsäure,
1-Cyclopropyl-7-(cis-2,8-diazabicyclo[4.3.0]-non-8-yl)-8-ethinyl-6-fluor-1,4-dihydro-4-oxo-3-chinolincarbonsäure,
7-(3-Amino-1-pyrrolidinyl)-1-cyclopropyl-8-ethinyl-6-fluor-1,4-dihydro-4-oxo-3-chinolincarbonsäure,
7-(3-Amino-3-methyl-1-pyrrolidinyl)-1-cyclopropyl-8-ethinyl-6-fluor-1,4-dihydro-4-oxo-3-chinolincarbonsäure-Trifluoracetat,
1-Cyclopropyl-6-fluor-1,4-dihydro-8-(3-methyl-but-3-en-1-inyl)-7-(4-methyl-1-piperazinyl)-4-oxo-3-chinolincarbonsäure,
1-Cyclopropyl-8-ethinyl-6-fluor-1,4-dihydro-7-(2-oxa5,8-diazabicyclo[4.3.0]non-8-yl)-4-oxo-3-chinolincarbonsäure,
1-Cyclopropyl-8-ethinyl-6-fluor-1,4-dihydro-7-(7-methyl-3,7-diazabicyclo[3.3.0]oct-3-yl)-4-oxo-3-chinolincarbonsäure,
1-Cyclopropyl-8-ethinyl-6-fluor-1,4-dihydro-7-(4-methylamino-1,3,3a,4,7,7a-hexahydroisoindol-2-yl)-4-oxo-3-chinolincarbonsäure und
1-Cyclopropyl-7-(cis-2,8-diazabicyclo[4.3.0]non-8-yl6-fluor-1,4-dihydro-4-oxo-8-vinyl-3-chinolincarbonsäure und deren
Hydrate und Säureadditionssalze sowie die Alkali-, Erdalkali-, Silber- und Guanidiniumsalze.

6. Chinolincarbonsäurederivate aus der Gruppe bestehend aus
8-Brom-1-(2,4-difluorphenyl)-6,7-difluor-1,4-dihydro-4-oxo-3-chinolincarbonsäure und
8-Brom-1-ethyl-6,7-difluor-1,4-dihydro-4-oxo-3-chinobonsäure und deren Hydrate und Säureadditionssalze sowie die Alkali-, Erdalkali-, Silber- und Guanidiniumsalze.

7. Verfahren zur Herstellung von Chinoloncarbonsäurederivaten gemäß Anspruch 1, dadurch gekennzeichnet, daß man eine Verbindung der Formel (II) 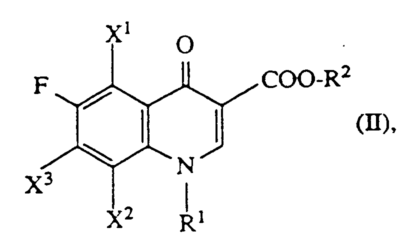 in welcher
R¹, R², X¹ und X² die Bedeutung gemäß Anspruch 1 haben und
X³ für Halogen, insbesondere Fluor oder Chlor, steht,
mit Verbindungen der Formel (III)
Y-H (III),
in welcher
Y die Bedeutung gemäß Anspruch 1 hat,
gegebenenfalls in Gegenwart von Säurefängern umsetzt.

8. Chinoloncarbonsäurederivate gemäß Ansprüchen 1 bis 5 zur Behandlung von Krankheiten.

9. Verwendung von Chinoloncarbonsäurederivaten gemäß Ansprüchen 1 bis 5 bei der Herstellung von Arzneimitteln.

## Claims (Claims for the following Contracting State(s): AT, BE, CH, DE, DK, FR, GB, IT, LI, LU, MC, NL, PT, SE)

1. Quinolonecarboxylic acid derivatives of the formula (I) 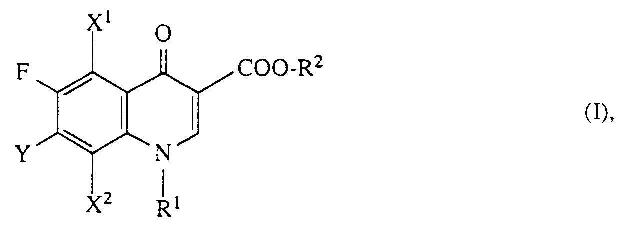 in which
R¹ represents straight-chain or branched C₁-C₄-alkyl which is optionally substituted by hydroxyl, halogen or C₁-C₃-alkoxy, or represents optionally halogen- or C₁-C₃-alkyl-substituted C₃-C₆-cycloalkyl, C₂-C₄-alkenyl, furthermore represents C₁-C₃-alkoxy, amino, monoalkylamino having 1 to 3 C atoms, dialkylamino having 2 to 6 C atoms, or phenyl which is optionally monosubstituted to trisubstituted by halogen,
R² represents hydrogen, alkyl having 1 to 4 carbon atoms or (5-methyl-2-oxo-1,3-dioxol-4-yl)-methyl,
X¹ represents hydrogen, fluorine, chlorine, amino or methyl,
X² represents 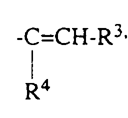 -C≡C-R⁵ or -CH₂-CH=CH₂,
where
R³ represents hydrogen, C₁-C₃-alkyl, C₁-C₃-alkoxy or alkoxymethyl having 1 to 3 C atoms in the alkoxy moiety,
R⁴ represents hydrogen or halogen and
R⁵ represents hydrogen, C₁-C₆-alkyl which is optionally monosubstituted to trisubstituted by halogen, or C₂-C₃-alkenyl, alkoxy having 1 to 3 C atoms, alkoxymethyl having 1 to 3 C atoms in the alkoxy moiety, halogen or trimethylsilyl, and
Y represents 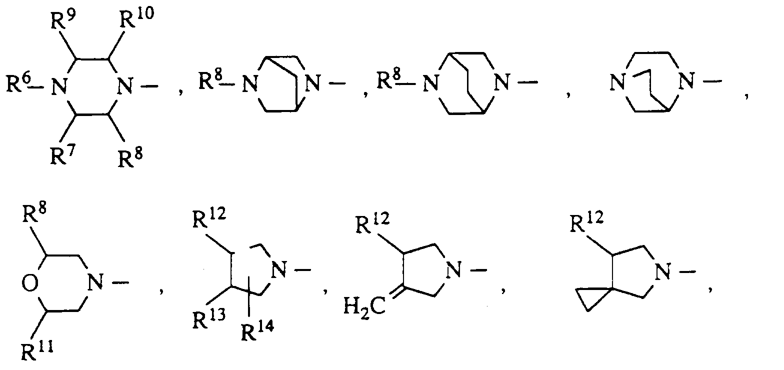 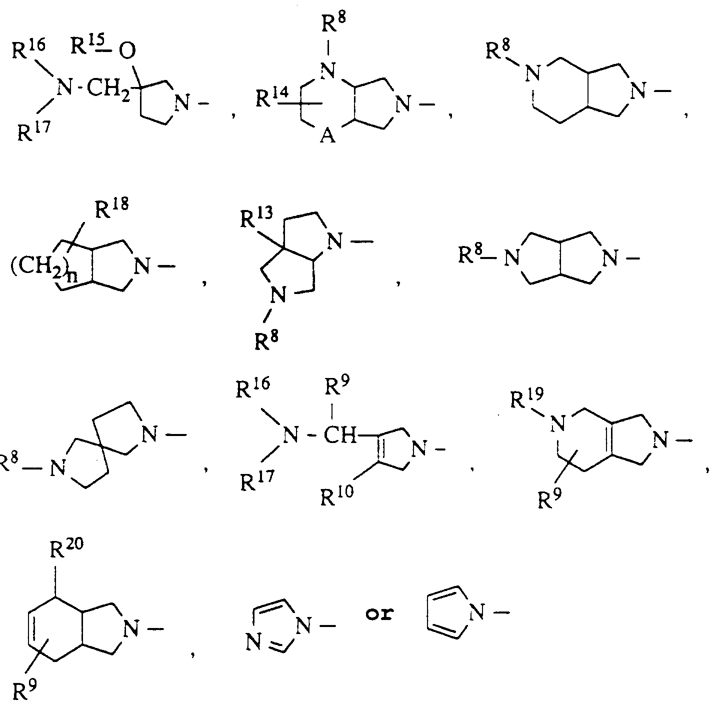 where
R⁶ represents hydrogen, optionally hydroxyl- or methoxy-substituted straight-chain or branched C₁-C₄-alkyl, cyclopropyl, oxoalkyl having 1 to 4 C atoms or acyl having 1 to 3 C atoms,
R⁷ represents hydrogen, methyl, phenyl, thienyl or pyridyl,
R⁸ represents hydrogen or methyl,
R⁹ represents hydrogen or methyl,
R¹⁰ represents hydrogen or methyl,
R¹¹ represents hydrogen, methyl or 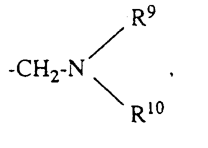
R¹² represents hydrogen, methyl, amino, optionally hydroxyl-substituted alkyl- or dialkylamino having 1 or 2 C atoms in the alkyl moiety, aminomethyl, aminoethyl, optionally hydroxyl-substituted alkyl- or dialkylaminomethyl having 1 or 2 C atoms in the alkyl moiety or 1-imidazolyl,
R¹³ represents hydrogen, hydroxyl, methoxy, methylthio or halogen, methyl or hydroxymethyl,
R¹⁴ represents hydrogen or methyl,
R¹⁵ represents hydrogen, methyl or ethyl,
R¹⁶ represents hydrogen, methyl or ethyl,
R¹⁷ represents hydrogen, methyl or ethyl,
R¹⁸ represents hydroxyl, 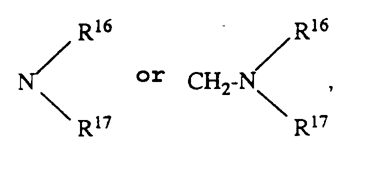
R¹⁹ represents hydrogen, optionally hydroxyl-substituted C₁-C₃-alkyl, alkoxycarbonyl having 1 to 4 carbon atoms in the alkoxy moiety or C₁-C₃-acyl,
R²⁰ represents hydrogen, hydroxyl, 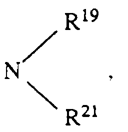 hydroxymethyl or 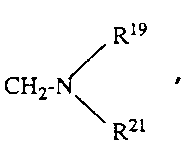 where
R²¹ denotes hydrogen or methyl,
A represents CH₂, O or a direct bond and
n represents 1 or 2,
and their pharmaceutically acceptable hydrates and acid addition salts as well as the alkali metal salts, alkaline earth metal salts, silver salts and guanidinium salts.

2. Quinolonecarboxylic acid derivatives according to Claim 1,
where
R¹ represents optionally hydroxyl-substituted C₁-C₂-alkyl, C₃-C₅-cycloalkyl, vinyl, amino, monoalkylamino having 1 to 2 C atoms, dialkylamino having 2 to 4 C atoms, or phenyl which is optionally monosubstituted or disubstituted by halogen,
R² represents hydrogen, alkyl having 1 to 3 carbon atoms or (5-methyl-2-oxo-1,3-dioxol-4-yl)-methyl,
X¹ represents hydrogen, fluorine, chlorine, amino or methyl,
X² represents 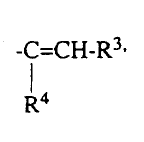 -C≡C-R⁵ or -CH₂-CH=CH₂ ,
where
R³ represents hydrogen, C₁-C₂-alkyl, methoxy or methoxymethyl,
R⁴ represents hydrogen and
R⁵ represents hydrogen, C₁-C₄-alkyl which is optionally monosubstituted to trisubstituted by fluorine, or C₂-C₃-alkenyl, methoxy or trimethylsilyl, and
Y represents 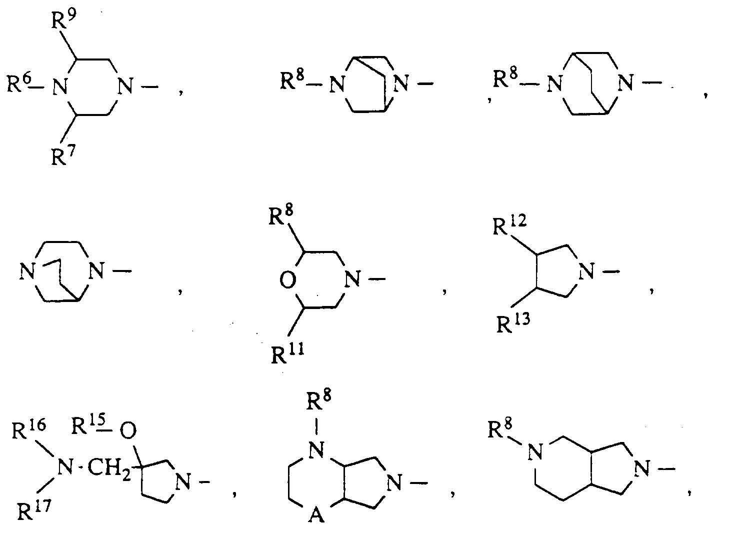 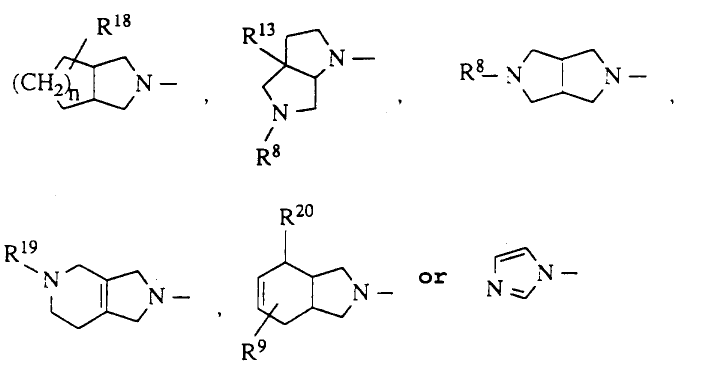 where
R⁶ represents hydrogen, optionally hydroxyl-substituted straight-chain or branched C₁-C₃-alkyl, or oxoalkyl having 1 to 4 C atoms,
R⁷ represents hydrogen, methyl or phenyl,
R⁸ represents hydrogen or methyl,
R⁹ represents hydrogen or methyl,
R¹¹ represents hydrogen, methyl or -CH₂-NH₂,
R¹² represents hydrogen, methyl, amino, methylamino, dimethylamino, aminomethyl, methylaminomethyl or ethylaminomethyl,
R¹³ represents hydrogen, hydroxyl, methoxy, fluorine, methyl or hydroxymethyl,
R¹⁵ represents hydrogen or methyl,
R¹⁶ represents hydrogen or methyl,
R¹⁷ represents hydrogen or methyl,
R¹⁸ represents 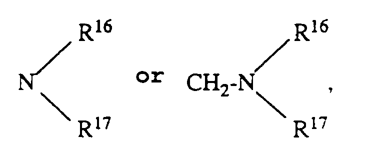
R¹⁹ represents hydrogen, methyl or ethyl,
R²⁰ represents 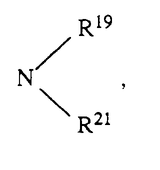 where
R²¹ denotes hydrogen or methyl,
A represents CH₂, O or a direct bond and
n represents 1 or 2.

3. Quinolonecarboxylic acid derivatives according to Claim 1,
where
R¹ represents methyl, ethyl, cyclopropyl or phenyl which is optionally monosubstituted or disubstituted by fluorine,
R² represents hydrogen, methyl or ethyl,
X¹ represents hydrogen, fluorine, chlorine, amino or methyl,
X² represents -CH=CH₂ or -C≡C-R⁵
where
R⁵ denotes hydrogen, C₁-C₄-alkyl, C₂-C₃-alkenyl or trimethylsilyl and
Y represents 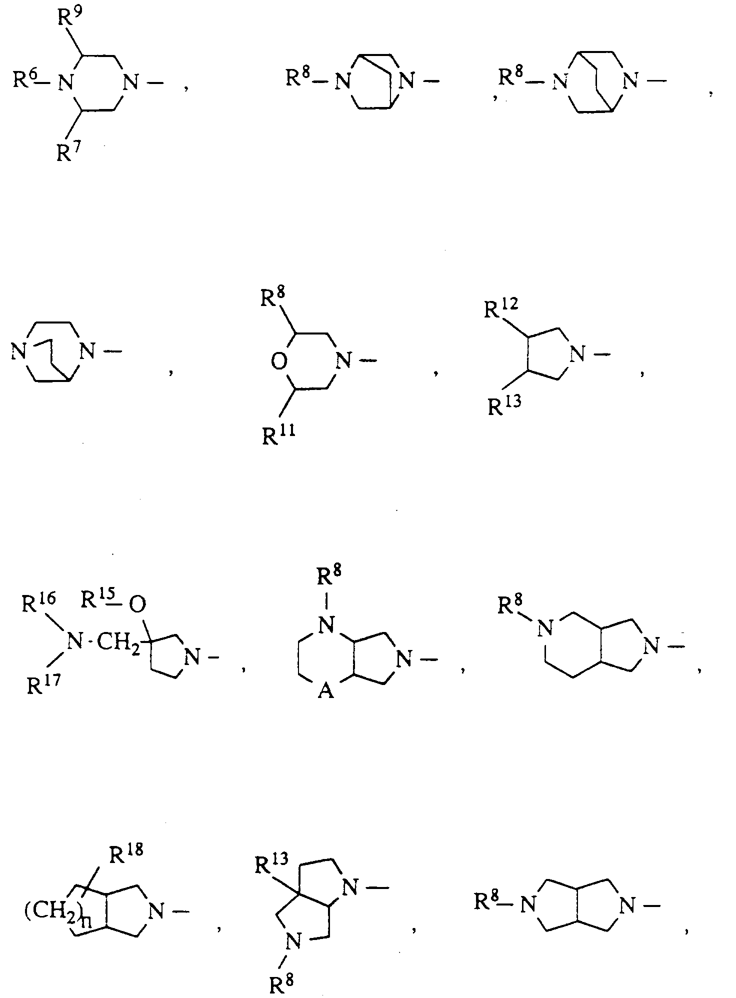 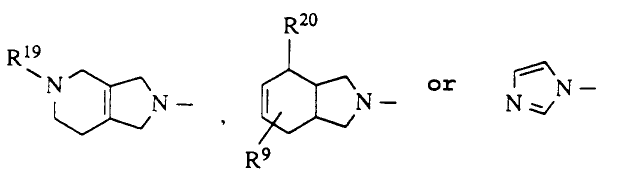 where
R⁶ represents hydrogen, methyl, optionally hydroxyl-substituted ethyl,
R⁷ represents hydrogen or methyl,
R⁸ represents hydrogen or methyl,
R⁹ represents hydrogen or methyl,
R¹¹ represents hydrogen or -CH₂-NH₂,
R¹² represents hydrogen, methyl, amino, methylamino, aminomethyl or ethylaminomethyl,
R¹³ represents hydrogen, hydroxyl or methoxy,
R¹⁵ represents hydrogen or methyl,
R¹⁶ represents hydrogen or methyl,
R¹⁷ represents hydrogen or methyl,
R¹⁸ represents 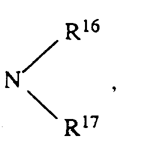
R¹⁹ represents hydrogen or methyl,
R²⁰ represents 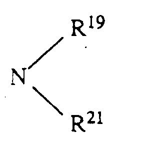 where
R²¹ denotes hydrogen or methyl,
A represents CH₂, O or a direct bond and
n represents 1.

4. Quinolonecarboxylic acid derivatives of the formula (II) 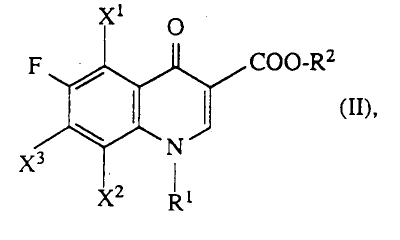 in which
R¹, R², X¹ and X² are as defined in Claim 1 and
X³ represents halogen, in particular fluorine or chlorine.

5. Quinolinecarboxylic acid derivatives of the group consisting of
1-cyclopropyl-8-ethinyl-6-fluoro-1,4-dihydro-7-(4-methyl-1-piperazinyl)-4-oxo-3-quinolinecarboxylic acid,
1-cyclopropyl-7-(cis-2,8-diazabicylco[4.3.0]-non-8-yl)-8-ethinyl-6-fluoro-1,4-dihydro-4-oxo-3-quinolinecarboxylic acid,
7-(3-amino-1-pyrrolidinyl)-1-cyclopropyl-8-ethinyl-6-fluoro-1,4-dihydro-4-oxo-3-quinolinecarboxylic acid,
7-(3-amino-3-methyl-1-pyrrolidinyl)-1-cyclopropyl-8-ethinyl-6-fluoro-1,4-dihydro-4-oxo-3-quinolinecarboxylic acid trifluoroacetate,
1-cyclopropyl-6-fluoro-1,4-dihydro-8-(3-methyl-but-3-en-1-inyl)-7-(4-methyl-1-piperazinyl)-4-oxo-3-quinolinecarboxylic acid,
1-cyclopropyl-8-ethinyl-6-fluoro-1,4-dihydro-7-(2-oxa-5,8-diazabicyclo[4.3.0]non-8-yl)-4-oxo-3-quinoline- carboxylic acid,
1-cyclopropyl-8-ethinyl-6-fluoro-1,4-dihydro-7-(7-methyl-3,7-diazabicyclo[3.3.0]oct-3-yl)-4-oxo-3-quinolinecarboxylic acid,
1-cyclopropyl-8-ethinyl-6-fluoro-1,4-dihydro-7-(4-methylamino-1,3,3a,4,7,7a-hexahydroisoindol-2-yl)-4-oxo-3-quinolinecarboxylic acid and
1-cyclopropyl-7-(cis-2,8-diazabicylco[4.3.0]non-8-yl-6-fluoro-1,4-dihydro-4-oxo-8-vinyl-3-quinolinecarboxylic acid and their pharmacologically acceptable hydrates and acid addition salts and also the alkali metal, alkaline earth metal, silver and guanidinium salts.

6. Quinolinecarboxylic acid derivatives from the group consisting of
8-bromo-1-(2,4-difluorophenyl)-6,7-difluoro-1,4-dihydro-4-oxo-3-quinolinecarboxylic acid and
8-bromo-1-ethyl-6,7-difluoro-1,4-dihydro-4-oxo-3-chinolinecarboxylic acid and their hydrates and acid addition salts and also the alkali metal, alkaline earth metal, silver and guanidinium salts.

7. Process for the preparation of quinolonecarboxylic acid derivatives according to Claim 1, characterised in that a compound of the formula (II) 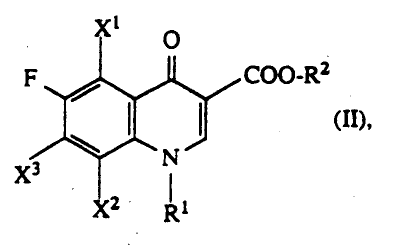 in which
R¹, R², X¹ and X² are as defined in Claim 1 and
X³ represents halogen, in particular fluorine or chlorine,
is reacted with compounds of the formula (III)
Y-H (III)
in which
Y is as defined in Claim 1,
if appropriate in the presence of acid scavengers.

8. Quinolonecarboxylic acid derivatives according to Claims 1 to 5 for treating diseases.

9. Medicament containing quinolonecarboxylic acid derivatives according to Claims 1 to 5.

10. Use of quinolonecarboxylic acid derivatives according to Claims 1 to 5 in the preparation of medicaments.

## Claims (Claims for the following Contracting State(s): ES)

1. Process for the preparation of quinolonecarboxylic acid derivatives of the formula (I) 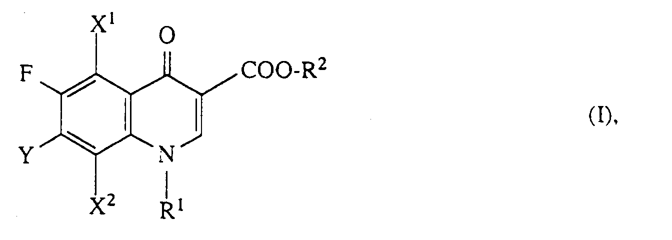 in which
R¹ represents straight-chain or branched C₁-C₄-alkyl which is optionally substituted by hydroxyl, halogen or C₁-C₃-alkoxy, or represents optionally halogen- or C₁-C₃-alkyl-substituted C₃-C₆-cycloalkyl, C₂-C₄-alkenyl, furthermore represents C₁-C₃-alkoxy, amino, monoalkylamino having 1 to 3 C atoms, dialkylamino having 2 to 6 C atoms, or phenyl which is optionally monosubstituted to trisubstituted by halogen,
R² represents hydrogen, alkyl having 1 to 4 carbon atoms or (5-methyl-2-oxo-1,3-dioxol-4-yl)-methyl,
X¹ represents hydrogen, fluorine, chlorine, amino or methyl,
X² represents 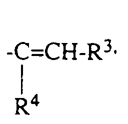 -C≡C-R⁵ or -CH₂-CH=CH₂,
where
R³ represents hydrogen, C₁-C₃-alkyl, C₁-C₃-alkoxy or alkoxymethyl having 1 to 3 C atoms in the alkoxy moiety,
R⁴ represents hydrogen or halogen and
R⁵ represents hydrogen, C₁-C₆-alkyl which is optionally monosubstituted to trisubstituted by halogen, or C₂-C₃-alkenyl, alkoxy having 1 to 3 C atoms, alkoxymethyl having 1 to 3 C atoms in the alkoxy moiety, halogen or trimethylsilyl, and
Y represents 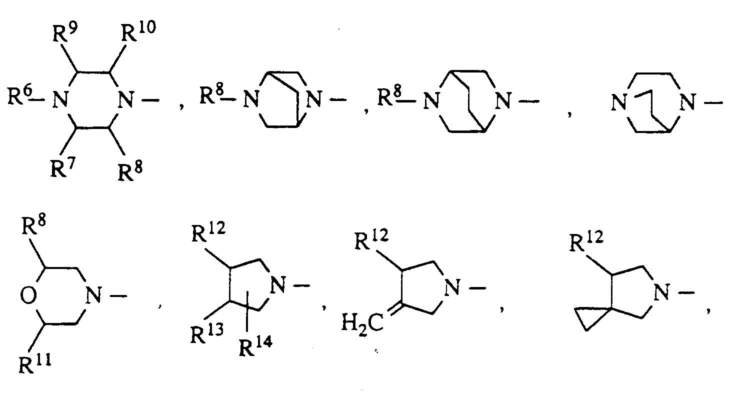 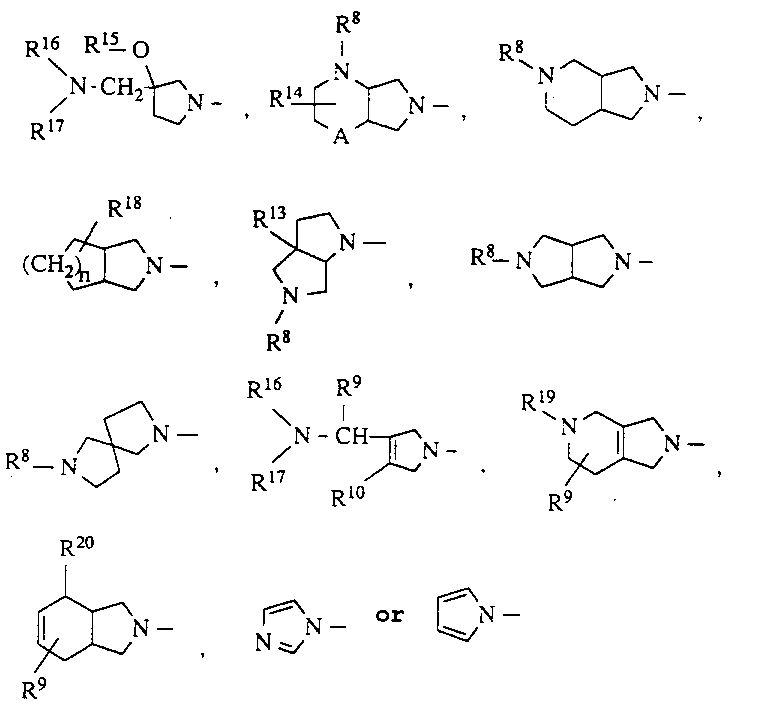 where
R⁶ represents hydrogen, optionally hydroxyl- or methoxy-substituted straight-chain or branched C₁-C₄-alkyl, cyclopropyl, oxoalkyl having 1 to 4 C atoms or acyl having 1 to 3 C atoms,
R⁷ represents hydrogen, methyl, phenyl, thienyl or pyridyl,
R⁸ represents hydrogen or methyl,
R⁹ represents hydrogen or methyl,
R¹⁰ represents hydrogen or methyl,
R¹¹ represents hydrogen, methyl or 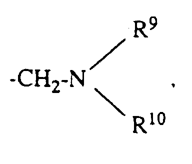
R¹² represents hydrogen, methyl, amino, optionally hydroxyl-substituted alkyl- or dialkylamino having 1 or 2 C atoms in the alkyl moiety, aminomethyl, aminoethyl, optionally hydroxyl-substituted alkyl- or dialkylaminomethyl having 1 or 2 C atoms in the alkyl moiety or 1-imidazolyl,
R¹³ represents hydrogen, hydroxyl, methoxy, methylthio or halogen, methyl or hydroxymethyl,
R¹⁴ represents hydrogen or methyl,
R¹⁵ represents hydrogen, methyl or ethyl,
R¹⁶ represents hydrogen, methyl or ethyl,
R¹⁷ represents hydrogen, methyl or ethyl,
R¹⁸ represents hydroxyl, 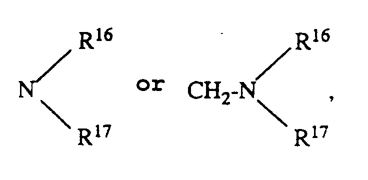
R¹⁹ represents hydrogen, optionally hydroxyl-substituted C₁-C₃-alkyl, alkoxycarbonyl having 1 to 4 carbon atoms in the alkoxy moiety or C₁-C₃-acyl,
R²⁰ represents hydrogen, hydroxyl, 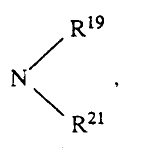 hydroxymethyl or 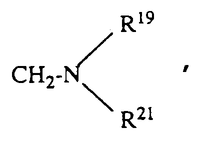 where
R²¹ denotes hydrogen or methyl,
A represents CH₂, O or a direct bond and
n represents 1 or 2,
and their pharmaceutically acceptable hydrates and acid addition salts as well as the alkali metal salts, alkaline earth metal salts, silver salts and guanidinium salts, characterised in that a compound of the formula (II) 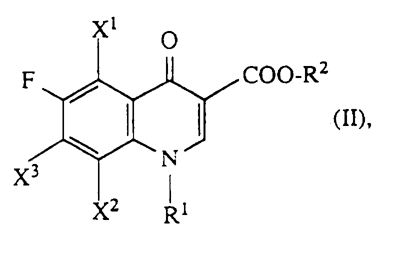 in which
R¹, R², X¹ and X² are as defined above and
X³ represents halogen, in particular fluorine or chlorine,
is reacted with compounds of the formula (III)
Y-H (III)
in which
Y is as defined above,
if appropriate in the presence of acid scavengers, and the compounds obtained in this manner are optionally converted in a manner known per se into their pharmaceutically usable hydrates and acid addition salts and also the alkali metal, alkaline earth metal, silver and guanidinium salts.

2. Process according to Claim 1, characterised in that quinolonecarboxylic acid derivatives of the formula (I) are prepared
where
R¹ represents optionally hydroxyl-substituted C₁-C₂-alkyl, C₃-C₅-cycloalkyl, vinyl, amino, monoalkylamino having 1 to 2 C atoms, dialkylamino having 2 to 4 C atoms, or phenyl which is optionally monosubstituted or disubstituted by halogen,
R² represents hydrogen, alkyl having 1 to 3 carbon atoms or (5-methyl-2-oxo-1,3-dioxol-4-yl)-methyl,
X¹ represents hydrogen, fluorine, chlorine, amino or methyl,
X² represents 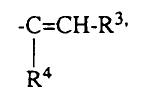 -C≡C-R⁵ or -CH₂-CH=CH₂ ,
where
R³ represents hydrogen, C₁-C₂-alkyl, methoxy or methoxymethyl,
R⁴ represents hydrogen and
R⁵ represents hydrogen, C₁-C₄-alkyl which is optionally monosubstituted to trisubstituted by fluorine, or C₂-C₃-alkenyl, methoxy or trimethylsilyl, and
Y represents 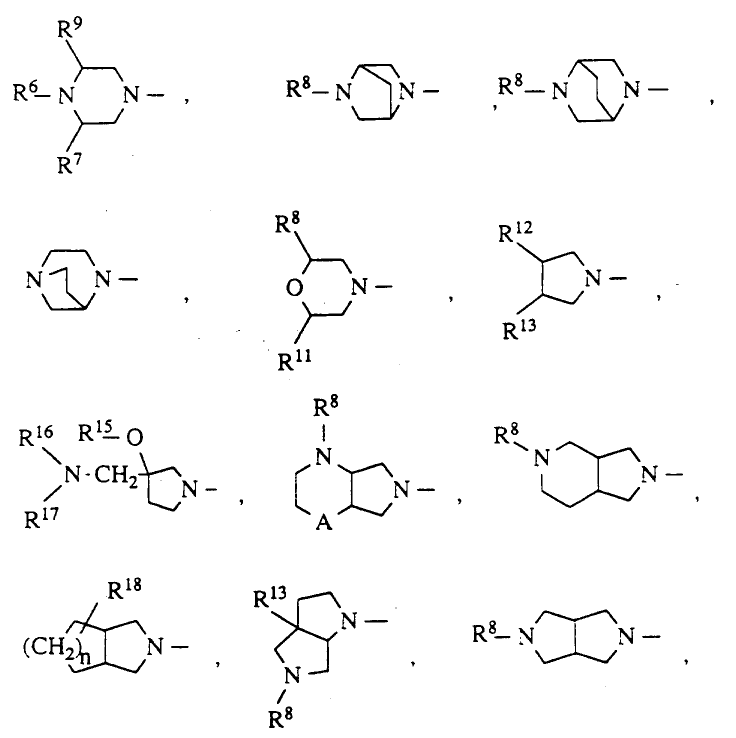 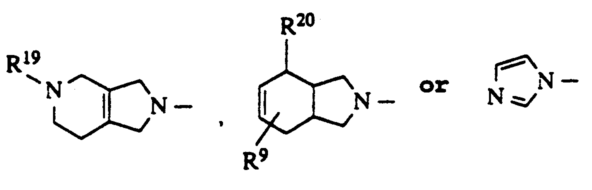 where
R⁶ represents hydrogen, optionally hydroxyl-substituted straight-chain or branched C₁-C₃-alkyl, or oxoalkyl having 1 to 4 C atoms,
R⁷ represents hydrogen, methyl or phenyl,
R⁸ represents hydrogen or methyl,
R⁹ represents hydrogen or methyl,
R¹¹ represents hydrogen, methyl or -CH₂-NH₂,
R¹² represents hydrogen, methyl, amino, methylamino, dimethylamino, aminomethyl, methylaminomethyl or ethylaminomethyl,
R¹³ represents hydrogen, hydroxyl, methoxy, fluorine, methyl or hydroxymethyl,
R¹⁵ represents hydrogen or methyl,
R¹⁶ represents hydrogen or methyl,
R¹⁷ represents hydrogen or methyl,
R¹⁸ represents 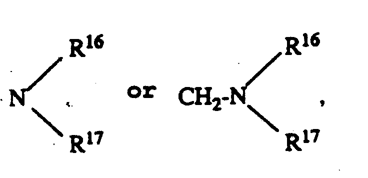
R¹⁹ represents hydrogen, methyl or ethyl,
R²⁰ represents 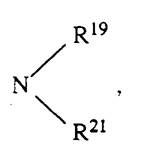 where
R²¹ denotes hydrogen or methyl,
A represents CH₂, O or a direct bond and
n represents 1 or 2.

3. Process according to Claim 1, characterised in that quinolonecarboxylic acid derivatives of the formula (I) are prepared
where
R¹ represents methyl, ethyl, cyclopropyl or phenyl which is optionally monosubstituted or disubstituted by fluorine,
R² represents hydrogen, methyl or ethyl,
X¹ represents hydrogen, fluorine, chlorine, amino or methyl,
X² represents -CH=CH₂ or -C≡C-R⁵
where
R⁵ denotes hydrogen, C₁-C₄-alkyl, C₂-C₃-alkenyl or trimethylsilyl and
Y represents 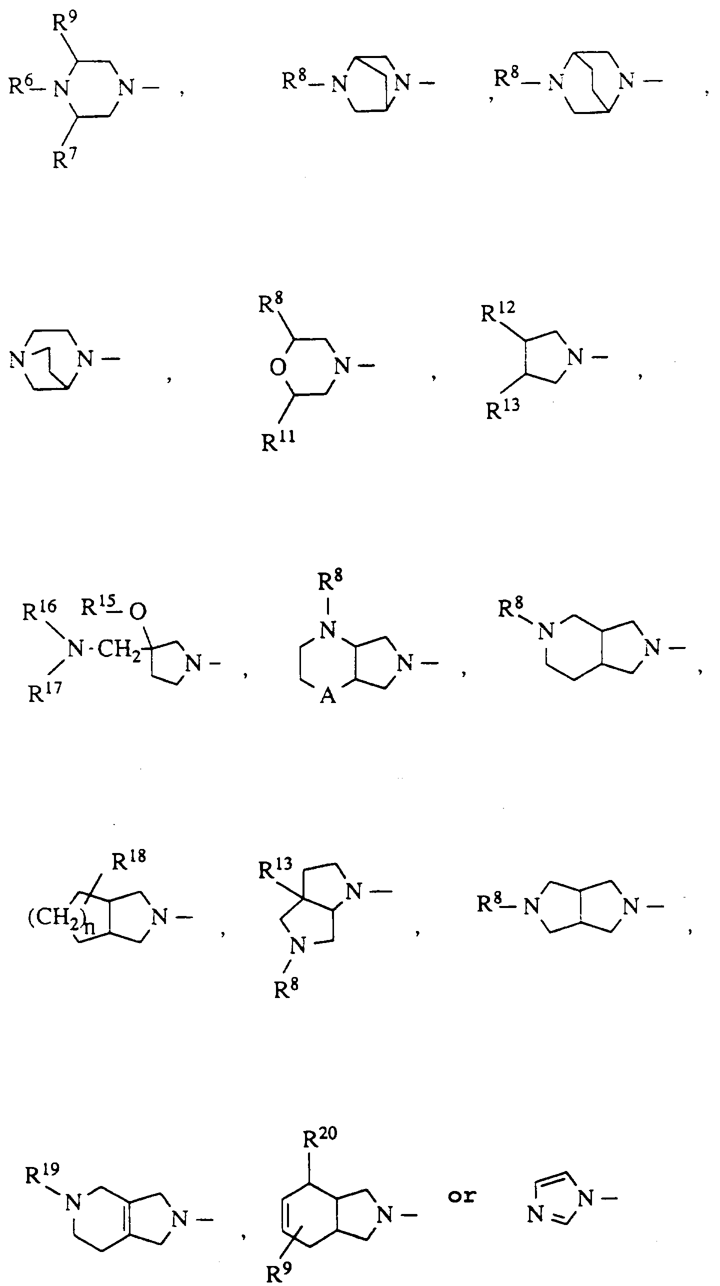 where
R⁶ represents hydrogen, methyl, optionally hydroxyl-substituted ethyl,
R⁷ represents hydrogen or methyl,
R⁸ represents hydrogen or methyl,
R⁹ represents hydrogen or methyl,
R¹¹ represents hydrogen or -CH₂-NH₂,
R¹² represents hydrogen, methyl, amino, methylamino, aminomethyl or ethylaminomethyl,
R¹³ represents hydrogen, hydroxyl or methoxy,
R¹⁵ represents hydrogen or methyl,
R¹⁶ represents hydrogen or methyl,
R¹⁷ represents hydrogen or methyl,
R¹⁸ represents 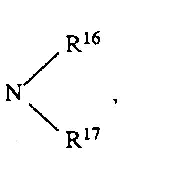
R¹⁹ represents hydrogen or methyl,
R²⁰ represents 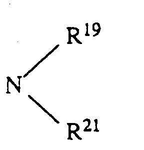 where
R²¹ denotes hydrogen or methyl,
A represents CH₂, O or a direct bond and
n represents 1.

4. Process according to Claim 1, characterised in that quinolinecarboxylic acid derivatives from the group consisting of
1-cyclopropyl-8-ethinyl-6-fluoro-1,4-dihydro-7-(4-methyl-1-piperazinyl)-4-oxo-3-quinolinecarboxylic acid,
1-cyclopropyl-7-(cis-2,8-diazabicylco[4.3.0]-non-8-yl)-8-ethinyl-6-fluoro-1,4-dihydro-4-oxo-3-quinolinecarboxylic acid,
7-(3-amino-1-pyrrolidinyl)-1-cyclopropyl-8-ethinyl-6-fluoro-1,4-dihydro-4-oxo-3-quinolinecarboxylic acid,
7-(3-amino-3-methyl-1-pyrrolidinyl)-1-cyclopropyl-8-ethinyl-6-fluoro-1,4-dihydro-4-oxo-3-quinolinecarboxylic acid trifluoroacetate,
1-cyclopropyl-6-fluoro-1,4-dihydro-8-(3-methyl-but-3-en-1-inyl)-7-(4-methyl-1-piperazinyl)-4-oxo-3-quinolinecarboxylic acid,
1-cyclopropyl-8-ethinyl-6-fluoro-1,4-dihydro-7-(2-oxa-5,8-diazabicyclo[4.3.0]non-8-yl)-4-oxo-3-quinolinecarboxylic acid,
1-cyclopropyl-8-ethinyl-6-fluoro-1,4-dihydro-7-(7-methyl-3,7-diazabicyclo[3.3.0]oct-3-yl)-4-oxo-3- quinolinecarboxylic acid,
1-cyclopropyl-8-ethinyl-6-fluoro-1,4-dihydro-7-(4-methylamino-1,3,3a,4,7,7a-hexahydroisoindol-2-yl)-4-oxo-3-quinolinecarboxylic acid and
1-cyclopropyl-7-(cis-2,8-diazabicylco[4.3.0]non-8-yl-6-fluoro-1,4-dihydro-4-oxo-8-vinyl-3-quinolinecarboxylic acid and their pharmacologically acceptable hydrates and acid addition salts and also the alkali metal, alkaline earth metal, silver and guanidinium salts are prepared.

5. Use of quinolonecarboxylic acid derivatives which can be obtained in accordance with Claim 1 in the preparation of medicaments.

## Claims (Claims for the following Contracting State(s): GR)

1. Quinolonecarboxylic acid derivatives of the formula (I) 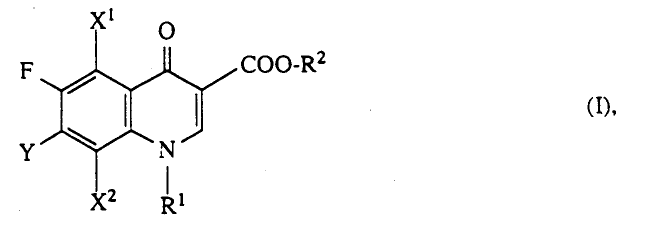 in which
R¹ represents straight-chain or branched C₁-C₄-alkyl which is optionally substituted by hydroxyl, halogen or C₁-C₃-alkoxy, or represents optionally halogen- or C₁-C₃-alkyl-substituted C₃-C₆-cycloalkyl, C₂-C₄-alkenyl, furthermore represents C₁-C₃-alkoxy, amino, monoalkylamino having 1 to 3 C atoms, dialkylamino having 2 to 6 C atoms, or phenyl which is optionally monosubstituted to trisubstituted by halogen,
R² represents hydrogen, alkyl having 1 to 4 carbon atoms or (5-methyl-2-oxo-1,3-dioxol-4-yl)-methyl,
X¹ represents hydrogen, fluorine, chlorine, amino or methyl,
X² represents 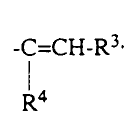 -C≡C-R⁵ or -CH₂-CH=CH₂,
where
R³ represents hydrogen, C₁-C₃-alkyl, C₁-C₃-alkoxy or alkoxymethyl having 1 to 3 C atoms in the alkoxy moiety,
R⁴ represents hydrogen or halogen and
R⁵ represents hydrogen, C₁-C₆-alkyl which is optionally monosubstituted to trisubstituted by halogen, or C₂-C₃-alkenyl, alkoxy having 1 to 3 C atoms, alkoxymethyl having 1 to 3 C atoms in the alkoxy moiety, halogen or trimethylsilyl, and
Y represents 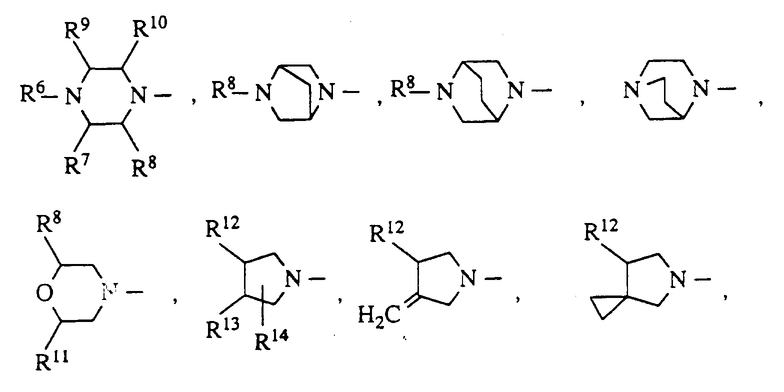 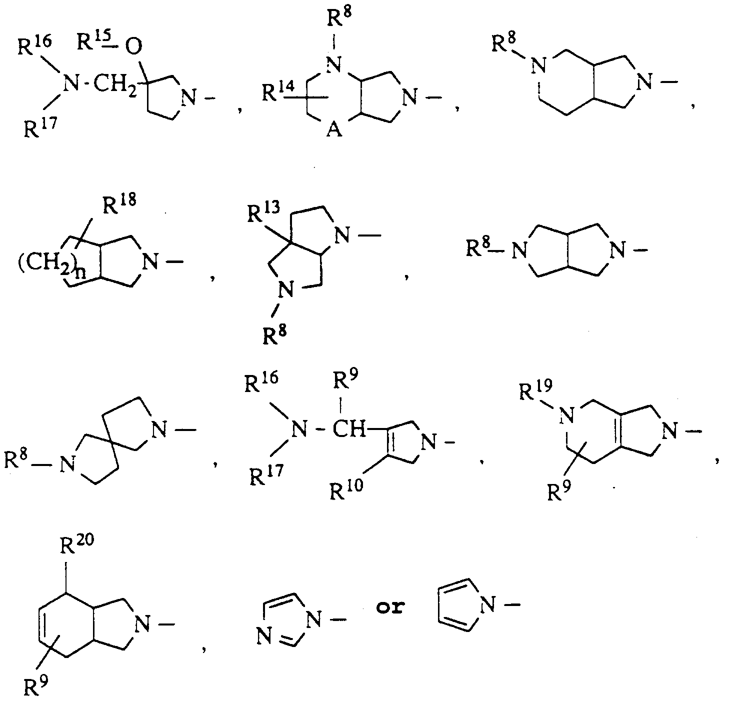 where
R⁶ represents hydrogen, optionally hydroxyl- or methoxy- substituted straight-chain or branched C₁-C₄-alkyl, cyclopropyl, oxoalkyl having 1 to 4 C atoms or acyl having 1 to 3 C atoms,
R⁷ represents hydrogen, methyl, phenyl, thienyl or pyridyl,
R⁸ represents hydrogen or methyl,
R⁹ represents hydrogen or methyl,
R¹⁰ represents hydrogen or methyl,
R¹¹ represents hydrogen, methyl or 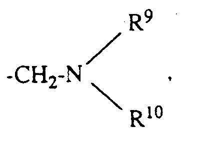
R¹² represents hydrogen, methyl, amino, optionally hydroxyl-substituted alkyl- or dialkylamino having 1 or 2 C atoms in the alkyl moiety, aminomethyl, aminoethyl, optionally hydroxyl-substituted alkyl- or dialkylaminomethyl having 1 or 2 C atoms in the alkyl moiety or 1-imidazolyl,
R¹³ represents hydrogen, hydroxyl, methoxy, methylthio or halogen, methyl or hydroxymethyl,
R¹⁴ represents hydrogen or methyl,
R¹⁵ represents hydrogen, methyl or ethyl,
R¹⁶ represents hydrogen, methyl or ethyl,
R¹⁷ represents hydrogen, methyl or ethyl,
R¹⁸ represents hydroxyl, 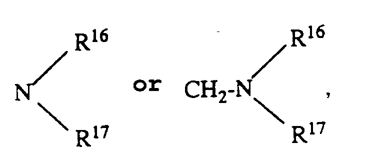
R¹⁹ represents hydrogen, optionally hydroxyl-substituted C₁-C₃-alkyl, alkoxycarbonyl having 1 to 4 carbon atoms in the alkoxy moiety or C₁-C₃-acyl,
R²⁰ represents hydrogen, hydroxyl, 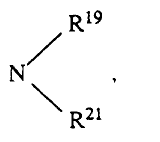 hydroxymethyl or 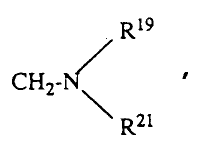 where
R²¹ denotes hydrogen or methyl,
A represents CH₂, O or a direct bond and
n represents 1 or 2,
and their hydrates and acid addition salts as well as the alkali metal salts, alkaline earth metal salts, silver salts and guanidinium salts.

2. Quinolonecarboxylic acid derivatives according to Claim 1,
where
R¹ represents optionally hydroxyl-substituted C₁-C₂-alkyl, C₃-C₅-cycloalkyl, vinyl, amino, monoalkylamino having 1 to 2 C atoms, dialkylamino having 2 to 4 C atoms, or phenyl which is optionally monosubstituted or disubstituted by halogen,
R² represents hydrogen, alkyl having 1 to 3 carbon atoms or (5-methyl-2-oxo-1,3-dioxol-4-yl)-methyl,
X¹ represents hydrogen, fluorine, chlorine, amino or methyl,
X² represents 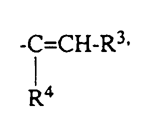 -C≡C-R⁵ or -CH₂-CH=CH₂ ,
where
R³ represents hydrogen, C₁-C₂-alkyl, methoxy or methoxymethyl,
R⁴ represents hydrogen and
R⁵ represents hydrogen, C₁-C₄-alkyl which is optionally monosubstituted to trisubstituted by fluorine, or C₂-C₃-alkenyl, methoxy or trimethylsilyl, and
Y represents 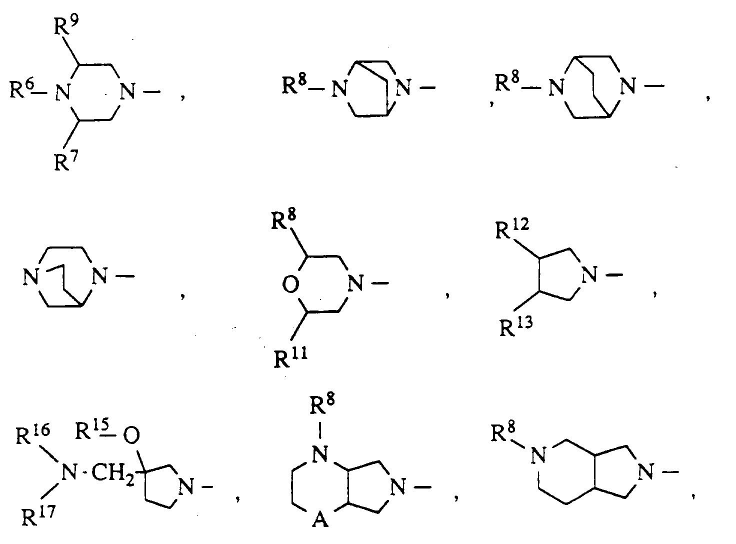 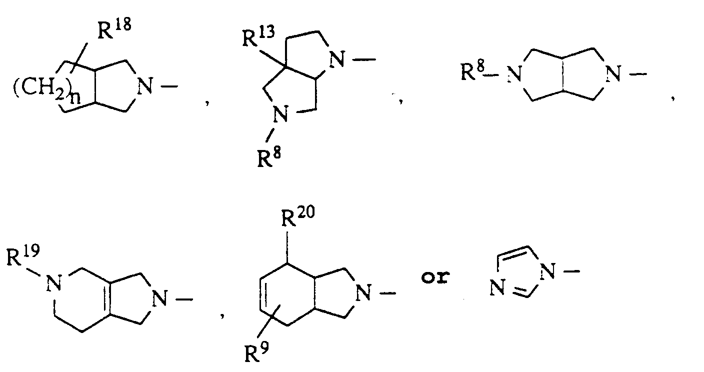 where
R⁶ represents hydrogen, optionally hydroxyl-substituted straight-chain or branched C₁-C₃-alkyl, or oxoalkyl having 1 to 4 C atoms,
R⁷ represents hydrogen, methyl or phenyl,
R⁸ represents hydrogen or methyl,
R⁹ represents hydrogen or methyl,
R¹¹ represents hydrogen, methyl or -CH₂-NH₂,
R¹² represents hydrogen, methyl, amino, methylamino, dimethylamino, aminomethyl, methylaminomethyl or ethylaminomethyl,
R¹³ represents hydrogen, hydroxyl, methoxy, fluorine, methyl or hydroxymethyl,
R¹⁵ represents hydrogen or methyl,
R¹⁶ represents hydrogen or methyl,
R¹⁷ represents hydrogen or methyl,
R¹⁸ represents 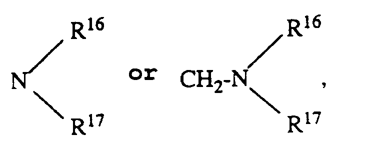
R¹⁹ represents hydrogen, methyl or ethyl,
R²⁰ represents 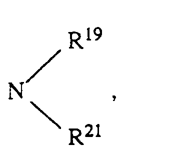 where
R²¹ denotes hydrogen or methyl,
A represents CH₂, O or a direct bond and
n represents 1 or 2.

3. Quinolonecarboxylic acid derivatives according to Claim 1,
where
R¹ represents methyl, ethyl, cyclopropyl or phenyl which is optionally monosubstituted or disubstituted by fluorine,
R² represents hydrogen, methyl or ethyl,
X¹ represents hydrogen, fluorine, chlorine, amino or methyl,
X² represents -CH=CH₂ or -C≡C-R⁵
where
R⁵ denotes hydrogen, C₁-C₄-alkyl, C₂-C₃-alkenyl or trimethylsilyl and
Y represents 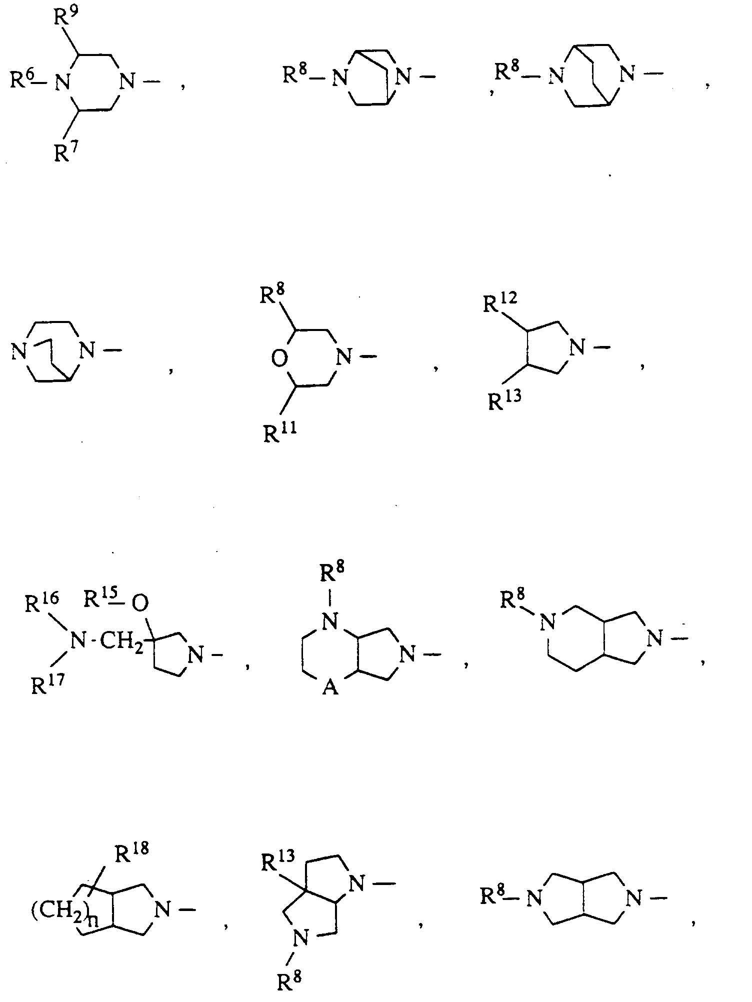 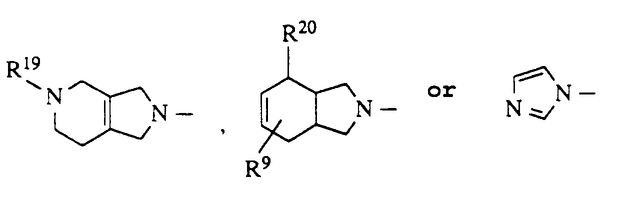 where
R⁶ represents hydrogen, methyl, optionally hydroxyl-substituted ethyl,
R⁷ represents hydrogen or methyl,
R⁸ represents hydrogen or methyl,
R⁹ represents hydrogen or methyl,
R¹¹ represents hydrogen or -CH₂-NH₂,
R¹² represents hydrogen, methyl, amino, methylamino, aminomethyl or ethylaminomethyl,
R¹³ represents hydrogen, hydroxyl or methoxy,
R¹⁵ represents hydrogen or methyl,
R¹⁶ represents hydrogen or methyl,
R¹⁷ represents hydrogen or methyl,
R¹⁸ represents 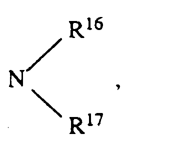
R¹⁹ represents hydrogen or methyl,
R²⁰ represents 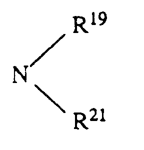 where
R²¹ denotes hydrogen or methyl,
A represents CH₂, O or a direct bond and
n represents 1.

4. Quinolonecarboxylic acid derivatives of the formula (II) 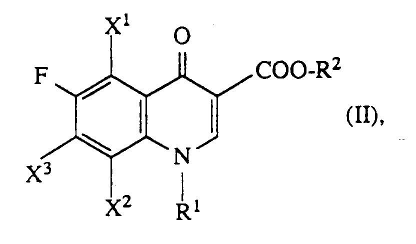 in which
R¹, R², X¹ and X² are as defined in Claim 1 and
X³ represents halogen, in particular fluorine or chlorine.

5. Quinolonecarboxylic acid derivatives of the group consisting of
1-cyclopropyl-8-ethinyl-6-fluoro-1,4-dihydro-7-(4-methyl-1-piperazinyl)-4-oxo-3-quinolinecarboxylic acid,
1-cyclopropyl-7-(cis-2,8-diazabicylco[4.3.0]-non-8-yl)-8-ethinyl-6-fluoro-1,4-dihydro-4-oxo-3-quinolinecarboxylic acid,
7-(3-amino-1-pyrrolidinyl)-1-cyclopropyl-8-ethinyl-6-fluoro-1,4-dihydro-4-oxo-3-quinolinecarboxylic acid,
7-(3-amino-3-methyl-1-pyrrolidinyl)-1-cyclopropyl-8-ethinyl-6-fluoro-1,4-dihydro-4-oxo-3-quinolinecarboxylic acid trifluoroacetate,
1-cyclopropyl-6-fluoro-1,4-dihydro-8-(3-methyl-but-3-en-1-inyl)-7-(4-methyl-1-piperazinyl)-4-oxo-3-quinolinecarboxylic acid,
1-cyclopropyl-8-ethinyl-6-fluoro-1,4-dihydro-7-(2-oxa-5,8-diazabicyclo[4.3.0]non-8-yl)-4-oxo-3-quinolinecarboxylic acid,
1-cyclopropyl-8-ethinyl-6-fluoro-1,4-dihydro-7-(7-methyl-3,7-diazabicyclo[3.3.0]oct-3-yl)-4-oxo-3-quinolinecarboxylic acid,
1-cyclopropyl-8-ethinyl-6-fluoro-1,4-dihydro-7-(4-methylamino-1,3,3a,4,7,7a-hexahydroisoindol-2-yl)-4-oxo-3-quinolinecarboxylic acid and
1-cyclopropyl-7-(cis-2,8-diazabicylco[4.3.0]non-8-yl-6-fluoro-1,4-dihydro-4-oxo-8-vinyl-3-quinolinecarboxylic acid and their hydrates and acid addition salts and also the alkali metal, alkaline earth metal, silver and guanidinium salts.

6. Quinolonecarboxylic acid derivatives from the group consisting of
8-bromo-1-(2,4-difluorophenyl)-6,7-difluoro-1,4-dihydro-4-oxo-3-quinolinecarboxylic acid and
8-bromo-1-ethyl-6,7-difluoro-1,4-dihydro-4-oxo-3-chinolinecarboxylic acid and their hydrates and acid addition salts and also the alkali metal, alkaline earth metal, silver and guanidinium salts.

7. Process for the preparation of quinolonecarboxylic acid derivatives according to Claim 1, characterised in that a compound of the formula (II) 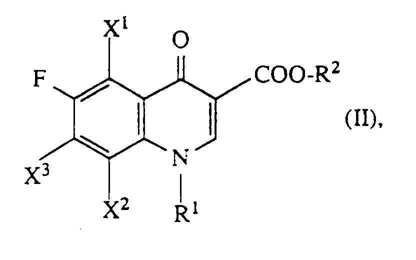 in which
R¹, R², X¹ and X² are as defined in Claim 1 and
X³ represents halogen, in particular fluorine or chlorine,
is reacted with compounds of the formula (III)
Y-H (III)
in which
Y is as defined in Claim 1,
if appropriate in the presence of acid scavengers.

8. Quinolonecarboxylic acid derivatives according to Claims 1 to 5 for treating diseases.

9. Use of quinolonecarboxylic acid derivatives according to Claims 1 to 5 in the preparation of medicaments.

## Revendications (Revendications pour l'(les) Etat(s) contractant(s) suivant(s): AT, BE, CH, DE, DK, FR, GB, IT, LI, LU, MC, NL, PT, SE)

1. Dérivés d'acide quinolone-carboxylique de formule (I) 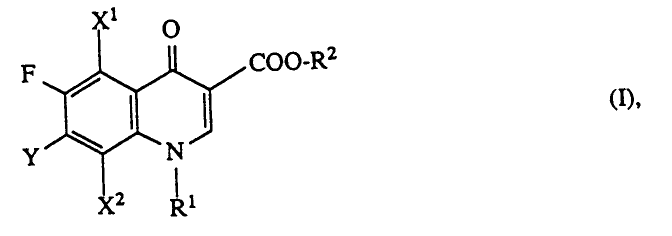 dans laquelle
R¹ est un groupe alkyle en C₁ à C₄ linéaire ou ramifié éventuellement substitué par un radical hydroxy, halogéno ou alkoxy en C₁ à C₃, un groupe cycloalkyle en C₃ à C₆ éventuellement substitué par un radical halogéno ou alkyle en C₁ à C₃, un groupe alcényle en C₂ à C₄, en outre un groupe alkoxy en C₁ à C₃, amino, monoalkylamino ayant 1 à 3 atomes de carbone, dialkylamino ayant 2 à 6 atomes de carbone ou un groupe phényle portant le cas échéant 1 à 3 substituants halogéno,
R² représente de l'hydrogène, un groupe alkyle ayant 1 à 4 atomes de carbone ou le groupe (5-méthyl-2-oxo-1,3-dioxol-4-yl)-méthyle,
X¹ représente de l'hydrogène, du fluor, du chlore, un groupe amino ou méthyle,
X² est un groupe 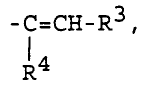 -C≡C-R⁵ ou -CH₂-CH=CH₂
dans lequel
R³ est de l'hydrogène, un radical alkyle en C₁ à C₃, alkoxy en C₁ à C₃ ou alkoxyméthyle ayant 1 à 3 atomes de carbone dans la partie alkoxy,
R⁴ est de l'hydrogène ou un halogène et
R⁵ est de l'hydrogène, un groupe alkyle en C₁ à C₆ éventuellement substitué 1 à 3 fois par un halogène, un groupe alcényle en C₂ ou C₃, un groupe alkoxy ayant 1 à 3 atomes de carbone, un groupe alkoxyméthyle ayant 1 à 3 atomes de carbone dans la partie alkoxy, un halogène ou un groupe triméthylsilyle et
Y représente 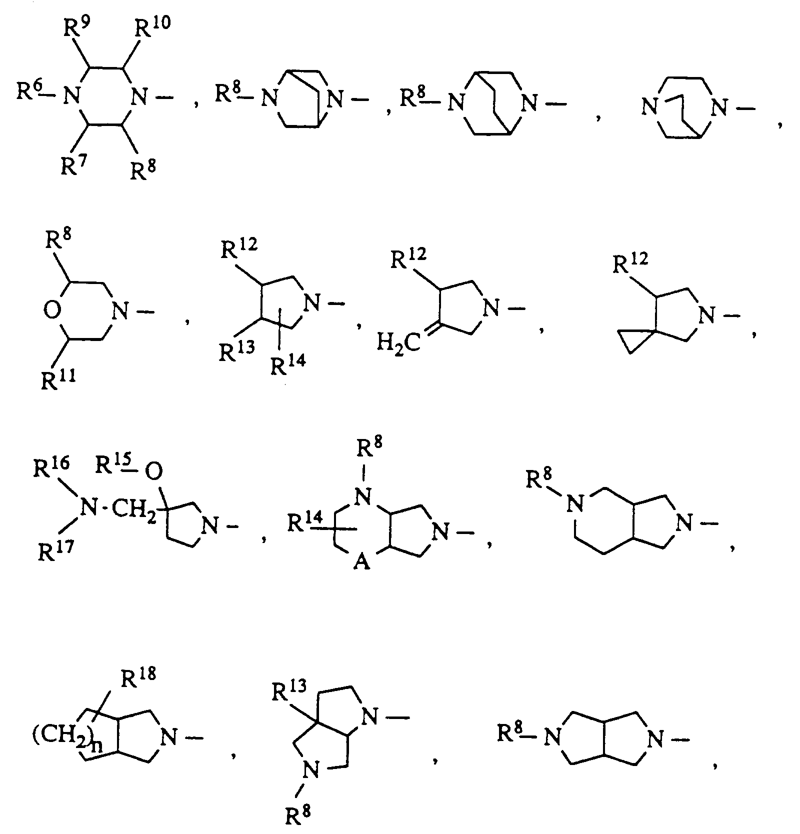 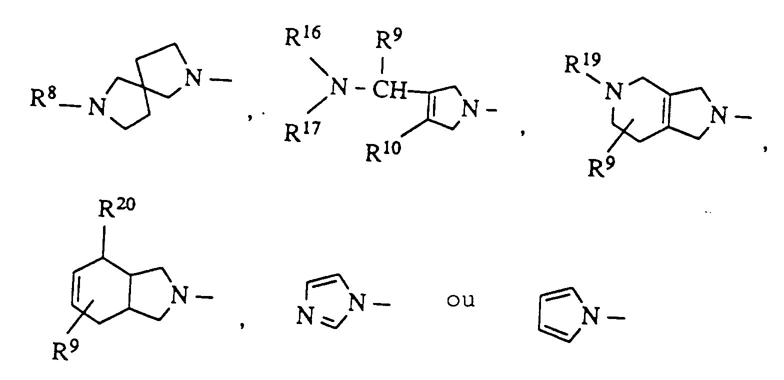 où
R⁶ est de l'hydrogène, un groupe alkyle en C₁ à C₄ linéaire ou ramifié éventuellement substitué par un radical hydroxy ou méthoxy, un groupe cyclopropyle, un groupe oxoalkyle ayant 1 à 4 atomes de carbone, un groupe acyle ayant 1 à 3 atomes de carbone,
R⁷ est de l'hydrogène, un groupe méthyle, phényle, thiényle ou pyridyle,
R⁸ est de l'hydrogène ou un groupe méthyle,
R⁹ est de l'hydrogène ou un groupe méthyle,
R¹⁰ est de l'hydrogène ou un groupe méthyle,
R¹¹ est de l'hydrogène, un groupe méthyle ou un groupe 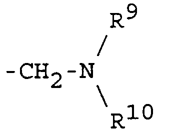
R¹² est de l'hydrogène, un groupe méthyle, amino, un groupe alkyl- ou dialkylamino ayant 1 ou 2 atomes de carbone dans la partie alkyle, éventuellement substitué par un radical hydroxy, un groupe aminométhyle, aminoéthyle, un groupe alkyl- ou dialkylaminométhyle ayant 1 ou 2 atomes de carbone dans la partie alkyle éventuellement substitué par un radical hydroxy, ou un groupe 1-imidazolyle,
R¹³ est de l'hydrogène, un groupe hydroxy, méthoxy, méthylthio ou un halogène, un groupe méthyle ou hydroxyméthyle,
R¹⁴ est de l'hydrogène ou un groupe méthyle,
R¹⁵ est de l'hydrogène, un groupe méthyle ou éthyle,
R¹⁶ est de l'hydrogène, un groupe méthyle ou éthyle,
R¹⁷ est de l'hydrogène, un groupe méthyle ou éthyle,
R¹⁸ est un groupe hydroxy, 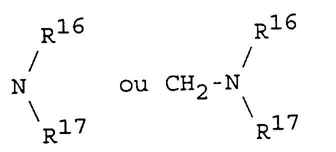
R¹⁹ est de l'hydrogène, un groupe alkyle en C₁ à C₃ éventuellement substitué par un radical hydroxy, un groupe alkoxycarbonyle ayant 1 à 4 atomes de carbone dans la partie alkoxy ou un groupe acyle en C₁ à C₃,
R²⁰ est de l'hydrogène, un groupe hydroxy, 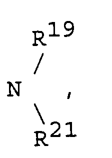 hydroxyméthyle ou 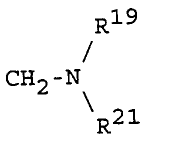 où
R²¹ est de l'hydrogène ou un groupe méthyle,
A représente CH₂, O ou une liaison directe, et
n a la valeur 1 ou 2,
et leurs hydrates et leurs sels d'addition d'acides acceptables du point de vue pharmaceutique ainsi que les sels de métaux alcalins, de métaux alcalino-terreux, d'argent et de guanidinium.

2. Dérivés d'acide quinolone-carboxylique suivant la revendication 1,
dans lesquels
R¹ est un groupe alkyle en C₁ ou C₂ éventuellement substitué par un radical hydroxy, un groupe cycloalkyle en C₃ à C₅, vinyle, amino, monoalkylamino ayant 1 ou 2 atomes de carbone, dialkylamino ayant 2 à 4 atomes de carbone ou un groupe phényle portant le cas échéant 1 ou 2 substituants halogéno,
R² est de l'hydrogène, un groupe alkyle ayant 1 à 3 atomes de carbone ou le groupe (5-méthyl-2-oxo-1,3-dioxol-4-yl)-méthyle,
X¹ est de l'hydrogène, du fluor, du chlore, un groupe amino ou méthyle,
X² est un groupe 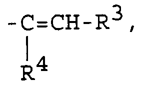 -C≡C-R⁵ ou -CH₂-CH=CH₂
dans lequel
R³ est de l'hydrogène, un groupe alkyle en C₁ ou C₂, méthoxy ou méthoxyméthyle,
R⁴ est de l'hydrogène et
R⁵ est de l'hydrogène, un groupe alkyle en C₁ à C₄ éventuellement substitué 1 à 3 fois par du fluor, un groupe alcényle en C₂ ou C₃, méthoxy ou triméthylsilyle et
Y est 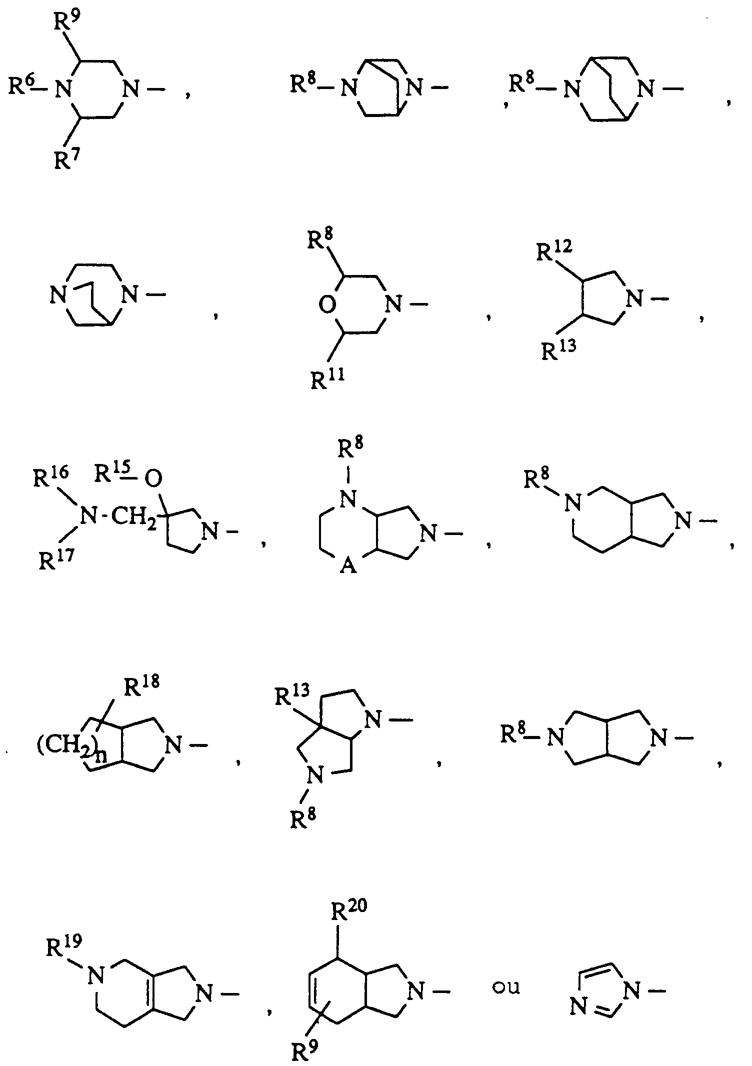 où
R⁶ est de l'hydrogène, un groupe alkyle en C₁ à C₃ linéaire ou ramifié éventuellement substitué par un radical hydroxy, un groupe oxoalkyle ayant 1 à 4 atomes de carbone,
R⁷ est de l'hydrogène, un groupe méthyle ou phényle,
R⁸ est de l'hydrogène ou un groupe méthyle,
R⁹ est de l'hydrogène ou un groupe méthyle,
R¹¹ est de l'hydrogène, un groupe méthyle ou -CH₂-NH₂
R¹² est de l'hydrogène, un groupe méthyle, amino, méthylamino, diméthylamino, aminométhyle, méthylaminométhyle ou éthylaminométhyle,
R¹³ est de l'hydrogène, un groupe hydroxy, méthoxy, du fluor, un groupe méthyle ou hydroxyméthyle,
R¹⁵ est de l'hydrogène ou un groupe méthyle,
R¹⁶ est de l'hydrogène ou un groupe méthyle,
R¹⁷ est de l'hydrogène ou un groupe méthyle,
R¹⁸ est un groupe 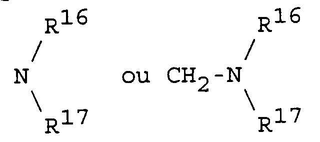
R¹⁹ est de l'hydrogène, un groupe méthyle ou éthyle,
R²⁰ est un groupe 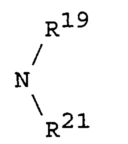 où
R²¹ est de l'hydrogène ou un groupe méthyle,
A représente un groupe CH₂, O ou une liaison directe et
n a la valeur 1 ou 2.

3. Dérivés d'acide quinolone-carboxylique suivant la revendication 1,
dans lesquels
R¹ est un groupe méthyle, éthyle, cyclopropyle ou un groupe phényle éventuellement substitué une ou deux fois par du fluor,
R² est de l'hydrogène, un groupe méthyle ou éthyle,
X¹ est de l'hydrogène, du fluor, du chlore, un groupe amino ou méthyle,
X² est un groupe -CH=CH₂ ou -C≡C-R⁵,
où
R⁵ est de l'hydrogène, un groupe alkyle en C₁ à C₄, alcényle en C₂ ou C₃ ou triméthylsilyle et
Y représente 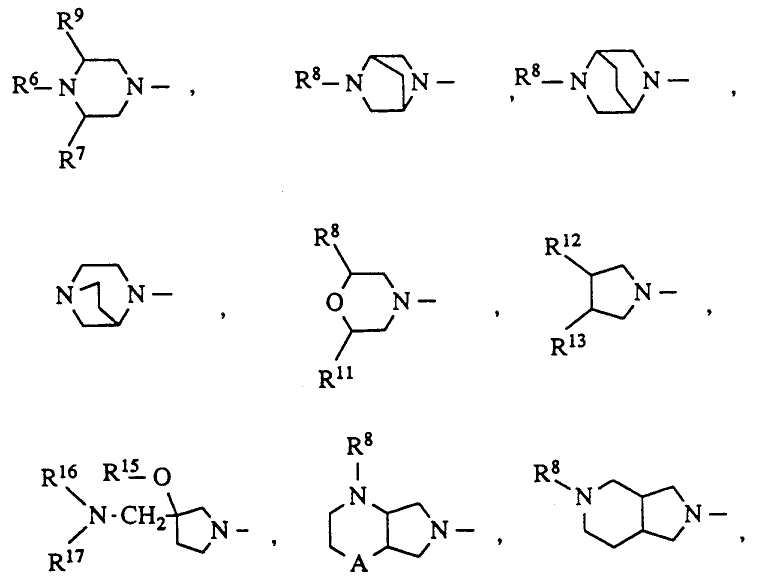 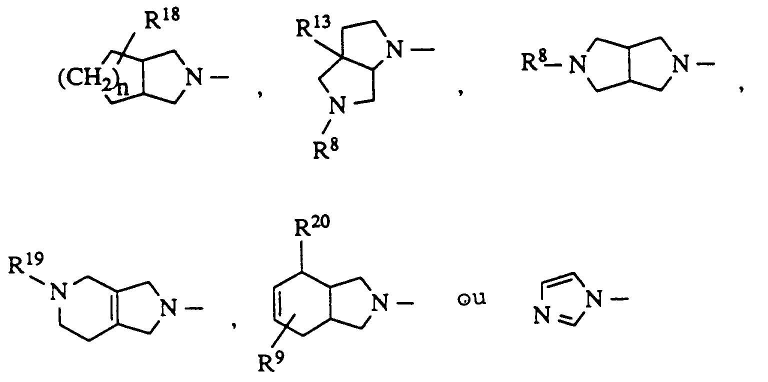 où
R⁶ est de l'hydrogène, un groupe méthyle, un groupe éthyle éventuellement substitué par un radical hydroxy,
R⁷ est de l'hydrogène ou un groupe méthyle,
R⁸ est de l'hydrogène ou un groupe méthyle,
R⁹ est de l'hydrogène ou un groupe méthyle,
R¹¹ est de l'hydrogène ou un groupe -CH₂-NH₂,
R¹² est de l'hydrogène, un groupe méthyle, amino, méthylamino, aminométhyle ou éthylaminométhyle,
R¹³ est de l'hydrogène, un groupe hydroxy ou méthoxy,
R¹⁵ est de l'hydrogène ou un groupe méthyle,
R¹⁶ est de l'hydrogène ou un groupe méthyle,
R¹⁷ est de l'hydrogène ou un groupe méthyle,
R¹⁸ est un groupe 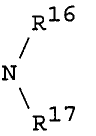
R¹⁹ est de l'hydrogène ou un groupe méthyle,
R²⁰ est un groupe 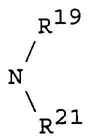 dans lequel
R²¹ est de l'hydrogène ou un groupe méthyle,
A représente CH₂, O ou une liaison directe et
n a la valeur 1.

4. Dérivés d'acide quinolone-carboxylique de formule (II) 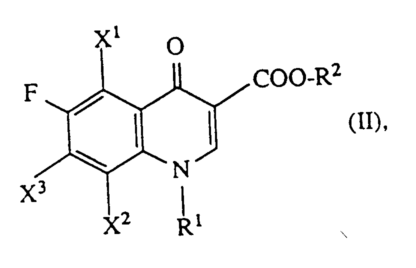 dans laquelle
R¹, R², X¹ et X² ont la définition indiquée dans la revendication 1 et
X³ représente un halogène, en particulier le fluor ou le chlore.

5. Dérivés d'acide quinoléine-carboxylique du groupe consistant en
l'acide 1-cyclopropyl-8-éthynyl-6-fluoro-1,4-dihydro-7-(4-méthyl-1-pipérazinyl)-4-oxo-3-quinoléinecarboxylique,
l'acide 1-cyclopropyl-7-(cis-2,8-diazabicyclo[4.3.0]-non-8-yl)-8-éthynyl-6-fluoro-1,4-dihydro-4-oxo-3-quinoléine-carboxylique,
l'acide 7-(3-amino-1-pyrrolidinyl)-1-cyclopropyl-8-éthynyl-6-fluoro-1,4-dihydro-4-oxo-3-quinoléine-carboxylique,
le trifluoracétate de l'acide 7-(3-amino-3-méthyl-1-pyrrolidinyl)-1-cyclopropyl-8-éthynyl-6-fluoro-1,4-dihydro-4-oxo-3-quinoléine-carboxylique,
l'acide 1-cyclopropyl-6-fluoro-1,4-dihydro-8-(3-méthyl-but-3-ène-1-ynyl)-7-(4-méthyl-1-pipérazinyl)-4-oxo-3-quinoléine-carboxylique,
l'acide 1-cyclopropyl-8-éthynyl-6-fluoro-1,4-dihydro-7-(2-oxa-5,8-diazabicyclo[4.3.0]-non-8-yl)-4-oxo-3-quinoléine-carboxylique,
l'acide 1-cyclopropyl-8-éthynyl-6-fluoro-1,4-dihydro-7-(7-méthyl-3,7-diazabicyclo[3.3.0]-oct-3-yl)-4-oxo-3-quinoléine-carboxylique,
l'acide 1-cyclopropyl-8-éthynyl-6-fluoro-1,4-dihydro-7-(4-méthylamino-1,3,3a,4,7,7a-hexahydroisoindole-2-yl)-4-oxo-3-quinoléine-carboxylique et
l'acide 1-cyclopropyl-7-(cis-2,8-diazabicyclo[4.3.0]-non-8-yl-6-fluoro-1,4-dihydro-4-oxo-8-vinyl-3-quinoléine-carboxylique et leurs hydrates et leurs sels d'addition d'acides acceptables du point de vue pharmacologique ainsi que les sels de métaux alcalins, de métaux alcalino-terreux, d'argent et de guanidinium.

6. Dérivés d'acide quinoléine-carboxylique du groupe consistant en :
l'acide 8-bromo-1-(2,4-difluorophényl)-6,7-difluoro-1,4-dihydro-4-oxo-3-quinoléine-carboxylique et
l'acide 8-bromo-1-éthyl-6,7-difluoro-1,4-dihydro-4-oxo-3-quinoléine-carboxylique et leurs hydrates et leurs sels d'addition d'acides ainsi que les sels de métaux alcalins, de métaux alcalino-terreux, d'argent et de guanidinium.

7. Procédé de production de dérivés d'acide quinolone-carboxylique suivant la revendication 1, caractérisé en ce qu'on fait réagir un composé de formule (II) 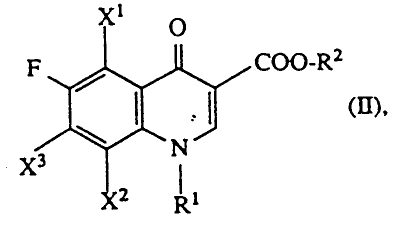 dans laquelle
R¹, R², X¹ et X² ont la définition indiquée suivant la revendication 1 et
X³ est un halogène, notamment le fluor ou le chlore,
avec des composés de formule (III)
Y-H (III)
dans laquelle
Y a la définition suivant la revendication 1,
le cas échéant en présence d'accepteurs d'acides.

8. Dérivés d'acide quinolone-carboxylique suivant les revendications 1 à 5, destinés au traitement de maladies.

9. Médicaments contenant des dérivés d'acide quinolone-carboxylique suivant les revendications 1 à 5.

10. Utilisation de dérivés d'acide quinolone-carboxylique suivant les revendications 1 à 5 pour la préparation de médicaments.

## Revendications (Revendications pour l'(les) Etat(s) contractant(s) suivant(s): ES)

1. Procédé de production de dérivés d'acide quinolone-carboxylique de formule (I) 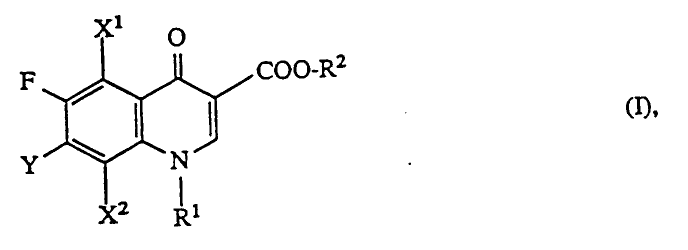 dans laquelle
R¹ est un groupe alkyle en C₁ à C₄ linéaire ou ramifié éventuellement substitué par un radical hydroxy, halogéno ou alkoxy en C₁ à C₃, un groupe cycloalkyle en C₃ à C₆ éventuellement substitué par un radical halogéno ou alkyle en C₁ à C₃, un groupe alcényle en C₂ à C₄, en outre un groupe alkoxy en C₁ à C₃, amino, monoalkylamino ayant 1 à 3 atomes de carbone, dialkylamino ayant 2 à 6 atomes de carbone ou un groupe phényle portant le cas échéant 1 à 3 substituants halogéno,
R² représente de l'hydrogène, un groupe alkyle ayant 1 à 4 atomes de carbone ou le groupe (5-méthyl-2-oxo-1,3-dioxol-4-yl)-méthyle,
X¹ représente de l'hydrogène, du fluor, du chlore, un groupe amino ou méthyle,
X² est un groupe 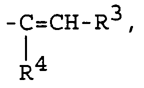 -C≡C-R⁵ ou -CH₂-CH=CH₂
dans lequel
R³ est de l'hydrogène, un radical alkyle en C₁ à C₃, alkoxy en C₁ à C₃ ou alkoxyméthyle ayant 1 à 3 atomes de carbone dans la partie alkoxy,
R⁴ est de l'hydrogène ou un halogène et
R⁵ est de l'hydrogène, un groupe alkyle en C₁ à C₆ éventuellement substitué 1 à 3 fois par un halogène, un groupe alcényle en C₂ ou C₃, un groupe alkoxy ayant 1 à 3 atomes de carbone, un groupe alkoxyméthyle ayant 1 à 3 atomes de carbone dans la partie alkoxy, un halogène ou un groupe triméthylsilyle et
Y représente 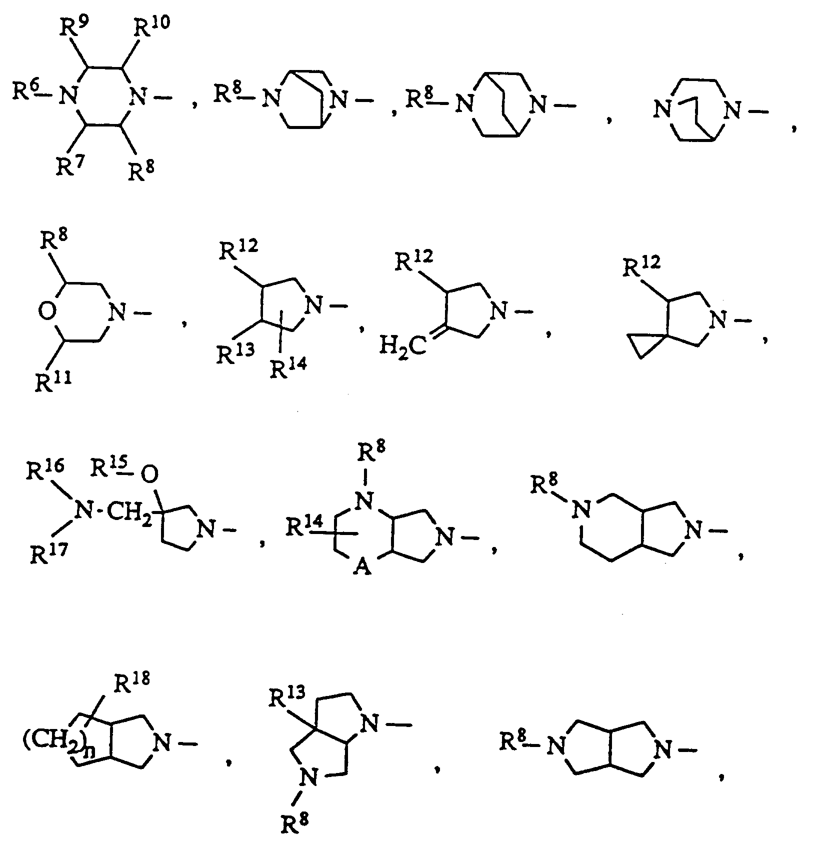 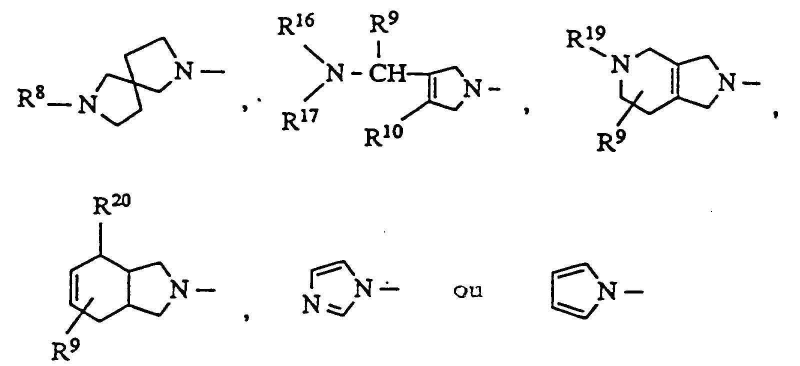 où
R⁶ est de l'hydrogène, un groupe alkyle en C₁ à C₄ linéaire ou ramifié éventuellement substitué par un radical hydroxy ou méthoxy, un groupe cyclopropyle, un groupe oxoalkyle ayant 1 à 4 atomes de carbone, un groupe acyle ayant 1 à 3 atomes de carbone,
R⁷ est de l'hydrogène, un groupe méthyle, phényle, thiényle ou pyridyle,
R⁸ est de l'hydrogène ou un groupe méthyle,
R⁹ est de l'hydrogène ou un groupe méthyle,
R¹⁰ est de l'hydrogène ou un groupe méthyle,
R¹¹ est de l'hydrogène, un groupe méthyle ou un groupe 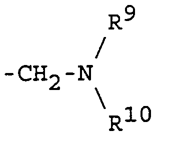
R¹² est de l'hydrogène, un groupe méthyle, amino, un groupe alkyl- ou dialkylamino ayant 1 ou 2 atomes de carbone dans la partie alkyle, éventuellement substitué par un radical hydroxy, un groupe aminométhyle, aminoéthyle, un groupe alkyl- ou dialkylaminométhyle ayant 1 ou 2 atomes de carbone dans la partie alkyle éventuellement substitué par un radical hydroxy, ou un groupe 1-imidazolyle,
R¹³ est de l'hydrogène, un groupe hydroxy, méthoxy, méthylthio ou un halogène, un groupe méthyle ou hydroxyméthyle,
R¹⁴ est de l'hydrogène ou un groupe méthyle,
R¹⁵ est de l'hydrogène, un groupe méthyle ou éthyle,
R¹⁶ est de l'hydrogène, un groupe méthyle ou éthyle,
R¹⁷ est de l'hydrogène, un groupe méthyle ou éthyle,
R¹⁸ est un groupe hydroxy, 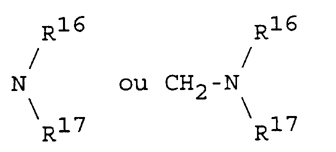
R¹⁹ est de l'hydrogène, un groupe alkyle en C₁ à C₃ éventuellement substitué par un radical hydroxy, un groupe alkoxycarbonyle ayant 1 à 4 atomes de carbone dans la partie alkoxy ou un groupe acyle en C₁ à C₃,
R²⁰ est de l'hydrogène, un groupe hydroxy, 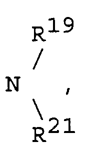 hydroxyméthyle ou 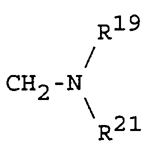 où
R²¹ est de l'hydrogène ou un groupe méthyle,
A représente CH₂, O ou une liaison directe, et
n a la valeur 1 ou 2,
et de leurs hydrates et leurs sels d'addition d'acides acceptables du point de vue pharmaceutique ainsi que les sels de métaux alcalins, de métaux alcalino-terreux, d'argent et de guanidinium, caractérisé en ce qu'on fait réagir un composé de formule (II) 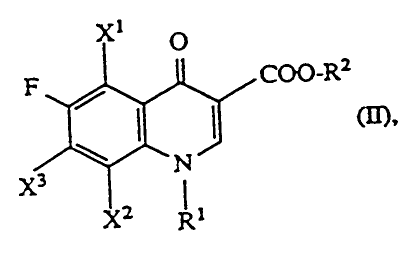 dans laquelle
R¹, R², X¹ et X² ont la définition indiquée ci-dessus et
X³ est un halogène, notamment le fluor ou le chlore,
avec des composés de formule (III)
Y-H (III)
dans laquelle
Y a la définition indiquée ci-dessus,
le cas échéant en présence d'accepteurs d'acides, et on transforme éventuellement les composés ainsi obtenus d'une manière connue en leurs hydrates et leurs sels d'addition d'acides acceptables du point de vue pharmaceutique ainsi que leurs sels de métaux alcalins, de métaux alcalino-terreux, d'argent et de guanidinium.

2. Procédé suivant la revendication 1, caractérisé en ce qu'on prépare les dérivés d'acide quinolone-carboxylique de formule (I)
dans laquelle
R¹ est un groupe alkyle en C₁ ou C₂ éventuellement substitué par un radical hydroxy, un groupe cycloalkyle en C₃ à C₅, vinyle, amino, monoalkylamino ayant 1 ou 2 atomes de carbone, dialkylamino ayant 2 à 4 atomes de carbone ou un groupe phényle portant le cas échéant 1 ou 2 substituants halogéno,
R² est de l'hydrogène, un groupe alkyle ayant 1 à 3 atomes de carbone ou le groupe (5-méthyl-2-oxo-1,3-dioxol-4-yl)-méthyle,
X¹ est de l'hydrogène, du fluor, du chlore, un groupe amino ou méthyle,
X² est un groupe 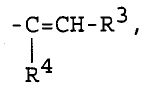 -C≡C-R⁵ ou -CH₂-CH=CH₂
dans lequel
R³ est de l'hydrogène, un groupe alkyle en C₁ ou C₂, méthoxy ou méthoxyméthyle,
R⁴ est de l'hydrogène et
R⁵ est de l'hydrogène, un groupe alkyle en C₁ à C₄ éventuellement substitué 1 à 3 fois par du fluor, un groupe alcényle en C₂ ou C₃, méthoxy ou triméthylsilyle et
Y est 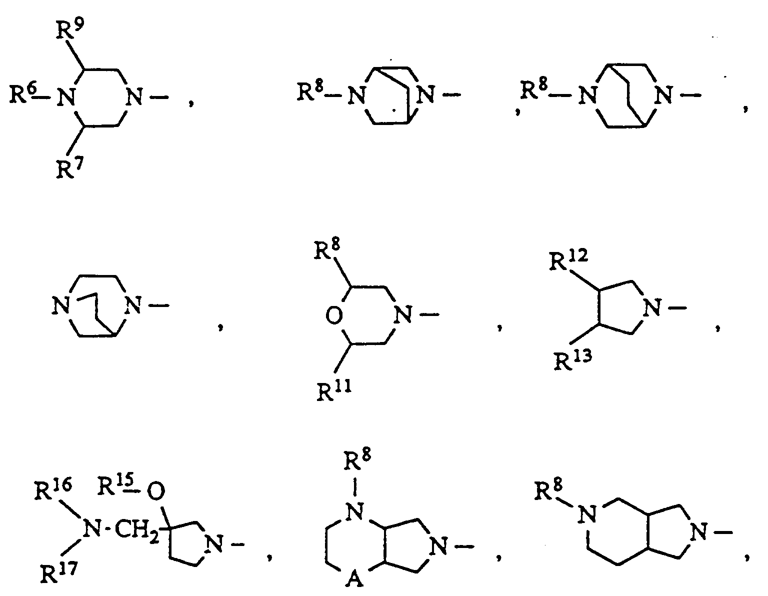 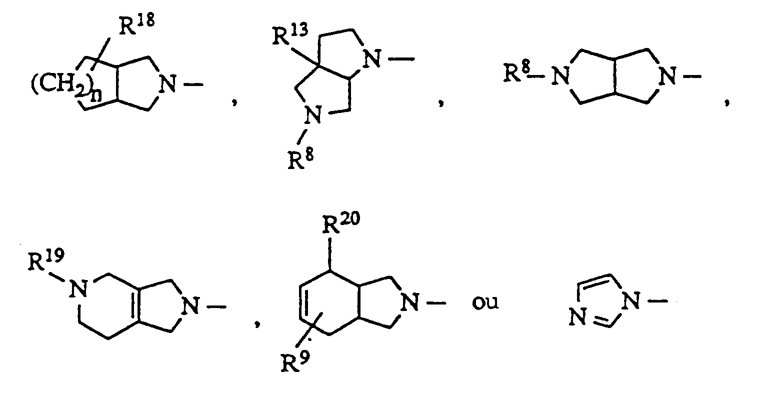 où
R⁶ est de l'hydrogène, un groupe alkyle en C₁ à C₃ linéaire ou ramifié éventuellement substitué par un radical hydroxy, un groupe oxoalkyle ayant 1 à 4 atomes de carbone,
R⁷ est de l'hydrogène, un groupe méthyle ou phényle,
R⁸ est de l'hydrogène ou un groupe méthyle,
R⁹ est de l'hydrogène ou un groupe méthyle,
R¹¹ est de l'hydrogène, un groupe méthyle ou -CH₂-NH₂
R¹² est de l'hydrogène, un groupe méthyle, amino, méthylamino, diméthylamino, aminométhyle, méthylaminométhyle ou éthylaminométhyle,
R¹³ est de l'hydrogène, un groupe hydroxy, méthoxy, du fluor, un groupe méthyle ou hydroxyméthyle,
R¹⁵ est de l'hydrogène ou un groupe méthyle,
R¹⁶ est de l'hydrogène ou un groupe méthyle,
R¹⁷ est de l'hydrogène ou un groupe méthyle,
R¹⁸ est un groupe 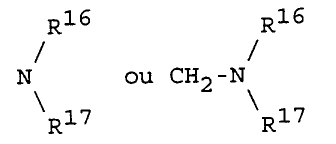
R¹⁹ est de l'hydrogène, un groupe méthyle ou éthyle,
R²⁰ est un groupe 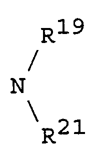 où
R²¹ est de l'hydrogène ou un groupe méthyle,
A représente un groupe CH₂, O ou une liaison directe et
n a la valeur 1 ou 2.

3. Procédé suivant la revendication 1, caractérisé en ce qu'on prépare des dérivés d'acide quinolone-carboxylique de formule (I)
dans laquelle
R¹ est un groupe méthyle, éthyle, cyclopropyle ou un groupe phényle éventuellement substitué une ou deux fois par du fluor,
R² est de l'hydrogène, un groupe méthyle ou éthyle,
X¹ est de l'hydrogène, du fluor, du chlore, un groupe amino ou méthyle,
X² est un groupe -CH=CH₂ ou -C≡C-R⁵,
où
R⁵ est de l'hydrogène, un groupe alkyle en C₁ à C₄, alcényle en C₂ ou C₃ ou triméthylsilyle et
Y représente 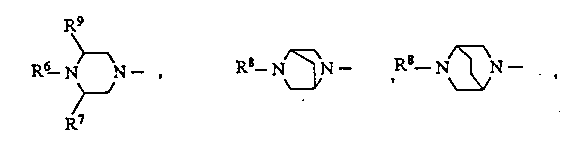 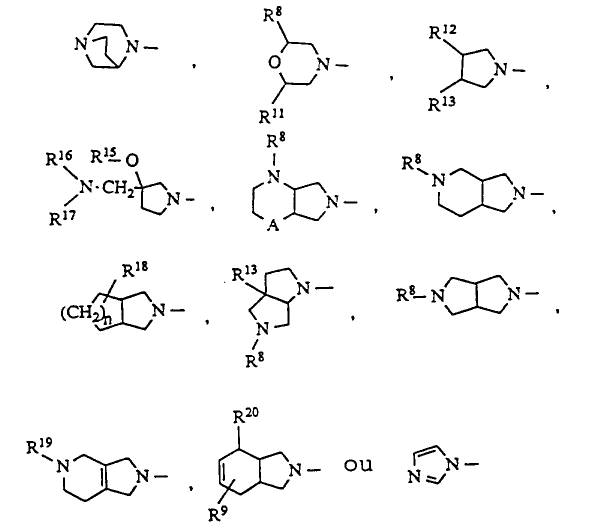 où
R⁶ est de l'hydrogène, un groupe méthyle, un groupe éthyle éventuellement substitué par un radical hydroxy,
R⁷ est de l'hydrogène ou un groupe méthyle,
R⁸ est de l'hydrogène ou un groupe méthyle,
R⁹ est de l'hydrogène ou un groupe méthyle,
R¹¹ est de l'hydrogène ou un groupe -CH₂-NH₂,
R¹² est de l'hydrogène, un groupe méthyle, amino, méthylamino, aminométhyle ou éthylaminométhyle,
R¹³ est de l'hydrogène, un groupe hydroxy ou méthoxy,
R¹⁵ est de l'hydrogène ou un groupe méthyle,
R¹⁶ est de l'hydrogène ou un groupe méthyle,
R¹⁷ est de l'hydrogène ou un groupe méthyle,
R¹⁸ est un groupe 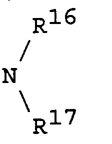
R¹⁹ est de l'hydrogène ou un groupe méthyle,
R²⁰ est un groupe 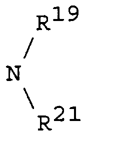 dans lequel
R²¹ est de l'hydrogène ou un groupe méthyle,
A représente CH₂, O ou une liaison directe et
n a la valeur 1.

4. Procédé suivant la revendication 1, caractérisé en ce qu'on prépare des dérivés d'acide quinoléine-carboxylique du groupe consistant en :
l'acide 1-cyclopropyl-8-éthynyl-6-fluoro-1,4-dihydro-7-(4-méthyl-1-pipérazinyl)-4-oxo-3-quinoléine-carboxylique,
l'acide 1-cyclopropyl-7-(cis-2,8-diazabicyclo[4.3.0]-non-8-yl)-8-éthynyl-6-fluoro-1,4-dihydro-4-oxo-3-quinoléine-carboxylique,
l'acide 7-(3-amino-1-pyrrolidinyl)-1-cyclopropyl-8-éthynyl-6-fluoro-1,4-dihydro-4-oxo-3-quinoléine-carboxylique,
le trifluoracétate de l'acide 7-(3-amino-3-méthyl-1-pyrrolidinyl)-1-cyclopropyl-8-éthynyl-6-fluoro-1,4-dihydro-4-oxo-3-quinoléine-carboxylique,
l'acide 1-cyclopropyl-6-fluoro-1,4-dihydro-8-(3-méthyl-but-3-ène-1-ynyl)-7-(4-méthyl-1-pipérazinyl)-4-oxo-3-quinoléine-carboxylique,
l'acide 1-cyclopropyl-8-éthynyl-6-fluoro-1,4-dihydro-7-(2-oxa-5,8-diazabicyclo[4.3.0]-non-8-yl)-4-oxo-3-quinoléine-carboxylique,
l'acide 1-cyclopropyl-8-éthynyl-6-fluoro-1,4-dihydro-7-(7-méthyl-3,7-diazabicyclo[3.3.0]-oct-3-yl)-4-oxo-3-quinoléine-carboxylique,
l'acide 1-cyclopropyl-8-éthynyl-6-fluoro-1,4-dihydro-7-(4-méthylamino-1,3,3a,4,7,7a-hexahydroisoindole-2-yl)-4-oxo-3-quinoléine-carboxylique et
l'acide 1-cyclopropyl-7-(cis-2,8-diazabicyclo[4.3.0]-non-8-yl-6-fluoro-1,4-dihydro-4-oxo-8-vinyl-3-quinoléine-carboxylique et leurs hydrates et leurs sels d'addition d'acides acceptables du point de vue pharmacologique ainsi que les sels de métaux alcalins, de métaux alcalino-terreux, d'argent et de guanidinium.

5. Utilisation de dérivés d'acide quinolone-carboxylique obtenus conformément à la revendication 1 dans la préparation de médicaments.

## Revendications (Revendications pour l'(les) Etat(s) contractant(s) suivant(s): GR)

1. Dérivés d'acide quinolone-carboxylique de formule (I) 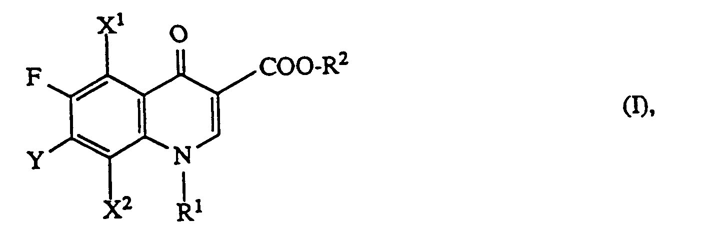 dans laquelle
R¹ est un groupe alkyle en C₁ à C₄ linéaire ou ramifié éventuellement substitué par un radical hydroxy, halogéno ou alkoxy en C₁ à C₃, un groupe cycloalkyle en C₃ à C₆ éventuellement substitué par un radical halogéno ou alkyle en C₁ à C₃, un groupe alcényle en C₂ à C₄, en outre un groupe alkoxy en C₁ à C₃, amino, monoalkylamino ayant 1 à 3 atomes de carbone, dialkylamino ayant 2 à 6 atomes de carbone ou un groupe phényle portant le cas échéant 1 à 3 substituants halogéno,
R² représente de l'hydrogène, un groupe alkyle ayant 1 à 4 atomes de carbone ou le groupe (5-méthyl-2-oxo-1,3-dioxol-4-yl)-méthyle,
X¹ représente de l'hydrogène, du fluor, du chlore, un groupe amino ou méthyle,
X² est un groupe 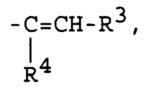 -C≡C-R⁵ ou -CH₂-CH=CH₂
dans lequel
R³ est de l'hydrogène, un radical alkyle en C₁ à C₃, alkoxy en C₁ à C₃ ou alkoxyméthyle ayant 1 à 3 atomes de carbone dans la partie alkoxy,
R⁴ est de l'hydrogène ou un halogène et
R⁵ est de l'hydrogène, un groupe alkyle en C₁ à C₆ éventuellement substitué 1 à 3 fois par un halogène, un groupe alcényle en C₂ ou C₃, un groupe alkoxy ayant 1 à 3 atomes de carbone, un groupe alkoxyméthyle ayant 1 à 3 atomes de carbone dans la partie alkoxy, un halogène ou un groupe triméthylsilyle et
Y représente 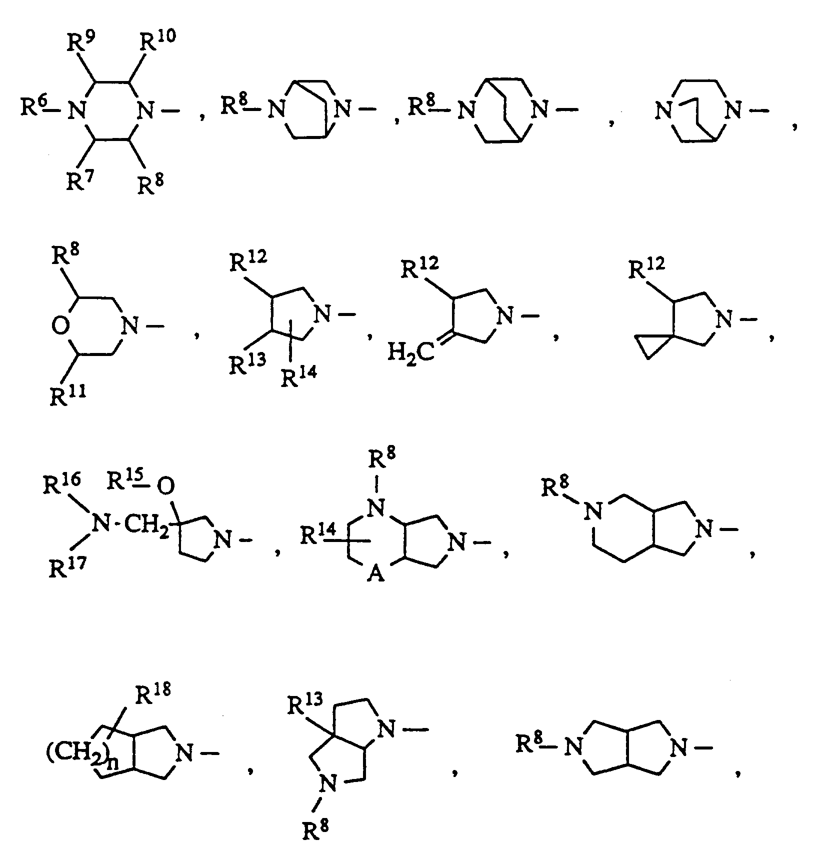 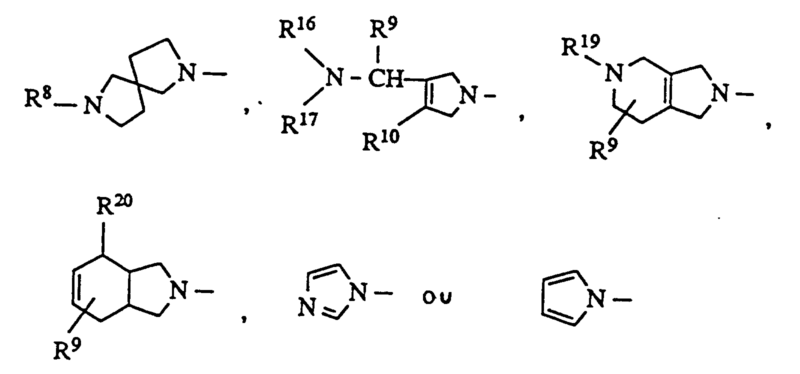 où
R⁶ est de l'hydrogène, un groupe alkyle en C₁ à C₄ linéaire ou ramifié éventuellement substitué par un radical hydroxy ou méthoxy, un groupe cyclopropyle, un groupe oxoalkyle ayant 1 à 4 atomes de carbone, un groupe acyle ayant 1 à 3 atomes de carbone,
R⁷ est de l'hydrogène, un groupe méthyle, phényle, thiényle ou pyridyle,
R⁸ est de l'hydrogène ou un groupe méthyle,
R⁹ est de l'hydrogène ou un groupe méthyle,
R¹⁰ est de l'hydrogène ou un groupe méthyle,
R¹¹ est de l'hydrogène, un groupe méthyle ou un groupe 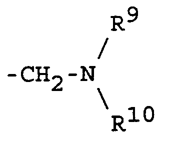
R¹² est de l'hydrogène, un groupe méthyle, amino, un groupe alkyl- ou dialkylamino ayant 1 ou 2 atomes de carbone dans la partie alkyle, éventuellement substitué par un radical hydroxy, un groupe aminométhyle, aminoéthyle, un groupe alkyl- ou dialkylaminométhyle ayant 1 ou 2 atomes de carbone dans la partie alkyle éventuellement substitué par un radical hydroxy, ou un groupe 1-imidazolyle,
R¹³ est de l'hydrogène, un groupe hydroxy, méthoxy, méthylthio ou un halogène, un groupe méthyle ou hydroxyméthyle,
R¹⁴ est de l'hydrogène ou un groupe méthyle,
R¹⁵ est de l'hydrogène, un groupe méthyle ou éthyle,
R¹⁶ est de l'hydrogène, un groupe méthyle ou éthyle,
R¹⁷ est de l'hydrogène, un groupe méthyle ou éthyle,
R¹⁸ est un groupe hydroxy, 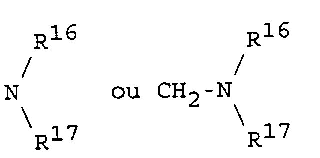
R¹⁹ est de l'hydrogène, un groupe alkyle en C₁ à C₃ éventuellement substitué par un radical hydroxy, un groupe alkoxycarbonyle ayant 1 à 4 atomes de carbone dans la partie alkoxy ou un groupe acyle en C₁ à C₃,
R²⁰ est de l'hydrogène, un groupe hydroxy, 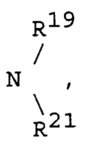 hydroxyméthyle ou 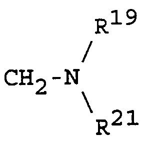 où
R²¹ est de l'hydrogène ou un groupe méthyle,
A représente CH₂, O ou une liaison directe, et
n a la valeur 1 ou 2,
et leurs hydrates et leurs sels d'addition d'acides ainsi que les sels de métaux alcalins, de métaux alcalino-terreux, d'argent et de guanidinium.

2. Dérivés d'acide quinolone-carboxylique suivant la revendication 1,
dans lesquels
R¹ est un groupe alkyle en C₁ ou C₂ éventuellement substitué par un radical hydroxy, un groupe cycloalkyle en C₃ à C₅, vinyle, amino, monoalkylamino ayant 1 ou 2 atomes de carbone, dialkylamino ayant 2 à 4 atomes de carbone ou un groupe phényle portant le cas échéant 1 ou 2 substituants halogéno,
R² est de l'hydrogène, un groupe alkyle ayant 1 à 3 atomes de carbone ou le groupe (5-méthyl-2-oxo-1,3-dioxol-4-yl)-méthyle,
X¹ est de l'hydrogène, du fluor, du chlore, un groupe amino ou méthyle,
X² est un groupe 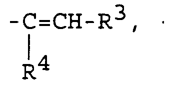 -C≡C-R⁵ ou -CH₂-CH=CH₂
dans lequel
R³ est de l'hydrogène, un groupe alkyle en C₁ ou C₂, méthoxy ou méthoxyméthyle,
R⁴ est de l'hydrogène et
R⁵ est de l'hydrogène, un groupe alkyle en C₁ à C₄ éventuellement substitué 1 à 3 fois par du fluor, un groupe alcényle en C₂ ou C₃, méthoxy ou triméthylsilyle et
Y est 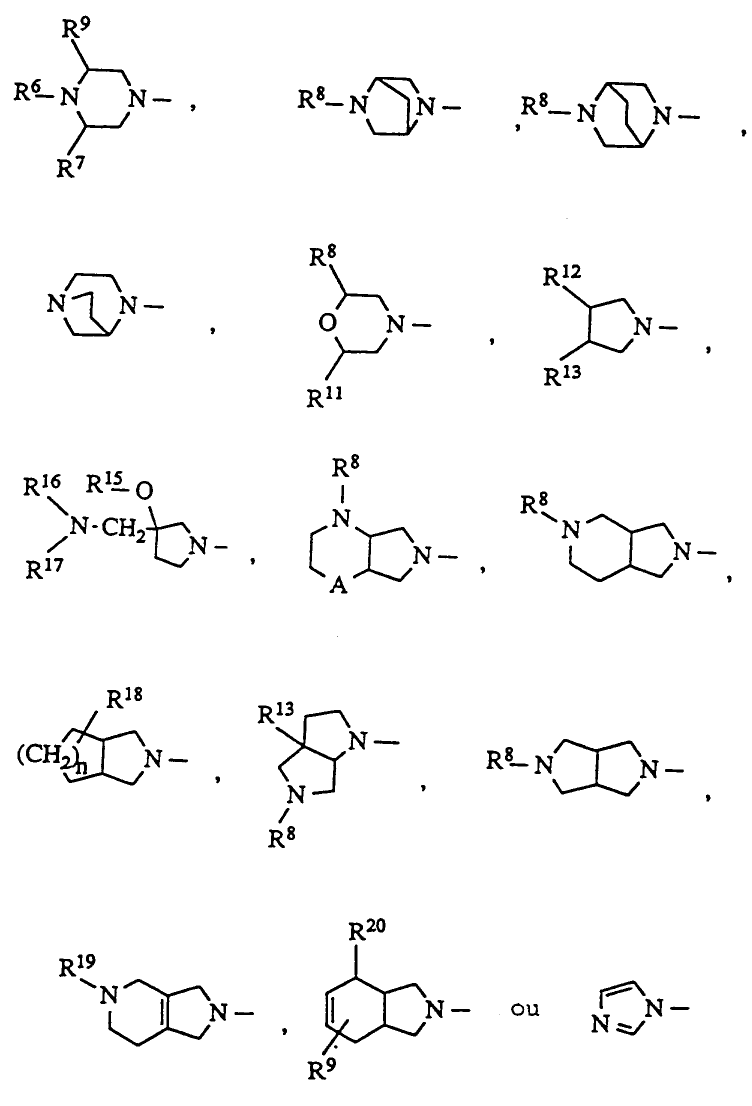 où
R⁶ est de l'hydrogène, un groupe alkyle en C₁ à C₃ linéaire ou ramifié éventuellement substitué par un radical hydroxy, un groupe oxoalkyle ayant 1 à 4 atomes de carbone,
R⁷ est de l'hydrogène, un groupe méthyle ou phényle,
R⁸ est de l'hydrogène ou un groupe méthyle,
R⁹ est de l'hydrogène ou un groupe méthyle,
R¹¹ est de l'hydrogène, un groupe méthyle ou -CH₂-NH₂
R¹² est de l'hydrogène, un groupe méthyle, amino, méthylamino, diméthylamino, aminométhyle, méthylaminométhyle ou éthylaminométhyle,
R¹³ est de l'hydrogène, un groupe hydroxy, méthoxy, du fluor, un groupe méthyle ou hydroxyméthyle,
R¹⁵ est de l'hydrogène ou un groupe méthyle,
R¹⁶ est de l'hydrogène ou un groupe méthyle,
R¹⁷ est de l'hydrogène ou un groupe méthyle,
R¹⁸ est un groupe 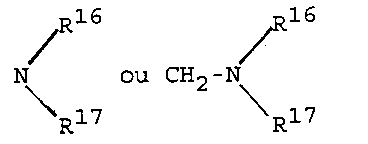
R¹⁹ est de l'hydrogène, un groupe méthyle ou éthyle,
R²⁰ est un groupe 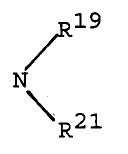 où
R²¹ est de l'hydrogène ou un groupe méthyle,
A représente un groupe CH₂, O ou une liaison directe et
n a la valeur 1 ou 2.

3. Dérivés d'acide quinolone-carboxylique suivant la revendication 1,
dans lesquels
R¹ est un groupe méthyle, éthyle, cyclopropyle ou un groupe phényle éventuellement substitué une ou deux fois par du fluor,
R² est de l'hydrogène, un groupe méthyle ou éthyle,
X¹ est de l'hydrogène, du fluor, du chlore, un groupe amino ou méthyle,
X² est un groupe -CH=CH₂ ou -C≡C-R⁵,
où
R⁵ est de l'hydrogène, un groupe alkyle en C₁ à C₄, alcényle en C₂ ou C₃ ou triméthylsilyle et
Y représente 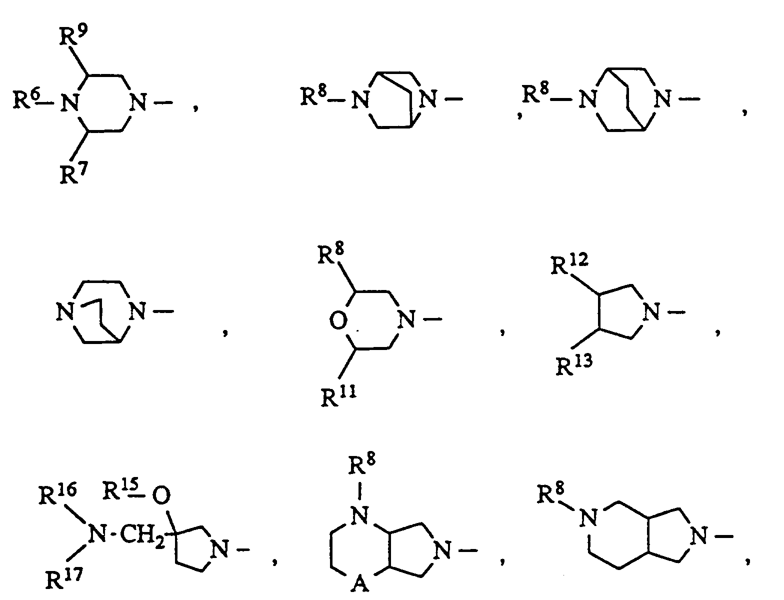 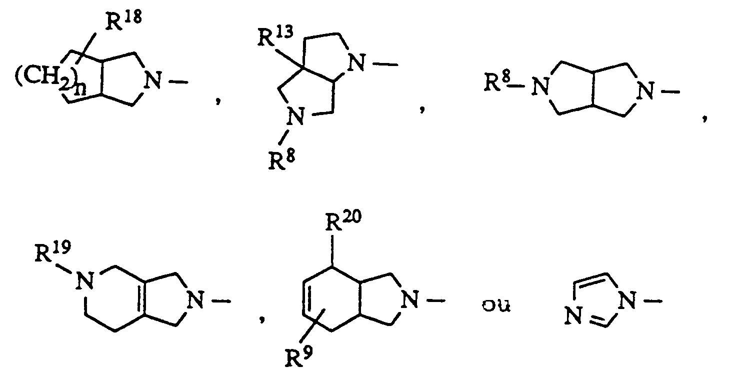 où
R⁶ est de l'hydrogène, un groupe méthyle, un groupe éthyle éventuellement substitué par un radical hydroxy,
R⁷ est de l'hydrogène ou un groupe méthyle,
R⁸ est de l'hydrogène ou un groupe méthyle,
R⁹ est de l'hydrogène ou un groupe méthyle,
R¹¹ est de l'hydrogène ou un groupe -CH₂-NH₂,
R¹² est de l'hydrogène, un groupe méthyle, amino, méthylamino, aminométhyle ou éthylaminométhyle,
R¹³ est de l'hydrogène, un groupe hydroxy ou méthoxy,
R¹⁵ est de l'hydrogène ou un groupe méthyle,
R¹⁶ est de l'hydrogène ou un groupe méthyle,
R¹⁷ est de l'hydrogène ou un groupe méthyle,
R¹⁸ est un groupe 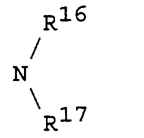
R¹⁹ est de l'hydrogène ou un groupe méthyle,
R²⁰ est un groupe 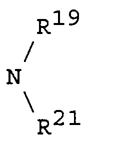 dans lequel
R²¹ est de l'hydrogène ou un groupe méthyle,
A représente CH₂, O ou une liaison directe et
n a la valeur 1.

4. Dérivés d'acide quinolone-carboxylique de formule (II) 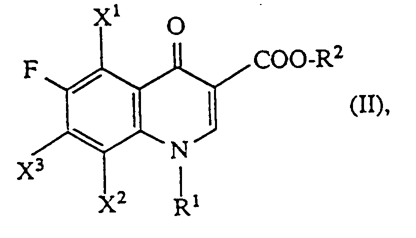 dans laquelle
R¹, R², X¹ et X² ont la définition indiquée dans la revendication 1 et
X³ représente un halogène, en particulier le fluor ou le chlore.

5. Dérivés d'acide quinoléine-carboxylique du groupe consistant en
l'acide 1-cyclopropyl-8-éthynyl-6-fluoro-1,4-dihydro-7-(4-méthyl-1-pipérazinyl)-4-oxo-3-quinoléine-carboxylique,
l'acide 1-cyclopropyl-7-(cis-2,8-diazabicyclo[4.3.0]-non-8-yl)-8-éthynyl-6-fluoro-1,4-dihydro-4-oxo-3-quinoléine-carboxylique,
l'acide 7-(3-amino-1-pyrrolidinyl)-1-cyclopropyl-8-éthynyl-6-fluoro-1,4-dihydro-4-oxo-3-quinoléine-carboxylique,
le trifluoracétate de l'acide 7-(3-amino-3-méthyl-1-pyrrolidinyl)-1-cyclopropyl-8-éthynyl-6-fluoro-1,4-dihydro-4-oxo-3-quinoléine-carboxylique,
l'acide 1-cyclopropyl-6-fluoro-1,4-dihydro-8-(3-méthyl-but-3-ène-1-ynyl)-7-(4-méthyl-1-pipérazinyl)-4-oxo-3-quinoléine-carboxylique,
l'acide 1-cyclopropyl-8-éthynyl-6-fluoro-1,4-dihydro-7-(2-oxa-5,8-diazabicyclo[4.3.0]-non-8-yl)-4-oxo-3-quinoléine-carboxylique,
l'acide 1-cyclopropyl-8-éthynyl-6-fluoro-1,4-dihydro-7-(7-méthyl-3,7-diazabicyclo[3.3.0]-oct-3-yl)-4-oxo-3-quinoléine-carboxylique,
l'acide 1-cyclopropyl-8-éthynyl-6-fluoro-1,4-dihydro-7-(4-méthylamino-1,3,3a,4,7,7a-hexahydroisoindole-2-yl)-4-oxo-3-quinoléine-carboxylique et
l'acide 1-cyclopropyl-7-(cis-2,8-diazabicyclo[4.3.0]-non-8-yl-6-fluoro-1,4-dihydro-4-oxo-8-vinyl-3-quinoléine-carboxylique et leurs hydrates et leurs sels d'addition d'acides ainsi que les sels de métaux alcalins, de métaux alcalino-terreux, d'argent et de guanidinium.

6. Dérivés d'acide quinoléine-carboxylique du groupe consistant en :
l'acide 8-bromo-1-(2,4-difluorophényl)-6,7-difluoro-1,4-dihydro-4-oxo-3-quinoléine-carboxylique et
l'acide 8-bromo-1-éthyl-6,7-difluoro-1,4-dihydro-4-oxo-3-quinoléine-carboxylique et leurs hydrates et leurs sels d'addition d'acides ainsi que les sels de métaux alcalins, de métaux alcalino-terreux, d'argent et de guanidinium.

7. Procédé de production de dérivés d'acide quinolone-carboxylique suivant la revendication 1, caractérisé en ce qu'on fait réagir un composé de formule (II) 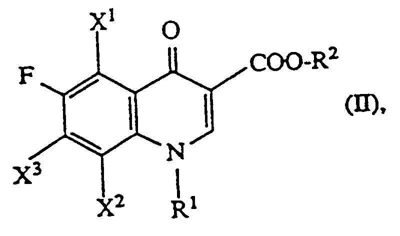 dans laquelle
R¹, R², X¹ et X² ont la définition indiquée suivant la revendication 1 et
X³ est un halogène, notamment le fluor ou le chlore,
avec des composés de formule (III)
Y-H (III)
dans laquelle
Y a la définition suivant la revendication 1,
le cas échéant en présence d'accepteurs d'acides.

8. Dérivés d'acide quinolone-carboxylique suivant les revendications 1 à 5, destinés au traitement de maladies.

9. Utilisation de dérivés d'acide quinolone-carboxylique suivant les revendications 1 à 5 pour la préparation de médicaments.
